# EUROPÄISCHE PATENTSCHRIFT

(11) **EP 0 859 774 B1**
(45) Veröffentlichungstag und Bekanntmachung des Hinweises auf die Patenterteilung: **03.04.2002**
(21) Anmeldenummer: 96937202.8
(22) Anmeldetag: 21.10.1996
(51) Int. Cl.: C07D 409/12, C07D 249/12, C07D 413/12, C07D 271/06

(54) **SUBSTITUIERTE SULFONYLAMINO(THIO)CARBONYLVERBINDUNGEN ALS HERBIZIDE**
SUBSTITUTED SULPHONYLAMINO (THIO)CARBONYL COMPOUNDS AS HERBICIDES
COMPOSES SULFONYLAMINO(THIO)CARBONYLE SUBSTITUES UTILISES COMME HERBICIDES

(30) Priorität: 02.11.1995 DE 19540737
(43) Veröffentlichungstag der Anmeldung: 26.08.1998
(73) Patentinhaber: BAYER AG, 51368 Leverkusen (DE)
(72) Erfinder: MÜLLER, Klaus-Helmut, D-40593 Düsseldorf (DE); DREWES, Mark, Wilhelm, D-40764 Langenfeld (DE); FINDEISEN, Kurt, D-51375 Leverkusen (DE); GESING, Ernst, Rudolf, F., D-40699 Erkrath (DE); JANSEN, Johannes, R., D-40789 Monheim (DE); KIRSTEN, Rolf, D-40789 Monheim (DE); KLUTH, Joachim, D-40764 Langenfeld (DE); PHILIPP, Ulrich, D-51065 Köln (DE); RIEBEL, Hans-Jochem, D-42113 Wuppertal (DE); DOLLINGER, Markus, D-51381 Leverkusen (DE); SANTEL, Hans-Joachim, D-51371 Leverkusen (DE)
(86) Internationale Anmeldenummer: EP9604559
(87) Internationale Veröffentlichungsnummer: WO9716449

(56) Entgegenhaltungen:
- EP-A- 0 341 489
- EP-A- 0 431 291
- EP-A- 0 482 349
- EP-A- 0 507 171
- EP-A- 0 534 266
- EP-A- 0 569 193
- EP-A- 0 569 810
- US-A- 4 877 440

## Beschreibung

Die Erfindung betrifft substituierte Sulfonylamino(thio)carbonylverbindungen, mehrere Verfahren und Zwischenprodukte zu ihrer Herstellung sowie ihre Verwendung als Herbizide.

Es ist bereits bekannt, dass bestimmte Klassen von Sulfonylaminocarbonylverbindungen eine herbizide Wirkung zeigen (vgl. EP-A 569 810, EP-A 507 171, EP-A 482 349, EP-A 431 291, EP-A 534 266, EP-A 341 489).

Weiterhin ist bereits bekannt, daß bestimmte Sulfonylaminocarbonylverbindungen, wie z.B. die Verbindungen N-(2-Chlor-6-methoxycarbonyl-phenylsulfonyl)-5-phenyl-1,2,4-oxadiazol-3-carboxamid (vgl. EP 569 810, Beispiel 204), N-(2-Chlor-6-methoxycarbonyl-phenylsulfonyl)-oxazol-2-carboxamid (vgl. EP 569 810, Beispiel 239), N-(2-Chlor-6-methoxycarbonyl-phenylsulfonyl)-4-methyl-oxazol-2-carboxamid (vgl. EP 569 810, Beispiel 278), N-(2-Chlor-6-methoxycarbonyl-phenylsulfonyl)-4-ethyl-5-methyl-oxazol-2-carboxamid (vgl. EP 569 810, Beispiel 329), N-(2-Chlor-6-methoxycarbonyl-phenylsulfonyl)-5-ethyl-oxazol-4-carboxamid (vgl. EP 569 810, Beispiel 366), N-(2-Chlor-6-methoxycarbonyl-phenylsulfonyl)-5-ethylthiazol-2-carboxamid (vgl. EP 569810, Beispiel 441), N-(2-Chlor-6-methoxy-carbonyl-phenylsulfonyl)-5-methyl-4-methylthio-thiazol-2-carboxamid (vgl. EP 569 810, Beispiel 532), N-(2-Chlor-6-methoxycarbonyl-phenylsulfonyl)-2,5-dimethyl-thiazol-4-carboxamid (vgl. EP 569810, Beispiel 576), N-(2-Chlor-6-methoxycarbonyl-phenylsulfonyl)-2-chlor-thiazol-5-carboxamid (vgl. EP 569810, Beispiel 607), N-(2-Chlor-6-methoxycarbonyl-phenylsulfonyl)-1,3,4-oxadiazol-2-carboxamid (vgl. EP 569810, Beispiel 641), N-(2-Chlor-6-methoxycarbonyl-phenylsulfonyl)-1,3,4-thiadiazol-2-carboxamid (vgl. EP 569810, Beispiel 701), N-(2-Chlor-6-methoxycarbonyl-phenylsulfonyl)-5-chlor-1,3,4-thiadiazol-2-carboxamid (vgl. EP 569810, Beispiel 735), N-(2-Chlor-6-methoxycarbonyl-phenylsulfonyl)-5-phenyl-1,3,4-thiadiazol-2-carboxamid (vgl. EP 569810, Beispiel 757), N-(2-Chlor-6-methoxycarbonyl-phenylsulfonyl)-5-methyl-isoxazol-3-carboxamid (vgl. EP 569810, Beispiel 791), N-(2-Chlor-6-methoxycarbonyl-phenylsulfonyl)-isoxazol-3-carboxamid (vgl. EP 569810, Beispiel 861), N-(2-Chlor-6-methoxycarbonyl-phenylsulfonyl)-isoxazol-5-carboxamid (vgl. EP 569810, Beispiel 871), N-(2-Chlor-6-methoxycarbonyl-phenylsulfonyl)-5-methyl-isoxazol-4-carboxamid (vgl. EP 569810, Beispiel 918) und N-(2-Chlor-6-methoxycarbonyl-phenylsulfonyl)-3,5-dimethylisoxazol-4-carboxamid (vgl. EP 569810, Beispiel 925) herbizide Eigenschaften aufweisen (vgl. auch EP 341489, EP 422469, EP 425948, EP 431291, EP 507171, EP 534266, DE 4029753). Die Wirkung dieser Verbindungen ist jedoch nicht in allen Belangen zufriedenstellend.

Auch ähnliche Sulfonylaminoverbindungen mit pharmazeutischer Anwendungsmöglichkeit sind bekannt geworden (vgl. EP-A 569 193).

Es wurden nun die neuen substituierten Sulfonylamino(thio)carbonylverbindungen der allgemeinen Formel (I), 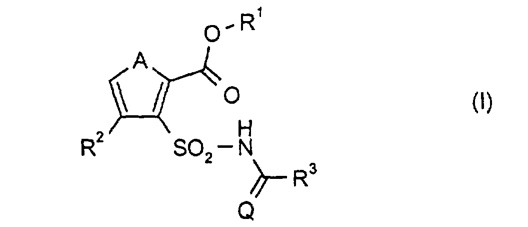 in welcher
- A: für Schwefel oder -CH=CH- steht,
- Q: für Sauerstoff oder Schwefel steht,
- R¹: für gegebenenfalls durch Cyano, Nitro, Halogen oder C₁-C₄-Alkoxy substituiertes C₁-C₆-Alkyl, für jeweils gegebenenfalls durch Cyano oder Halogen substituiertes C₂-C₆-Alkenyl oder C₂-C₆-Alkinyl, für jeweils gegebenenfalls durch Cyano, Halogen oder C₁-C₄-Alkyl substituiertes C₃-C₆-Cycloalkyl, C₃-C₆-Cycloalkyl-C₁-C₄-alkyl, für jeweils gegebenenfalls durch Cyano, Nitro, Halogen, C₁-C₄-Alkyl, C₁-C₄-Halogenalkyl, C₁-C₄-Alkoxy oder C₁-C₄-Halogenalkoxy substituiertes Phenyl oder Phenyl-C₁-C₄-alkyl, oder für jeweils gegebenenfalls durch Cyano, Nitro, Halogen, C₁-C₄-Alkyl, C₁-C₄-Halogenalkyl, C₁-C₄-Alkoxy oder C₁-C₄-Halogenalkoxy substituiertes Heterocyclyl oder Heterocyclyl-C₁-C₄-alkyl steht, wobei jeweils die Heterocyclylgruppe aus der Reihe Oxetanyl, Thietanyl, Furyl, Tetrahydrofuryl, Thienyl, Tetrahydrothienyl, Oxazolyl, Isoxazolyl, Thiazolyl, Oxadiazolyl, Thiadiazolyl ausgewählt ist,
- R²: für Cyano, Nitro, Halogen, für jeweils gegebenenfalls durch Cyano, Halogen oder C₁-C₄-Alkoxy substituiertes C₁-C₄-Alkyl, C₁-C₄-Alkoxy, C₁-C₄-Alkoxy-carbonyl, C₁-C₄-Alkylthio, C₁-C₄-Alkylsulfinyl oder C₁-C₄-Alkylsulfonyl, oder für jeweils gegebenenfalls durch Cyano oder Halogen substituiertes C₂-C₄-Alkenyl, C₂-C₄-Alkinyl, C₂-C₄-Alkenyloxy oder C₂-C₄-Alkinyloxy steht, und
- R³: für gegebenenfalls substituiertes Heterocyclyl der nachstehenden Formel steht, 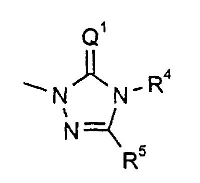 worin
- Q¹: für Sauerstoff oder Schwefel steht sowie
- R⁴: für Wasserstoff, Hydroxy, Amino, Cyano, für C₂-C₁₀-Alkylidenamino, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor, Chlor, Brom, Cyano, C₁-C₄-Alkoxy oder C₁-C₄-Alkoxy-carbonyl substituiertes C₁-C₆-Alkoxy, C₁-C₆-Alkylamino oder C₁-C₆-Alkyl-carbonylamino, für C₃-C₆-Alkenyloxy, für Di-(C₁-C₄-alkyl)-amino, für jeweils gegebenenfalls durch Fluor, Chlor, Brom, Cyano und/oder C₁-C₄-Alkyl substituiertes C₃-C₆-Cycloalkyl, C₃-C₆-Cycloalkylamino oder C₃-C₆-Cycloalkyl-C₁-C₄-alkyl, oder für jeweils gegebenenfalls durch Fluor, Chlor, Brom, Cyano, Nitro, C₁-C₄-Alkyl, Trifluormethyl und/oder C₁-C₄-Alkoxy substituiertes Phenyl oder Phenyl-C₁-C₄-alkyl steht, und
- R⁵: für Wasserstoff, Hydroxy, Mercapto, Amino, Cyano, Fluor, Chlor, Brom, Iod, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor, Chlor, Cyano, C₁-C₄-Alkoxy oder C₁-C₄-Alkoxy-carbonyl substituiertes C₁-C₆-Alkoxy, C₁-C₆-Alkylthio, C₁-C₆-Alkylamino oder C₁-C₆-Alkylcarbonylamino, für C₃-C₆-Alkenyloxy, C₃-C₆-Alkinyloxy, C₃-C₆-Alkenylthio, C₃-C₆-Alkinylthio, C₃-C₆-Alkenylamino oder C₃-C₆-Alkinylamino, für Di-(C₁-C₄-alkyl)-amino, für jeweils gegebenenfalls durch Methyl und/oder Ethyl substituiertes Aziridino, Pyrrolidino, Piperidino oder Morpholino, für jeweils gegebenenfalls durch Fluor, Chlor, Brom, Cyano und/oder C₁-C₄-Alkyl substituiertes C₃-C₆-Cycloalkyl, C₅-C₆-Cycloalkenyl, C₃-C₆-Cycloalkyloxy, C₃-C₆-Cycloalkylthio, C₃-C₆-Cycloalkylamino, C₃-C₆-Cycloalkyl-C₁-C₄-alkyl, C₃-C₆-Cycloalkyl-C₁-C₄-alkoxy, C₃-C₆-Cycloalkyl-C₁-C₄-alkylthio oder C₃-C₆-Cycloalkyl-C₁-C₄-alkylamino, oder für jeweils gegebenenfalls durch Fluor, Chlor, Brom, Cyano, Nitro, C₁-C₄-Alkyl, Trifluormethyl, C₁-C₄-Alkoxy und/oder C₁-C₄-Alkoxycarbonyl substituiertes Phenyl, Phenyl-C₁-C₄-alkyl, Phenoxy, Phenyl-C₁-C₄-alkoxy, Phenylthio, Phenyl-C₁-C₄-alkylthio, Phenylamino oder Phenyl-C₁-C₄-alkylamino steht, oder
- R⁴ und R⁵: zusammen für gegebenenfalls verzweigtes Alkandiyl mit 3 bis 11 Kohlenstoffatomen stehen.

Man erhält die neuen substituierten Sulfonylamino(thio)carbonylverbindungen der allgemeinen Formel (I), wenn man
(a) Sulfonamide der allgemeinen Formel (II) 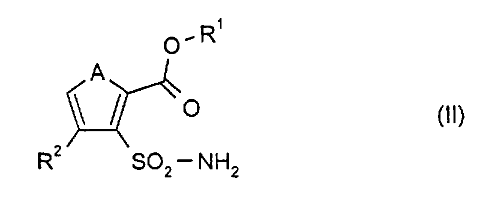 in welcher
   A, R¹ und R² die oben angegebene Bedeutung haben,
      mit (Thio)Carbonsäurederivaten der allgemeinen Formel (III) 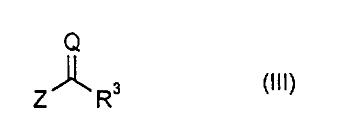 in welcher
   Q und R³ die oben angegebene Bedeutung haben und
      - Z: für Halogen, Alkoxy, Aryloxy oder Arylalkoxy steht,
      gegebenenfalls in Gegenwart eines Säureakzeptors und gegebenenfalls in Gegenwart eines Verdünnungsmittels umsetzt,
   oder wenn man
(b) Sulfonyliso(thio)cyanate der allgemeinen Formel (IV) 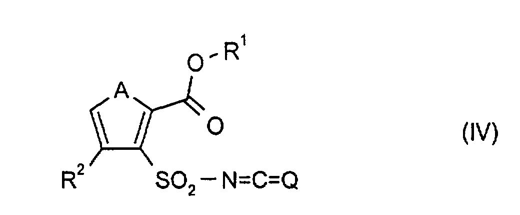 in welcher
   A, Q, R¹ und R² die oben angegebene Bedeutung haben,
      mit Heterocyclen der allgemeinen Formel (V)

      H-R³ (V)

      in welcher
      - R³: die oben angegebene Bedeutung hat,
      gegebenenfalls in Gegenwart eines Reaktionshilfsmittels und gegebenenfalls in Gegenwart eines Verdünnungsmittels umsetzt,
   oder wenn man
(c) Sulfonsäurechloride der allgemeinen Formel (VI) 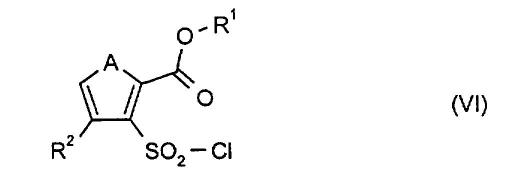 in welcher
   A, R¹ und R² die oben angegebene Bedeutung haben,
      mit Heterocyclen der allgemeinen Formel (V)

      H-R³ (V)

      in welcher
      - R³: die oben angegebene Bedeutung hat,
      und Metall(thio)cyanaten der allgemeinen Formel (VII)

      MQCN (VII)

      in welcher
      - Q: die oben angegebene Bedeutung hat,
      gegebenenfalls in Gegenwart eines Reaktionshilfsmittels und gegebenenfalls in Gegenwart eines Verdünnungsmittels umsetzt,
   oder wenn man
(c) Sulfonsäurechloride der allgemeinen Formel (VI) 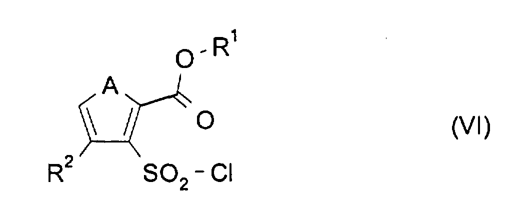 in welcher
   A, R¹ und R² die oben angegebene Bedeutung haben,
      mit (Thio)Carbonsäureamiden der allgemeinen Formel (VIII) 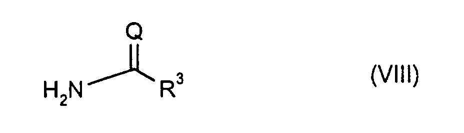 in welcher
   Q und R³ die oben angegebene Bedeutung haben,
   gegebenenfalls in Gegenwart eines Säureakzeptors und gegebenenfalls in Gegenwart eines Verdünnungsmittels umsetzt,
   oder wenn man
(d) Sulfonylamino(thio)carbonylverbindungen der allgemeinen Formel (IX) 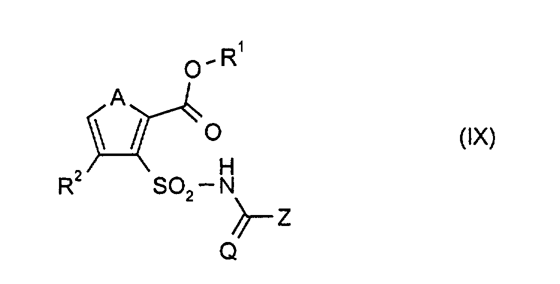
in welcher
A, Q, R¹ und R² die oben angegebene Bedeutung haben und
   - Z: für Halogen, Alkoxy, Aryloxy oder Arylalkoxy steht,
   mit Heterocyclen der allgemeinen Formel (V)

   H-R³ (V)

   in welcher
   - R³: die oben angegebene Bedeutung hat,
gegebenenfalls in Gegenwart eines Säureakzeptors und gegebenenfalls in Gegenwart eines Verdünnungsmittels umsetzt,
und gegebenenfalls die nach den Verfahren (a), (b), (c), (d) oder (e) erhaltenen Verbindungen der Formel (I) nach üblichen Methoden in Salze überführt.

Die substituierten Sulfonylamino(thio)carbonylverbindungen der allgemeinen Formel (I) zeichnen sich durch starke herbizide Wirksamkeit aus.

Gegenstand der Erfindung sind weiter vorzugsweise Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium, Tri-(C₁-C₄-alkyl)-sulfonium-, C₅- oder C₆-Cycloalkyl-ammonium- und Di-(C₁-C₂-alkyl)-benzyl-ammonium-Salze von Verbindungen der Formel (I), in welcher A, Q, R¹, R² und R³ die oben vorzugsweise angegebene Bedeutung haben.

Gegenstand der Erfindung sind insbesondere Verbindungen der Formel (I), in welcher
- R¹: für jeweils gegebenenfalls durch Cyano, Fluor, Chlor, Methoxy oder Ethoxy substituiertes Methyl, Ethyl, n- oder i-Propyl, n-, i- oder s-Butyl, für jeweils gegebenenfalls durch Cyano, Fluor oder Chlor substituiertes Propenyl, Butenyl, Propinyl oder Butinyl, für jeweils gegebenenfalls durch Cyano, Fluor, Chlor, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclo-hexylmethyl, für jeweils gegebenenfalls durch Cyano, Fluor, Chlor, Brom, Methyl, Ethyl, Trifluormethyl, Methoxy, Ethoxy, Difluormethoxy oder Trifluormethoxy substituiertes Phenyl, Phenylmethyl oder Phenylethyl, oder für jeweils gegebenenfalls durch Cyano, Fluor, Chlor, Brom, Methyl, Ethyl, Methoxy oder Ethoxy substituiertes Heterocyclyl oder Heterocyclyl-methyl steht, wobei jeweils die Heterocyclylgruppe aus der Reihe Oxetanyl, Thietanyl, Furyl, Tetrahydrofuryl, Thienyl, Tetrahydrothienyl ausgewählt ist,
- R²: für Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i- oder s-Butyl, Methoxy, Ethoxy, n- oder i-Propoxy, Methoxycarbonyl, Ethoxycarbonyl, n- oder i-Propoxycarbonyl, Methylthio, Ethylthio, Methylsulfinyl, Ethylsulfinyl, Methylsulfonyl oder Ethylsulfonyl, oder für jeweils gegebenenfalls durch Cyano, Fluor oder Chlor substituiertes Propenyl, Butenyl, Propinyl, Butinyl, Propenyloxy oder Propinyloxy steht, und
- R³: für gegebenenfalls substituiertes Heterocyclyl der nachstehenden Formel steht, 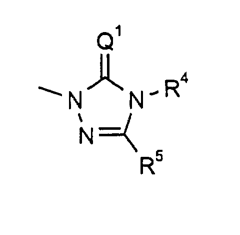
- R⁴: für Wasserstoff, Hydroxy, Amino, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylamino, Ethylamino, n- oder i-Propylamino, n-, i-, s- oder t-Butylamino, für Propenyloxy oder Butenyloxy, für Dimethylamino oder Diethylamino, für jeweils gegebenenfalls durch Fluor, Chlor, Methyl und/oder Ethyl substituiertes Cyclopropyl, Cyclobutyl, Cyclopentyl, Cyclohexyl, Cyclopropylamino, Cyclobutylamino, Cyclopentylamino, Cyclohexylamino, Cyclopropylmethyl, Cyclobutylmethyl, Cyclopentylmethyl oder Cyclohexylmethyl, oder für jeweils gegebenenfalls durch Fluor, Chlor, Methyl, Trifluormethyl und/oder Methoxy substituiertes Phenyl oder Benzyl steht
- R⁵: für Wasserstoff, Hydroxy, Mercapto, Amino, Fluor, Chlor, Brom, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Ethenyl, Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, für Propenyloxy, Butenyloxy, Propinyloxy, Butinyloxy, Propenylthio, Propadienylthio Butenylthio, Propinylthio, Butinylthio, Propenylamino, Butenylamino, Propinylamino oder Butinylamino, für Dimethylamino, Diethylamino oder Dipropylamino, für jeweils gegebenenfalls durch Fluor, Chlor, Methyl und/oder Ethyl substituiertes Cyclopropyl, Cyclobutyl, Cyclopentyl, Cyclohexyl, Cyclopentenyl, Cyclohexenyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopropylmethylthio, Cyclobutylmethylthio, Cyclopentylmethylthio, Cyclohexylmethylthio, Cyclopropylmethylamino, Cyclobutylmethylamino, Cyclopentylmethylamino oder Cyclohexylmethylamino, oder für jeweils gegebenenfalls durch Fluor, Chlor, Methyl, Trifluormethyl, Methoxy und/oder Methoxycarbonyl substituiertes Phenyl, Benzyl, Phenoxy, Benzyloxy, Phenylthio, Benzylthio, Phenylamino oder Benzylamino steht, oder
- R⁴ und R⁵: zusammen für gegebenenfalls verzweigtes Alkandiyl mit 3 bis 11 Kohlenstoffatomen stehen.

Eine ganz besonders bevorzugte Gruppe erfindungsgemäßer Verbindungen sind die Verbindungen der Formel (I), in welcher
- R¹: für Methyl, Ethyl, n- oder i-Propyl, 2-Cyano-ethyl, 2-Fluor-ethyl, 2,2-Difluorethyl, 2,2,2-Trifluor-ethyl, 2-Chlor-ethyl, 2,2-Dichlor-ethyl, 2,2,2-Trichlorethyl, 2-Methoxy-ethyl, 2-Ethoxy-ethyl oder Oxetanyl steht,
- R²: für Fluor, Chlor, Brom, Methyl, Ethyl, n- oder i-Propyl, n-, i- oder s-Butyl, Trifluormethyl, Methoxy, Ethoxy, n- oder i-Propoxy, Difluormethoxy, Trifluormethoxy oder Trifluorethoxy steht, und
- R³: für gegebenenfalls substituiertes Triazolinyl der nachstehenden Formel steht, 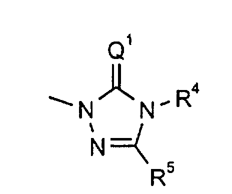 worin
Q¹ für Sauerstoff oder Schwefel steht sowie
- R⁴: für jeweils gegebenenfalls durch Fluor, Chlor, Methoxy oder Ethoxy substituiertes Methyl, Ethyl, n- oder i-Propyl, für jeweils gegebenenfalls durch Fluor oder Chlor substituiertes Propenyl oder Propinyl, für Methoxy, Ethoxy, n- oder i-Propoxy, Methylamino, Ethylamino, n- oder i-Propylamino, für Propenyloxy, für Dimethylamino oder für Cyclopropyl steht,
- R⁵: für Chlor oder Brom, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Ethenyl, Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, für Propenyloxy, Butenyloxy, Propinyloxy, Butinyloxy, Propenylthio, Propadienylthio, Butenylthio, Propinylthio, Butinylthio, Propenylamino, Butenylamino, Propinylamino oder Butinylamino, für Dimethylamino, Diethylamino oder Dipropylamino, für jeweils gegebenenfalls durch Fluor, Chlor, Methyl und/oder Ethyl substituiertes Cyclopropyl, Cyclobutyl, Cyclopentyl, Cyclohexyl, Cyclopentenyl, Cyclohexenyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopropylmethylthio, Cyclobutylmethylthio, Cyclopentylmethylthio, Cyclohexylmethylthio, Cyclopropylmethylamino, Cyclobutylmethylamino, Cyclopentylmethylamino oder Cyclohexylmethylamino, oder für jeweils gegebenenfalls durch Fluor, Chlor, Methyl, Trifluormethyl, Methoxy und/oder Methoxycarbonyl substituiertes Phenyl, Benzyl, Phenoxy, Benzyloxy, Phenylthio, Benzylthio, Phenylamino oder Benzylamino steht, oder
- R⁴ und R⁵: zusammen für gegebenenfalls verzweigtes Alkandiyl mit 3 bis 11 Kohlenstoffatomen stehen.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangsstoffe bzw. Zwischenprodukte. Diese Restedefinitionen können untereinander, also auch zwischen den angegebenen bevorzugten Bereichen beliebig kombiniert werden.

Verwendet man beispielsweise 2-Fluor-6-methoxycarbonyl-benzolsulfonamid und 5-Ethoxy-4-methyl-2-phenoxycarbonyl-2,4-dihydro-3H-1,2,4-triazol-3-thion als Ausgangsstoffe, so kann der Reaktionsablauf beim erfindungsgemäßen Verfahren (a) durch das folgende Formelschema skizziert werden:

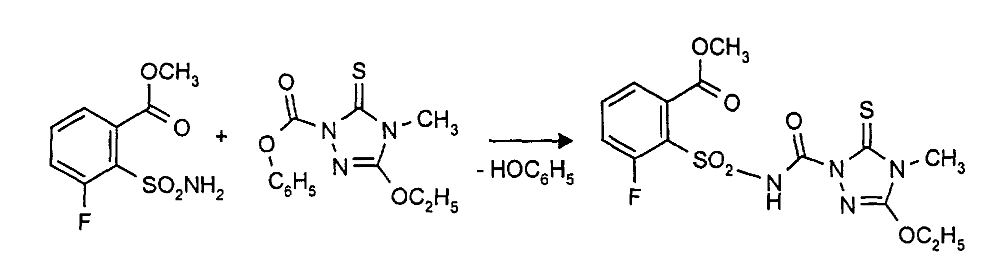

Verwendet man beispielsweise 4-Chlor-2-ethoxycarbonyl-3-thienylsulfonyl-isothiocyanat und 5-Ethyl-4-methoxy-2,4-dihydro-3H-1,2,4-triazol-3-on als Ausgangsstoffe, so kann der Reaktionsablauf beim erfindungsgemäßen Verfahren (b) durch das folgende Formelschema skizziert werden:

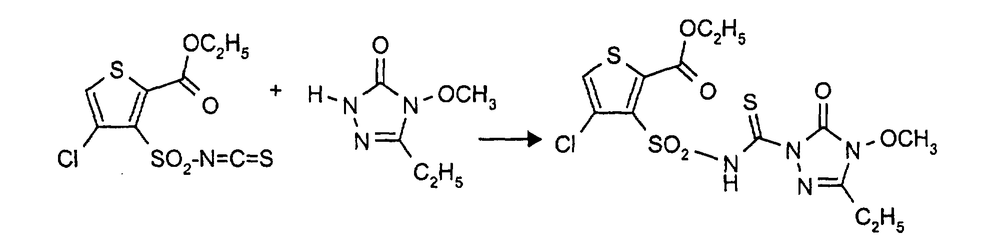

Verwendet man beispielsweise 2-Methoxycarbonyl-6-methyl-benzolsulfochlorid, 5-Ethylthio-4-methoxy-2,4-dihydro-3H-1,2,4-triazol-3-on und Kaliumcyanat als Ausgangsstoffe, so kann der Reaktionsablauf beim erfindungsgemäßen Verfahren (c) durch das folgende Formelschema skizziert werden:

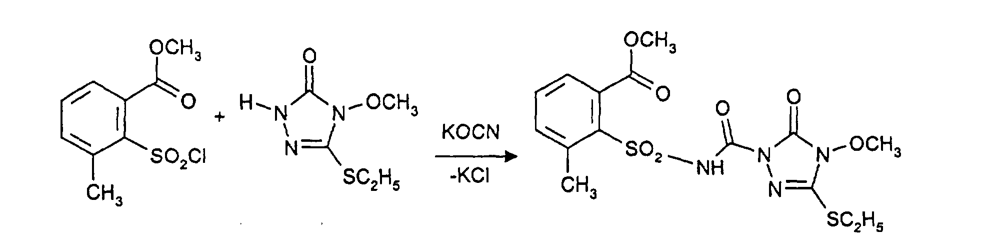

Verwendet man beispielsweise N-(2-Chlor-6-methoxycarbonyl-phenylsulfonyl)-O-methyl-urethan und 4-Methyl-5-methylthio-2,4-dihydro-3H-1,2,4-triazol-3-on als Ausgangsstoffe, so kann der Reaktionsablauf beim erfindungsgemäßen Verfahren (e) durch das folgende Formelschema skizziert werden:

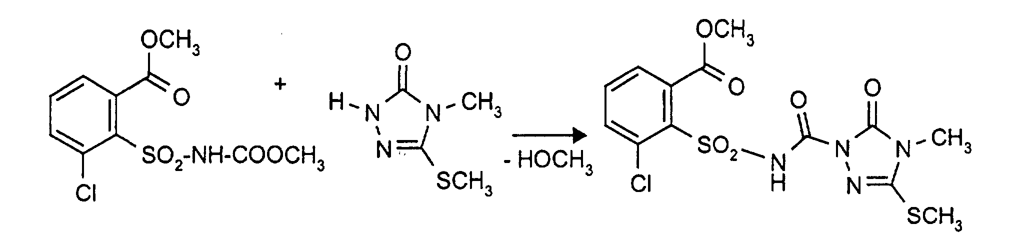

Die beim erfindungsgemäßen Verfahren (a) zur Herstellung der Verbindungen der Formel (I) als Ausgangsstoffe zu verwendenden Sulfonamide sind durch die Formel (II) allgemein definiert. In der Formel (II) haben A, R¹ und R² vorzugsweise bzw. insbesondere diejenige Bedeutung, die bereits oben bei der Beschreibung der erfindungsgemäßen Verbindungen der Formel (I) vorzugsweise bzw. als insbesondere bevorzugt für A, R¹ und R² angegeben wurde.

Die Ausgangsstoffe der Formel (II) sind bekannt und/oder können nach an sich bekannten Verfahren hergestellt werden (vgl. US 4546179, US 5084086, US 5157119, WO 8909214, WO 9115478, US 4877440).

Noch nicht aus der Literatur bekannt und als neue Stoffe Gegenstand der vorliegenden Anmeldung sind die Sulfonamide der allgemeinen Formel (IIa) 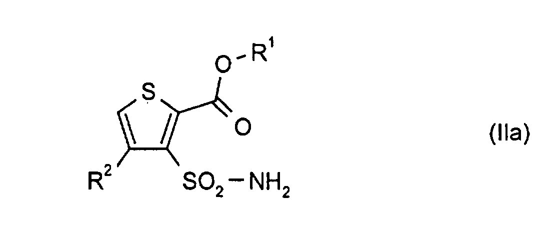 in welcher
- R¹: für jeweils gegebenenfalls durch Cyano, Fluor, Chlor, Methoxy oder Ethoxy substituiertes Methyl, Ethyl, n- oder i-Propyl, n-, i- oder s-Butyl, für jeweils gegebenenfalls durch Cyano, Fluor oder Chlor substituiertes Propenyl, Butenyl, Propinyl oder Butinyl, für jeweils gegebenenfalls durch Cyano, Fluor, Chlor, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclo-hexylmethyl, für jeweils gegebenenfalls durch Cyano, Fluor, Chlor, Brom, Methyl, Ethyl, Trifluormethyl, Methoxy, Ethoxy, Difluormethoxy oder Trifluormethoxy substituiertes Phenyl, Phenylmethyl oder Phenylethyl, oder für jeweils gegebenenfalls durch Cyano, Fluor, Chlor, Brom, Methyl, Ethyl, Methoxy oder Ethoxy substituiertes Heterocyclyl oder Heterocyclyl-methyl steht, wobei jeweils die Heterocyclylgruppe aus der Reihe Oxetanyl, Thietanyl, Furyl, Tetrahydrofuryl, Thienyl, Tetrahydrothienyl ausgewählt ist, und
- R²: für Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i- oder s-Butyl, Methoxy, Ethoxy, n- oder i-Propoxy, Methoxycarbonyl, Ethoxycarbonyl, n- oder i-Propoxycarbonyl, Methylsulfinyl, Ethylsulfinyl, Methylsulfonyl oder Ethylsulfonyl, oder für jeweils gegebenenfalls durch Cyano, Fluor oder Chlor substituiertes Propenyl, Butenyl, Propinyl, Butinyl, Propenyloxy oder Propinyloxy steht.

Man erhält die neuen Sulfonamide der Formel (IIa), wenn man Sulfonsäurechloride der Formel (VIa) 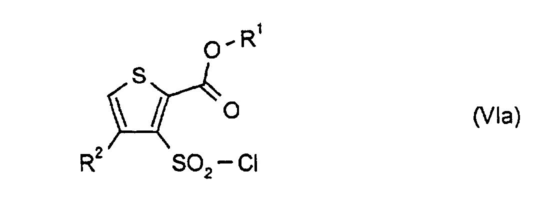 in welcher.
R¹ und R² die oben angegebene Bedeutung haben,
mit Ammoniak gegebenenfalls in Gegenwart eines Verdünnungsmittels, wie z.B. Wasser bei Temperaturen zwischen 0°C und 50°C umsetzt (vgl. die Herstellungsbeispiele).

Die Sulfonsäurechloride der Formel (VIa) sind noch nicht aus der Literatur bekannt; sie sind als neue Stoffe auch Gegenstand der vorliegenden Anmeldung.

Man erhält die neuen Sulfochloride der Formel (VIa), wenn man entsprechende Aminoverbindungen der allgemeinen Formel (X) 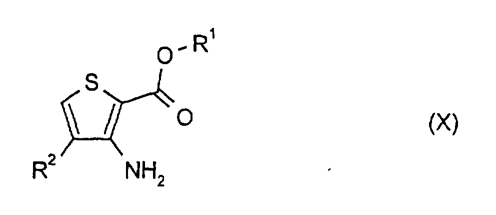 in welcher
R¹ und R² die oben angegebene Bedeutung haben,
mit einem Alkalimetallnitrit, wie z.B. Natriumnitrit, in Gegenwart von Salzsäure bei Temperaturen zwischen -10°C und +10°C umsetzt und die so erhaltene Diazoniumsalzlösung mit Schwefeldioxid in Gegenwart eines Verdünnungsmittels, wie z.B. Dichlormethan, 1,2-Dichlor-ethan oder Essigsäure, und in Gegenwart eines Katalysators, wie z.B. Kupfer(I)-chlorid und/oder Kupfer(II)-chlorid, bei Temperaturen zwischen -10°C und +50°C umsetzt (vgl. die Herstellungsbeispiele).

Die als Vorprodukte benötigten Aminoverbindungen der Formel (X) sind bekannt und/oder können nach an sich bekannten Verfahren hergestellt werden (vgl. DE 3018134, DE 3804794, EP 298542, Herstellungsbeispiele).

Die beim erfindungsgemäßen Verfahren (a) zur Herstellung der Verbindungen der Formel (I) weiter als Ausgangsstoffe zu verwendenden (Thio)Carbonsäurederivate sind durch die Formel (III) allgemein definiert. In der Formel (III) haben Q und R³ vorzugsweise bzw. insbesondere diejenige Bedeutung, die bereits oben bei der Beschreibung der erfindungsgemäßen Verbindungen der Formel (I) vorzugsweise bzw. als insbesondere bevorzugt für Q und R³ angegeben wurde; Z steht vorzugsweise für Fluor, Chlor, Brom, C₁-C₄-Alkoxy, Phenoxy oder Benzyloxy, insbesondere für Chlor, Methoxy, Ethoxy oder Phenoxy.

Die Ausgangsstoffe der Formel (III) sind bekannt und/oder können nach an sich bekannten Verfahren hergestellt werden (vgl. EP 459244, EP 341489, EP 422469, EP 425948, EP 431291, EP 507171, EP 534266).

Die beim erfindungsgemäßen Verfahren (b) zur Herstellung der Verbindungen der Formel (I) als Ausgangsstoffe zu verwendenden Sulfonyliso(thio)cyanate sind durch die Formel (IV) allgemein definiert. In der Formel (IV) haben A, Q, R¹ und R² vorzugsweise bzw. insbesondere diejenige Bedeutung, die bereits oben bei der Beschreibung der erfindungsgemäßen Verbindungen der Formel (I) vorzugsweise bzw. als insbesondere bevorzugt für A, Q, R¹ und R² angegeben wurde.

Die Ausgangsstoffe der Formel (IV) sind bekannt und/oder können nach an sich bekannten Verfahren hergestellt werden (vgl. EP 46626, US 4701535, Herstellungsbeispiele).

Die bei den erfindungsgemäßen Verfahren (b), (c) und (e) zur Herstellung der Verbindungen der Formel (I) als Ausgangsstoffe zu verwendenden Heterocyclen sind durch die Formel (V) allgemein definiert. In der Formel (V) hat R³ vorzugsweise bzw. insbesondere diejenige Bedeutung, die bereits oben bei der Beschreibung der erfindungsgemäßen Verbindungen der Formel (I) vorzugsweise bzw. als insbesondere bevorzugt für R³ angegeben wurde.

Die Ausgangsstoffe der Formel (V) sind bekannt und/oder können nach an sich bekannten Verfahren hergestellt werden (vgl. EP 341489, EP 422469, EP 425948, EP 431291, EP 507171, EP 534266).

Die bei den erfindungsgemäßen Verfahren (c) und (d) zur Herstellung der Verbindungen der Formel (I) als Ausgangsstoffe zu verwendenden Sulfonsäurechloride sind durch die Formel (VI) allgemein definiert. In der Formel (VI) haben A, R¹ und R² vorzugsweise bzw. insbesondere diejenige Bedeutung, die bereits oben bei der Beschreibung der erfindungsgemäßen Verbindungen der Formel (I) vorzugsweise bzw. als insbesondere bevorzugt für A, R¹ und R² angegeben wurde.

Die Ausgangsstoffe der Formel (VI) sind bekannt und/oder können nach an sich bekannten Verfahren hergestellt werden (vgl. US 4546179, US 5084086, US 5157119, WO 8909214, WO 9115478, WO 9213845, Herstellungsbeispiele). Neu sind jedoch, wie bereits angegeben, die Verbindungen der Formel (IV) mit A = S, d.h. die Untergruppe der Formel (IVa).

Die beim erfindungsgemäßen Verfahren (d) zur Herstellung der Verbindungen der Formel (I) als Ausgangsstoffe zu verwendenden (Thio)Carbonsäureamide sind durch die Formel (VIII) allgemein definiert. In der Formel (VIII) haben Q und R³ vorzugsweise bzw. insbesondere diejenige Bedeutung, die bereits oben bei der Beschreibung der erfindungsgemäßen Verbindungen der Formel (I) vorzugsweise bzw. als insbesondere bevorzugt für Q und R³ angegeben wurde.

Die Ausgangsstoffe der Formel (VIII) sind bekannt und/oder können nach an sich bekannten Verfahren hergestellt werden (vgl. EP 459244).

Die beim erfindungsgemäßen Verfahren (e) zur Herstellung der Verbindungen der Formel (I) als Ausgangsstoffe zu verwendenden Sulfonylamino(thio)carbonylverbindungen sind durch die Formel (IX) allgemein definiert. In der Formel (IX) haben A, Q, R¹ und R² vorzugsweise bzw. insbesondere diejenige Bedeutung, die bereits oben bei der Beschreibung der erfindungsgemäßen Verbindungen der Formel (I) vorzugsweise bzw. als insbesondere bevorzugt für A, Q, R¹ und R² angegeben wurde; Z steht vorzugsweise für Fluor, Chlor, Brom, C₁-C₄-Alkoxy, Phenoxy oder Benzyloxy, insbesondere für Chlor, Methoxy, Ethoxy oder Phenoxy.

Die Ausgangsstoffe der Formel (IX) sind bekannt und/oder können nach an sich bekannten Verfahren hergestellt werden.

Die erfindungsgemäßen Verfahren (a), (b), (c), (d) und (e) zur Herstellung der neuen Verbindungen der Formel (I) werden vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlormethan, Chlorbenzol und o-Dichlorbenzol; Ether wie Diethyl- und Dibutylether, Glykoldimethylether und Diglykoldimethylether, Tetrahydrofuran und Dioxan; Ketone wie Aceton, Methylethyl-, Methyl-isopropyl- und Methyl-isobutyl-keton; Ester wie Essigssäuremethylester und -ethylester; Nitrile wie z.B. Acetonitril und Propionitril; Amide wie z.B. Dimethylformamid, Dimethylacetamid und N-Methylpyrrolidon sowie Dimethylsulfoxid, Tetramethylensulfon und Hexamethylphosphorsäuretriamid.

Als Reaktionshilfsmittel bzw. als Säureakzeptoren können bei den erfindungsgemäßen Verfahren (a), (b), (c), (d) und (e) alle üblicherweise für derartige Umsetzungen verwendbaren Säurebindemittel eingesetzt werden. Vorzugsweise in Frage kommen Alkalimetallhydroxide wie z.B. Natrium- und Kaliumhydroxid, Erdalkalihydroxide wie z.B. Calciumhydroxid, Alkalicarbonate und -alkoholate wie Natrium- und Kaliumcarbonat, Natrium- und Kalium-tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methylpyridin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei den erfindungsgemäßen Verfahren (a), (b), (c), (d) und (e) in einem größeren Bereich variiert werden. Im allgemeinen arbeitet man bei Temperaturen zwischen -20°C und +150°C, vorzugsweise bei Temperaturen zwischen 0°C und +100°C.

Die erfindungsgemäßen Verfahren (a), (b), (c), (d) und (e) werden im allgemeinen unter Normaldruck durchgeführt. Es ist jedoch auch möglich, unter erhöhtem oder vermindertem Druck zu arbeiten.

Zur Durchführung der erfindungsgemäßen Verfahren (a), (b), (c), (d) und (e) werden die jeweils benötigten Ausgangsstoffe im allgemeinen in angenähert äquimolaren Mengen eingesetzt. Es ist jedoch auch möglich, eine der jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei den erfindungsgemäßen Verfahren (a), (b), (c), (d) und (e) jeweils nach üblichen Methoden (vgl. die Herstellungsbeispiele).

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z.B. Methylenchlorid, Aceton, tert-Butyl-methylether oder Toluol, und Zugabe einer geeigneten Base. Die Salze können dann - gegebenenfalls nach längerem Rühren - durch Einengen oder Absaugen isoliert werden.

Die erfindungsgemäßen Wirkstoffe können als Defoliants, Desiccants, Krautabtötungsmittel und insbesondere als Unkrautvemichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen sowohl im Vorauflauf- als auch im Nachauflauf-Verfahren. In gewissem Umfang zeigen Verbindungen der Formel (I) auch fungizide Wirkung, beispielsweise gegen Pyricularia oryzae an Reis.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-verbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel I

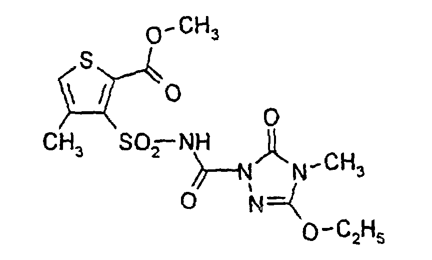

### (Verfahren (a))

Zu einer Lösung von 7,9 g (30 mMol) 5-Ethoxy-4-methyl-2-phenoxycarbonyl-2,4-di-hydra-3H-1,2,4-triazol-3-on in 150 ml Acetonitril gibt man nacheinander 7,5 g (32 mMol) 4-Methyl-2-methoxycarbonyl-thiophen-3-sulfonamid und 4,9 g (32 mMol) 1,8-Diazabicyclo-[5,4,0]-undec-7-en (DBU). Die Reaktionsmischung wird ca. 15 Stunden bei Raumtemperatur (ca. 20°C) gerührt und anschließend im Wasserstrahlvakuum eingeengt. Der Rückstand wird dann in Methylenchlorid aufgenommen, mit IN-Salzsäure und dann mit Wasser gewaschen. Die organische Phase wird mit Magnesiumsulfat getrocknet und filtriert. Das Filtrat wird im Wasserstrahlvakuum eingeengt und der Rückstand aus Isopropanol umkristallisiert.

Man erhält 9,2 g (76% der Theorie) 5-Ethoxy-4-methyl-2-(4-methyl-2-methoxycarbonyl-thien-3-yl-sulfonylaminocarbonyl)-2,4-dihydro-3H-1,2,4-triazol-3-on vom Schmelzpunkt 169°C.

### Beispiel 2

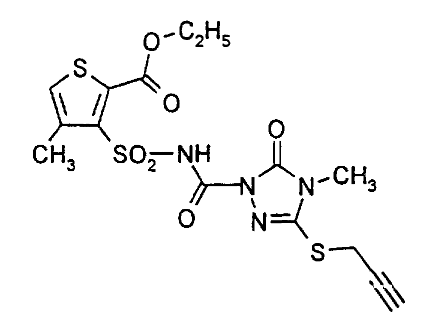

### (Verfahren (b))

2,1 g (8 mMol) 2-Ethoxycarbonyl-4-methyl-thien-3-yl-sulfonylisocyanat werden zu einer Lösung von 1,35 g (8 mMol) 4-Methyl-5-propargylthio-2,4-dihydro-3H-1,2,4-triazol-3-on in 50 ml Acetonitril gegeben. Die Mischung wird dann 8 Stunden unter Rückfluß erhitzt, anschließend im Wasserstrahlvakuum eingeengt, der Rückstand mit Diethylether verrührt und das kristallin angefallene Produkt durch Absaugen isoliert.

Man erhält 1,4 g (40% der Theorie) 4-Methyl-5-propargylthio-2-(2-ethoxycarbonyl-4-methyl-thien-3-yl-sulfonylaminocarbonyl)-2,4-dihydro-3H-1,2,4-triazol-3-on vom Schmelzpunkt 175°C.

### Beispiel 3

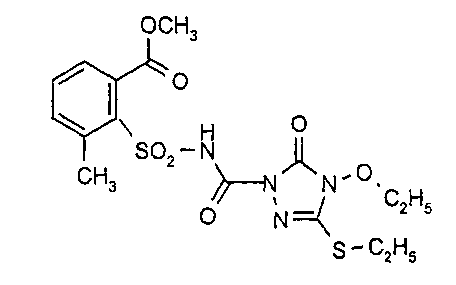

### (Verfahren (c))

Eine Lösung von 3,8 g (20 mMol) 4-Ethoxy-5-ethylthio-2,4-dihydro-3H-1,2,4-triazol-3-on in 50 ml Acetonitril wird mit 6,0 g (24 mMol) 2-Methyl-6-methoxycarbonyl-benzolsulfochlorid, 2,6 g (40 mMol) Natriumcyanat und 1,2 g (15 mMol) Pyridin versetzt und die Reaktionsmischung wird 3 Tage bei Raumtemperatur (ca. 20°C) gerührt. Dann wird mit etwa gleichen Volumenanteilen Methylenchlorid und Wasser auf etwa das dreifache Volumen verdünnt und mit IN-Salzsäure schwach angesäurert (pH ∼ 3). Die organische Phase wird abgetrennt, mit Natriumsulfat getrocknet und filtriert. Das Filtrat wird eingeengt und der Rückstand aus Isopropanol umkristallisiert.

Man erhält 5,0 g (56% der Theorie) 4-Ethoxy-5-ethylthio-2-(2-methyl-6-methoxycarbonyl-phenylsulfonylaminacarbonyl)-2,4-dihydro-3H-1,2,4-triazol-3-on vom Schmelzpunkt 135°C.

### Beispiel 4

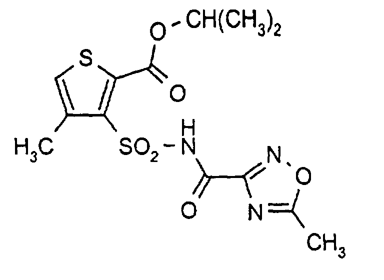

(nicht erfindungsgemäß)

### (Verfahren (d))

2,0 g (36 mMol) Kaliumhydroxid-Pulver werden bei 20°C bis maximal 35°C zu einer Lösung von 1,52 g (12,0 mMol) 5-Methyl-1,2,4-oxadiazol-3-carboxamid in 150 ml Dioxan gegeben. Nach 30 Minuten werden im Wasserstrahlvakuum bei 30°C bis 35°C ca. 50 ml Dioxan abdestilliert. Anschließend wird die Mischung portionsweise mit 3,6 g (12,6 mMol) 4-Methyl-2-i-propoxycarbonyl-thiophen-3-sulfochlorid versetzt und die Reaktionsmischung wird ca. 12 Stunden bei Raumtemperatur (ca. 20°C) gerührt. Anschließend wird im Wasserstrahlvakuum eingeengt, der Rückstand in Wasser aufgenommen und mit 2N-Salzsäure angesäuert. Dann wird zweimal mit je 100 ml Methylenchlorid extrahiert. Die vereinigten organischen Lösungen werden mit Wasser gewaschen, mit Magnesiumsulfat getrocknet und filtriert. Das Filtrat wird im Wasserstrahlvakuum eingeengt und der Rückstand aus Ethanol umkristallisiert.

Man erhält 2,3 g (52% der Theorie) N-(4-Methyl-2-i-propoxycarbonyl-thien-3-yl-sulfonyl)-5-methyl-1,2,4-oxadiazol-3-carboxamid vom Schmelzpunkt 142°C.

Analog zu den Beispielen 1 bis 4 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

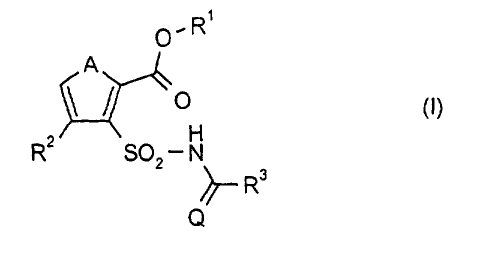

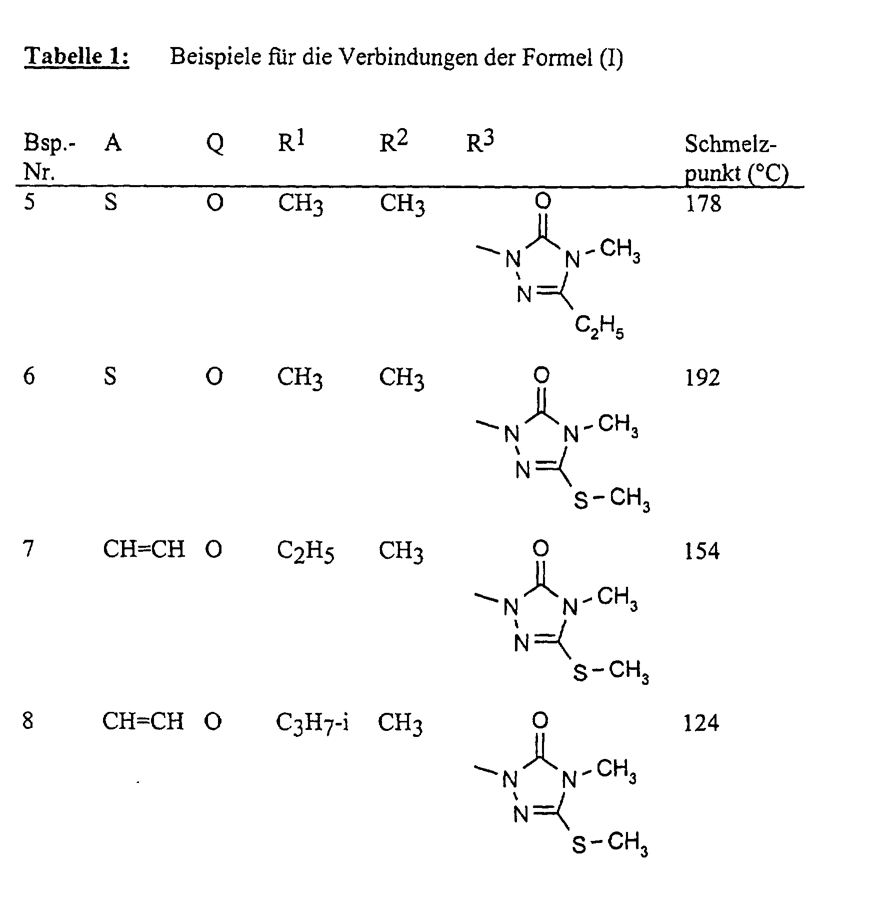

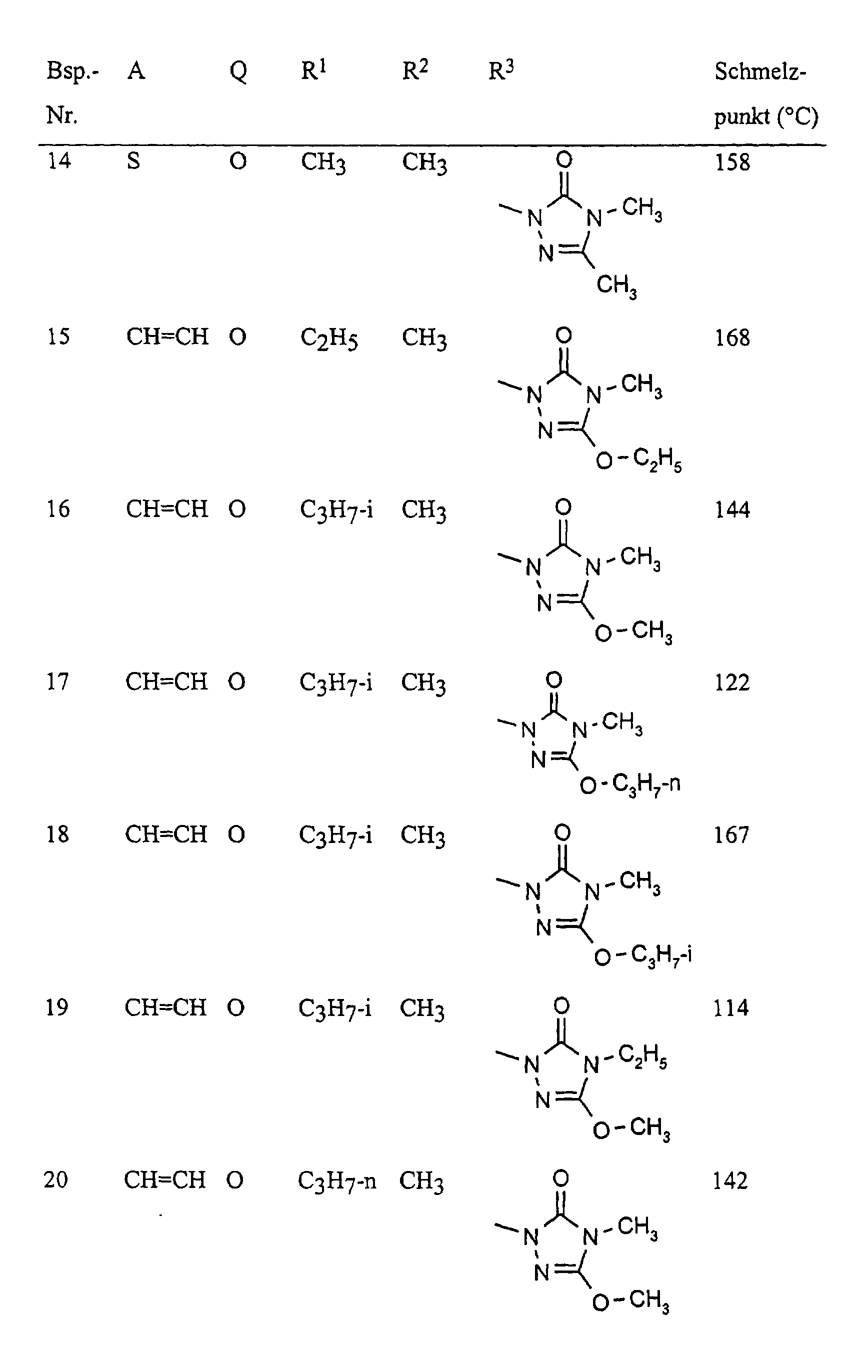

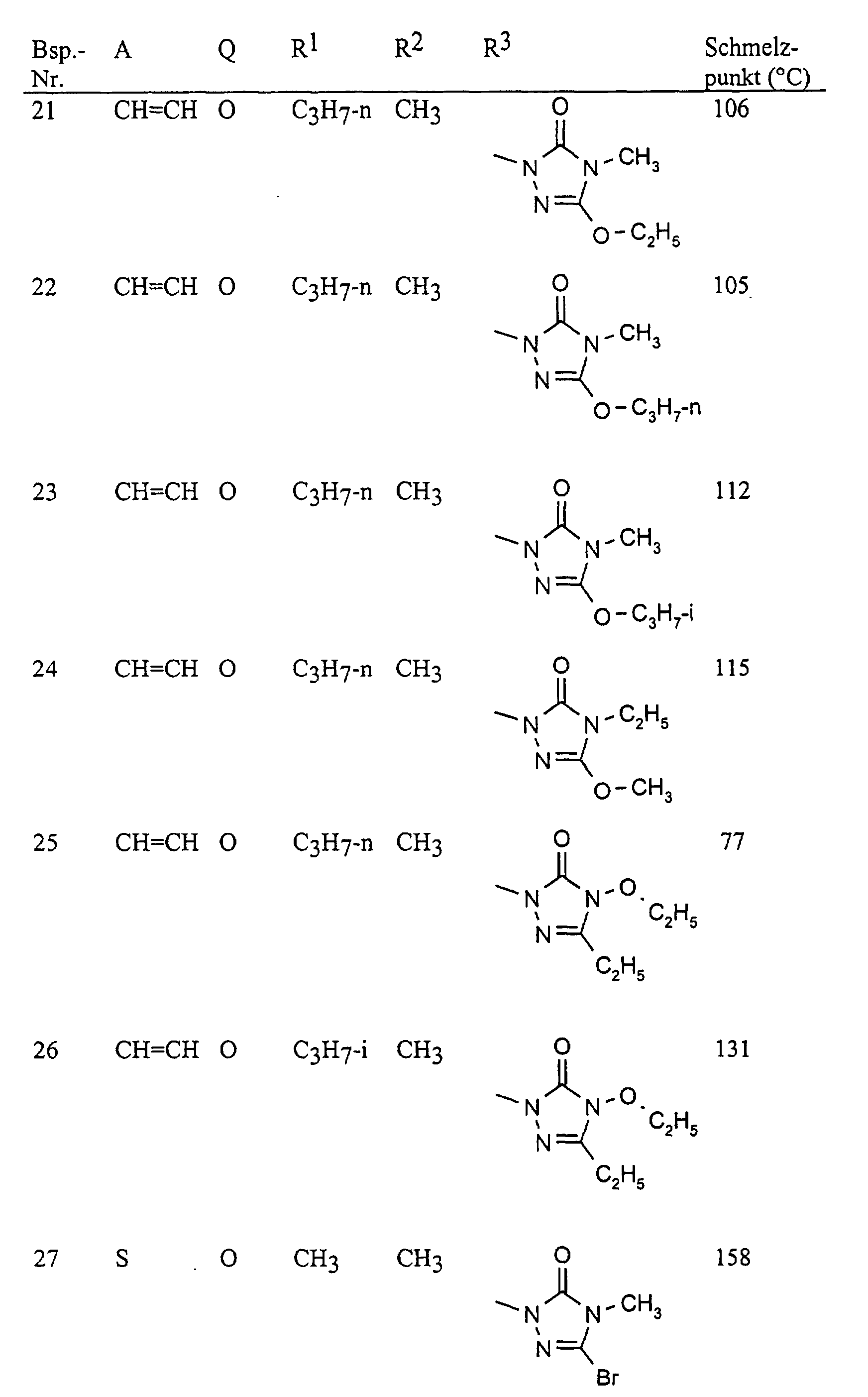

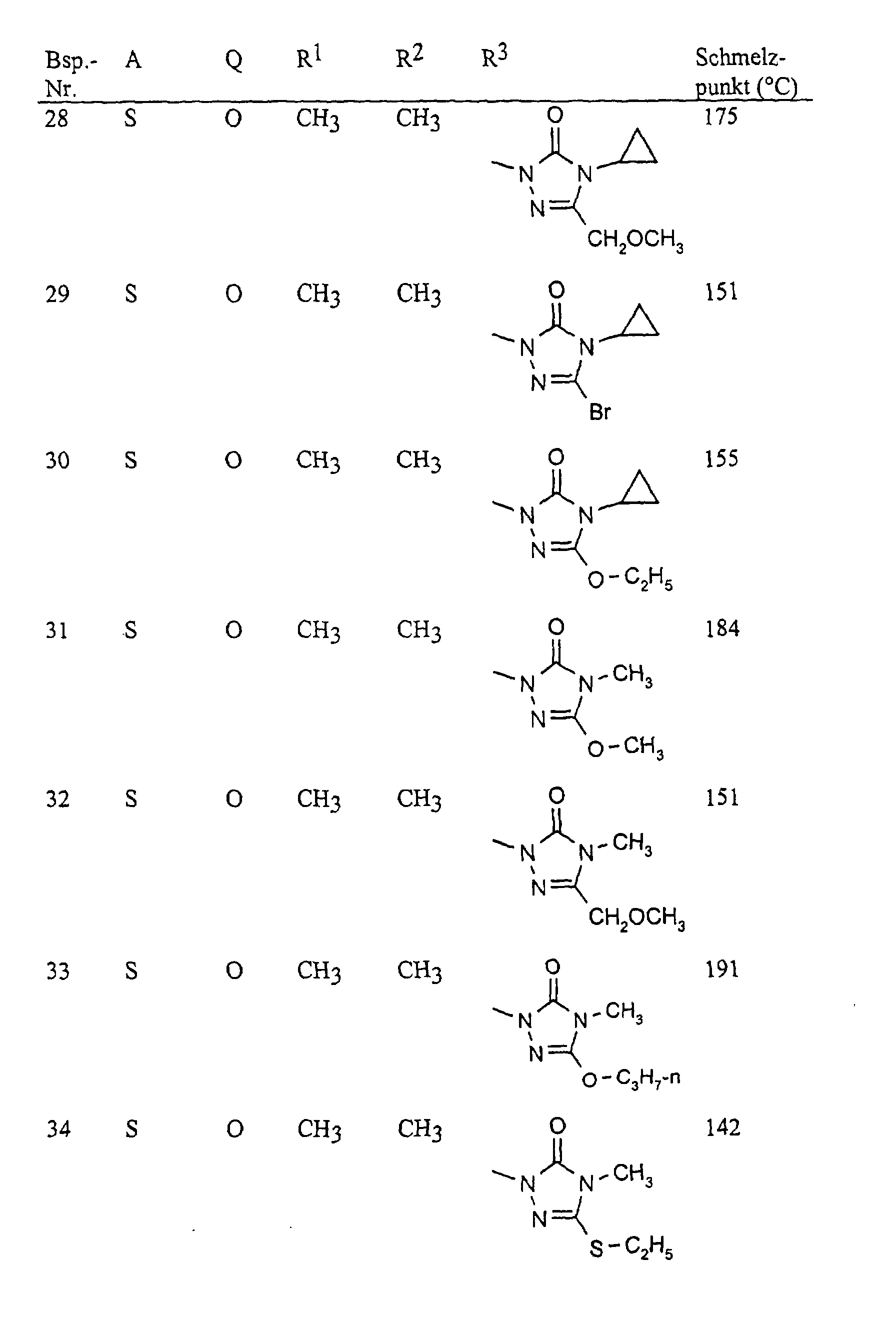

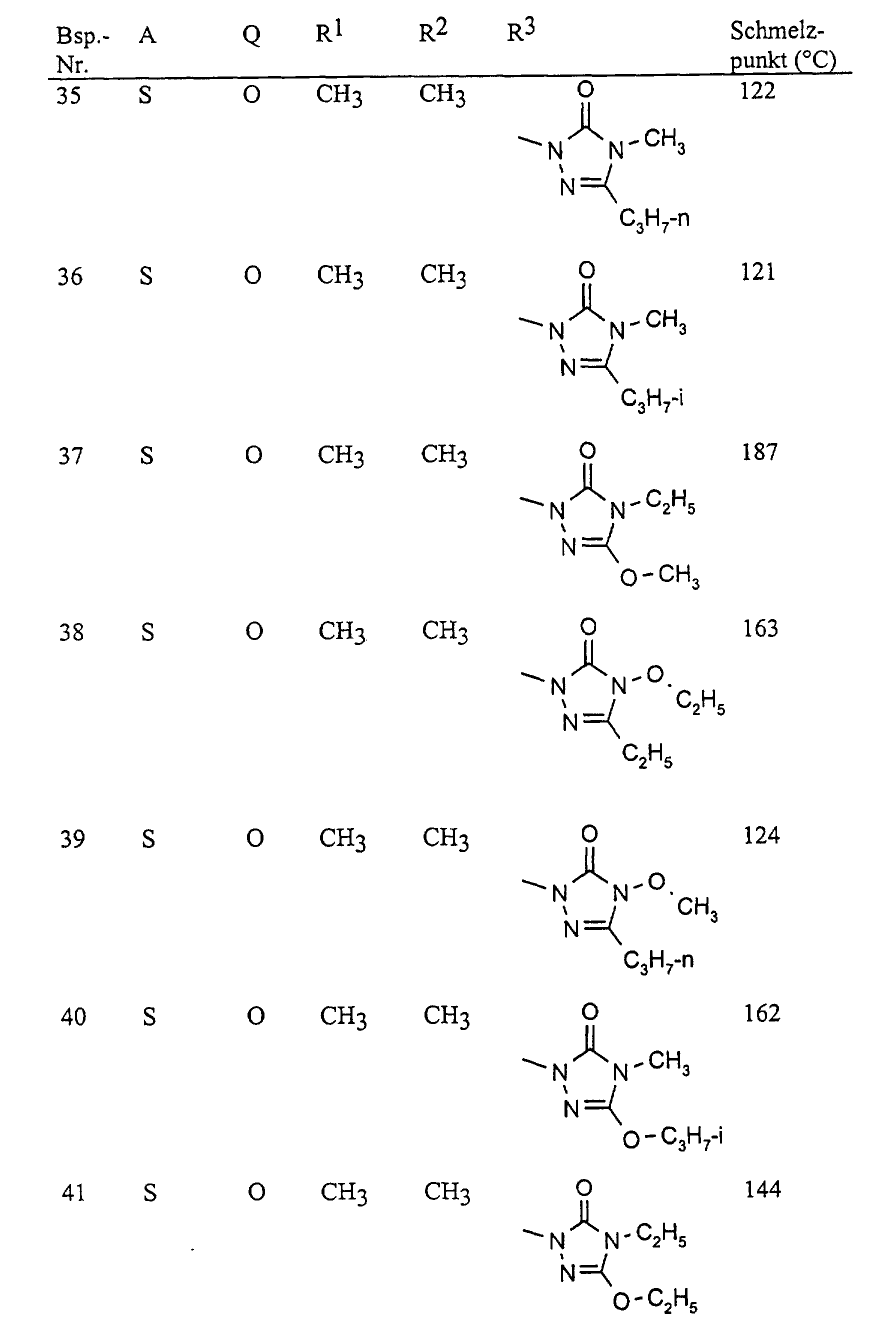

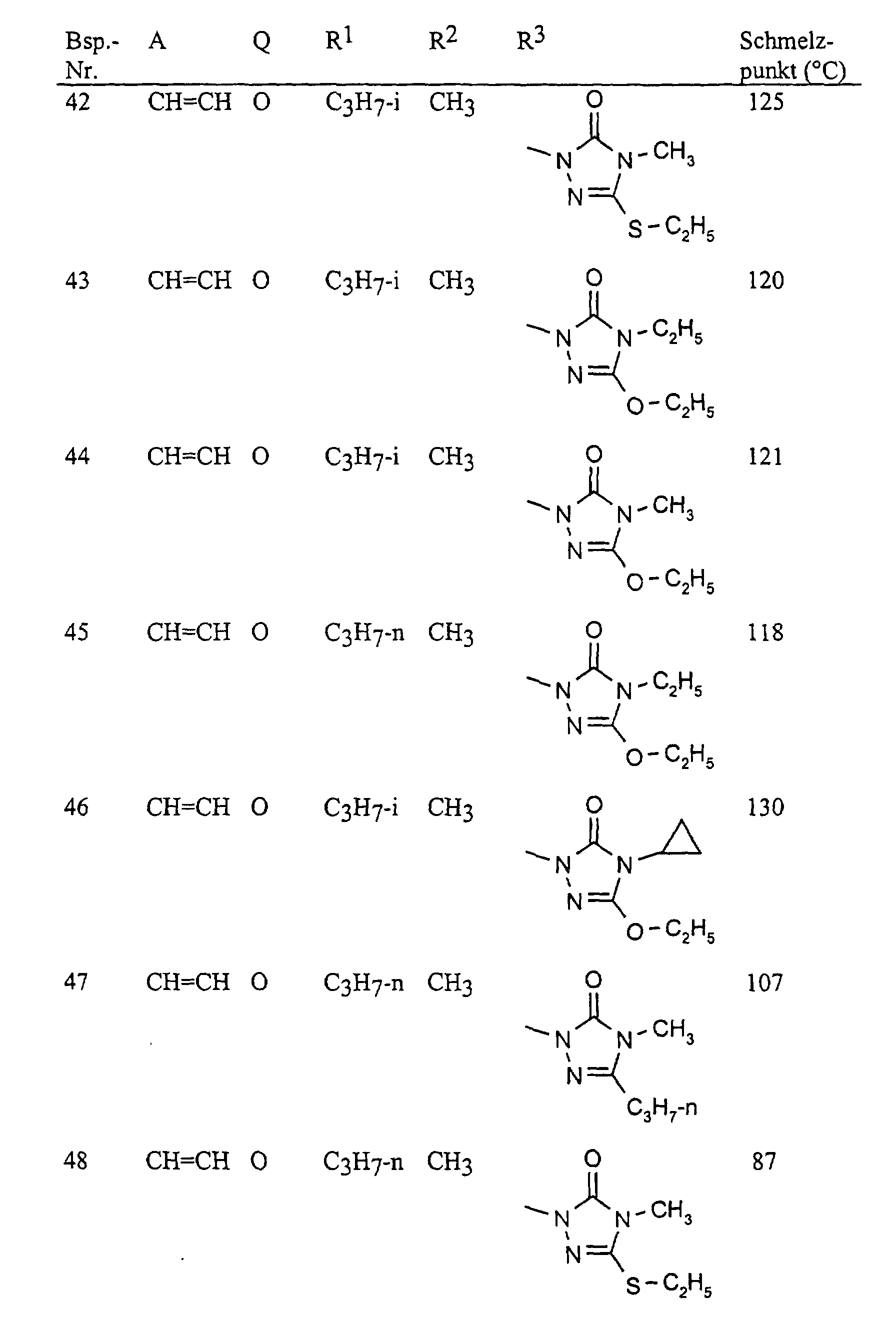

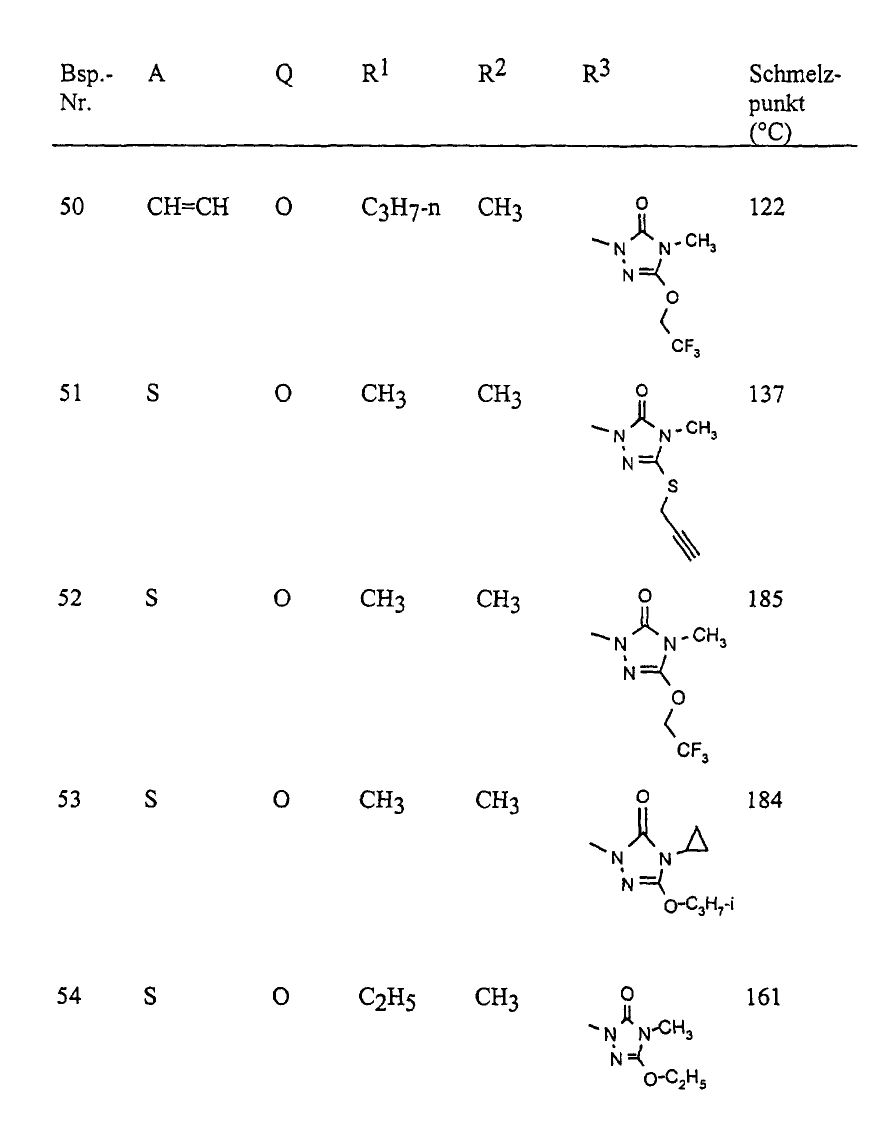

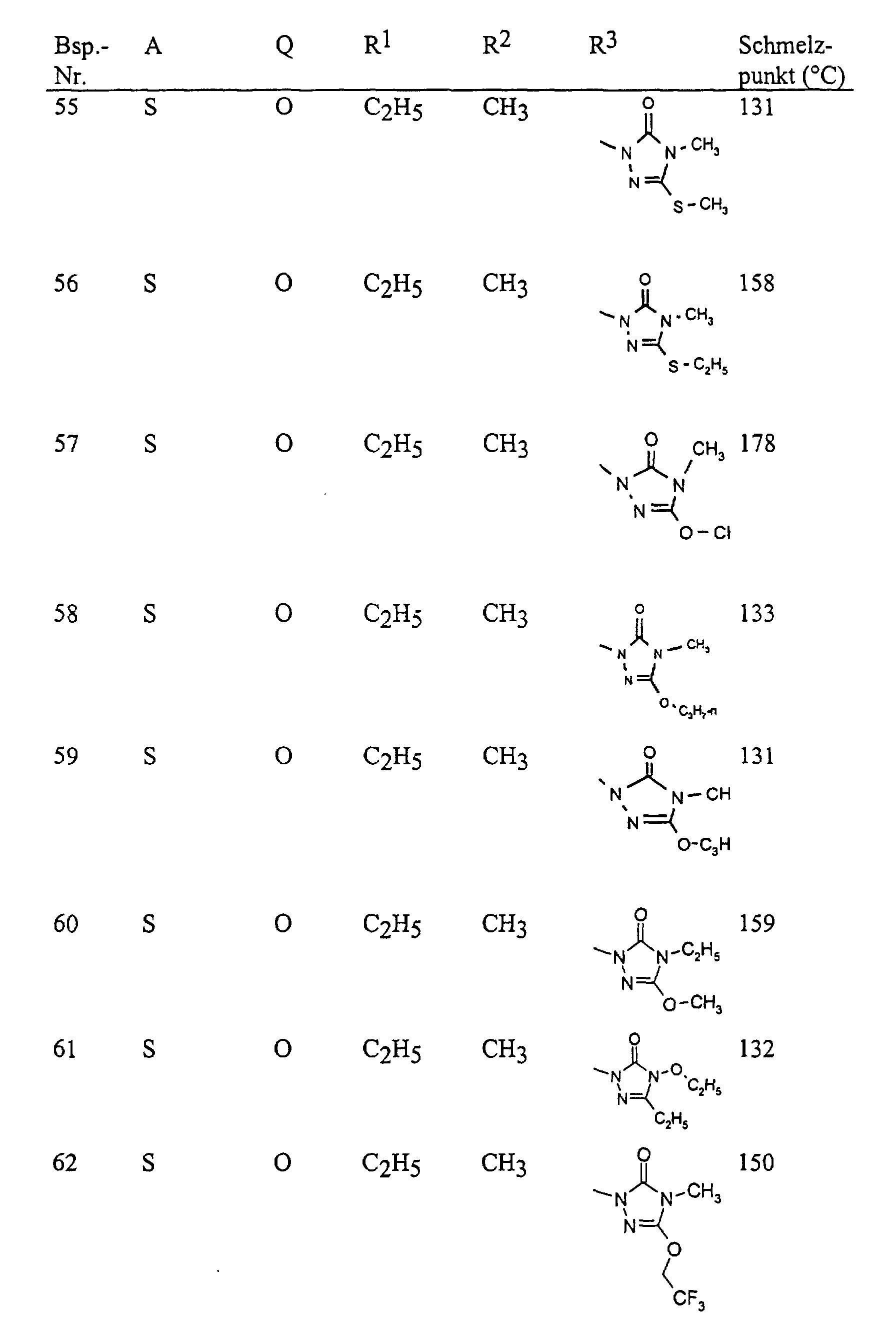

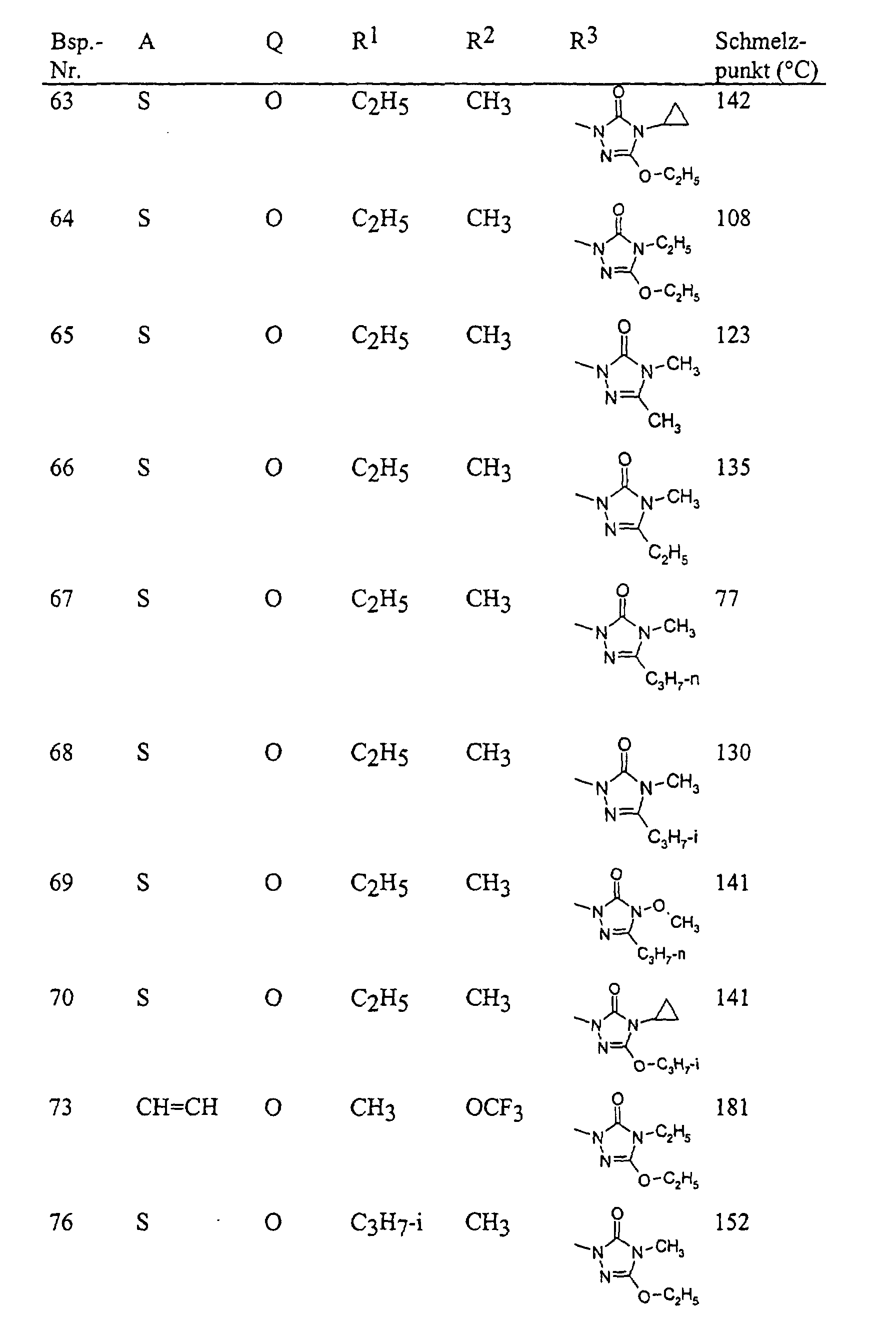

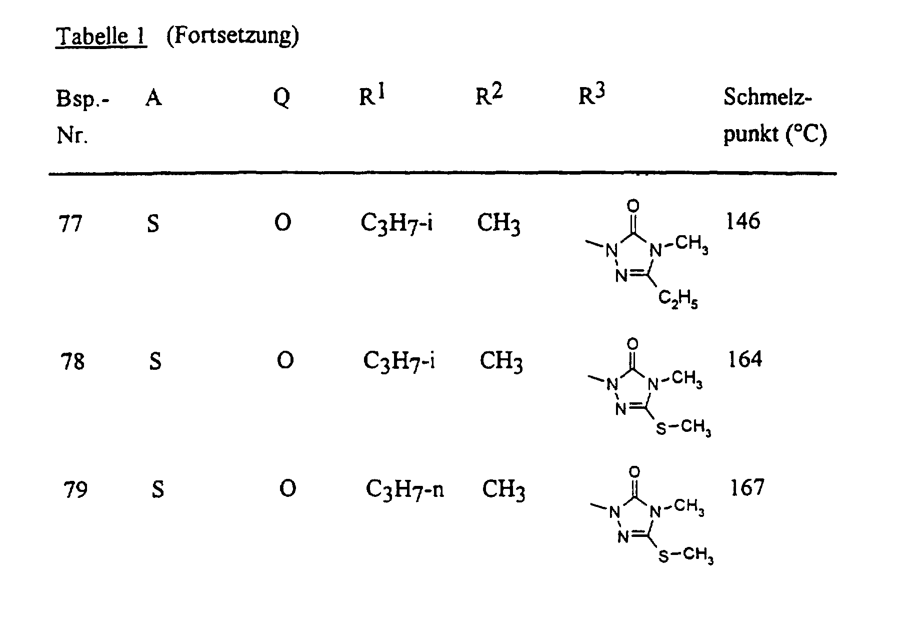

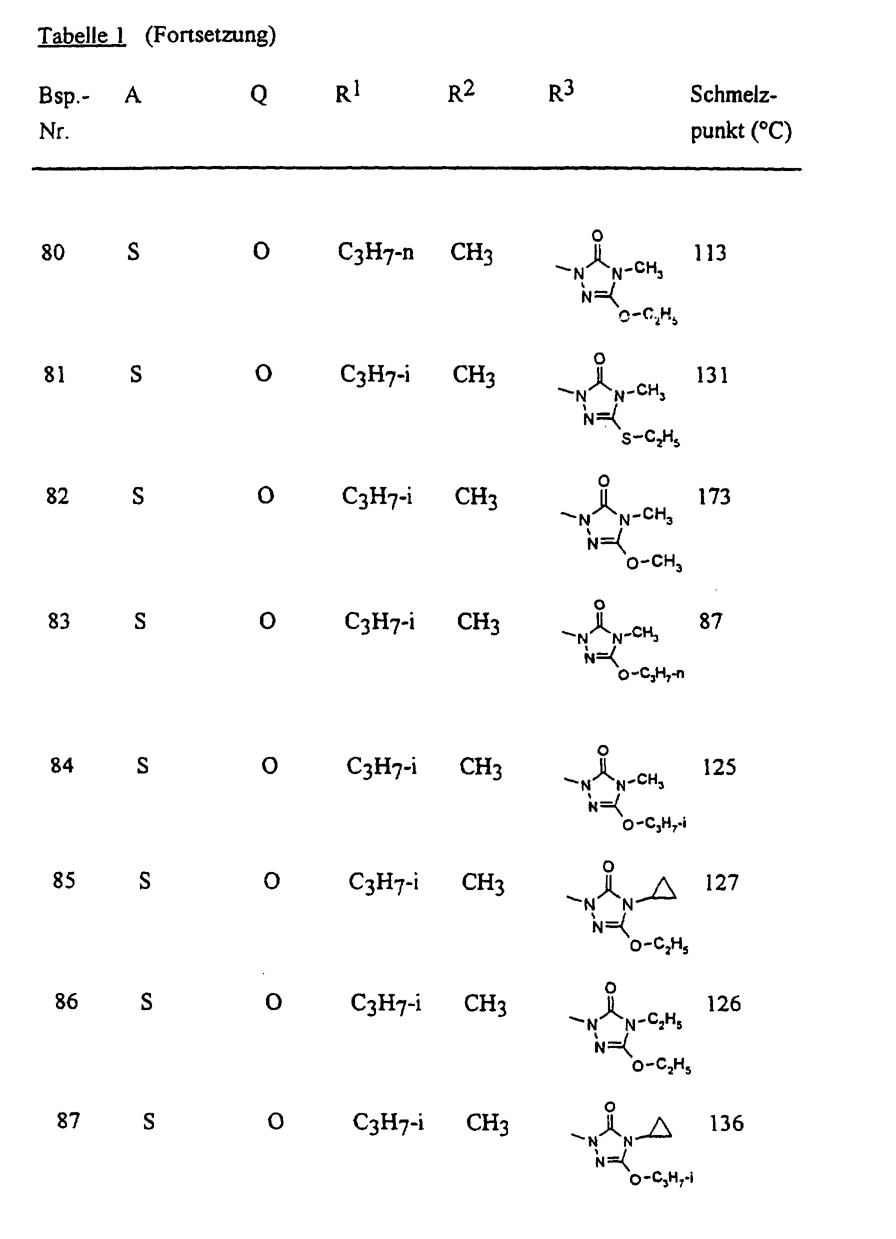

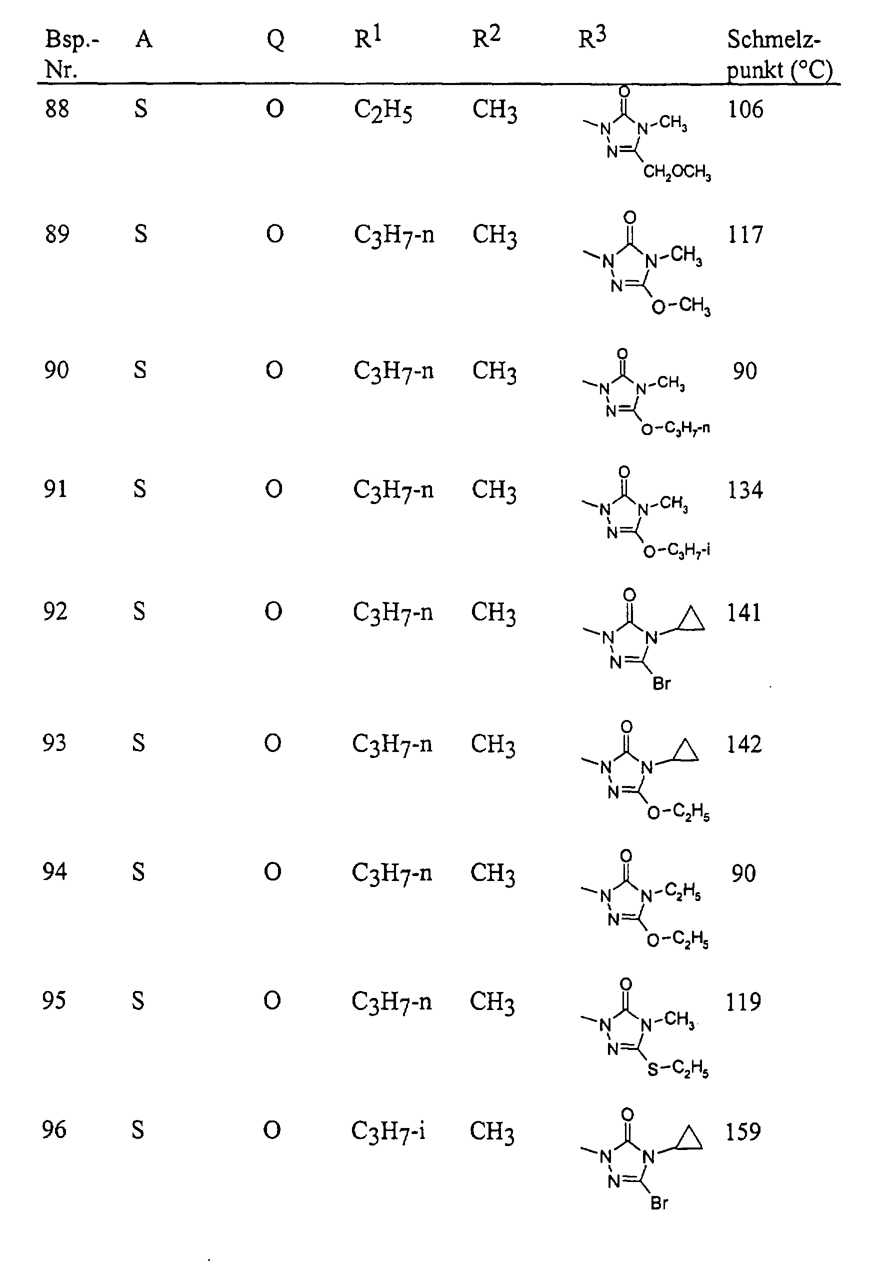

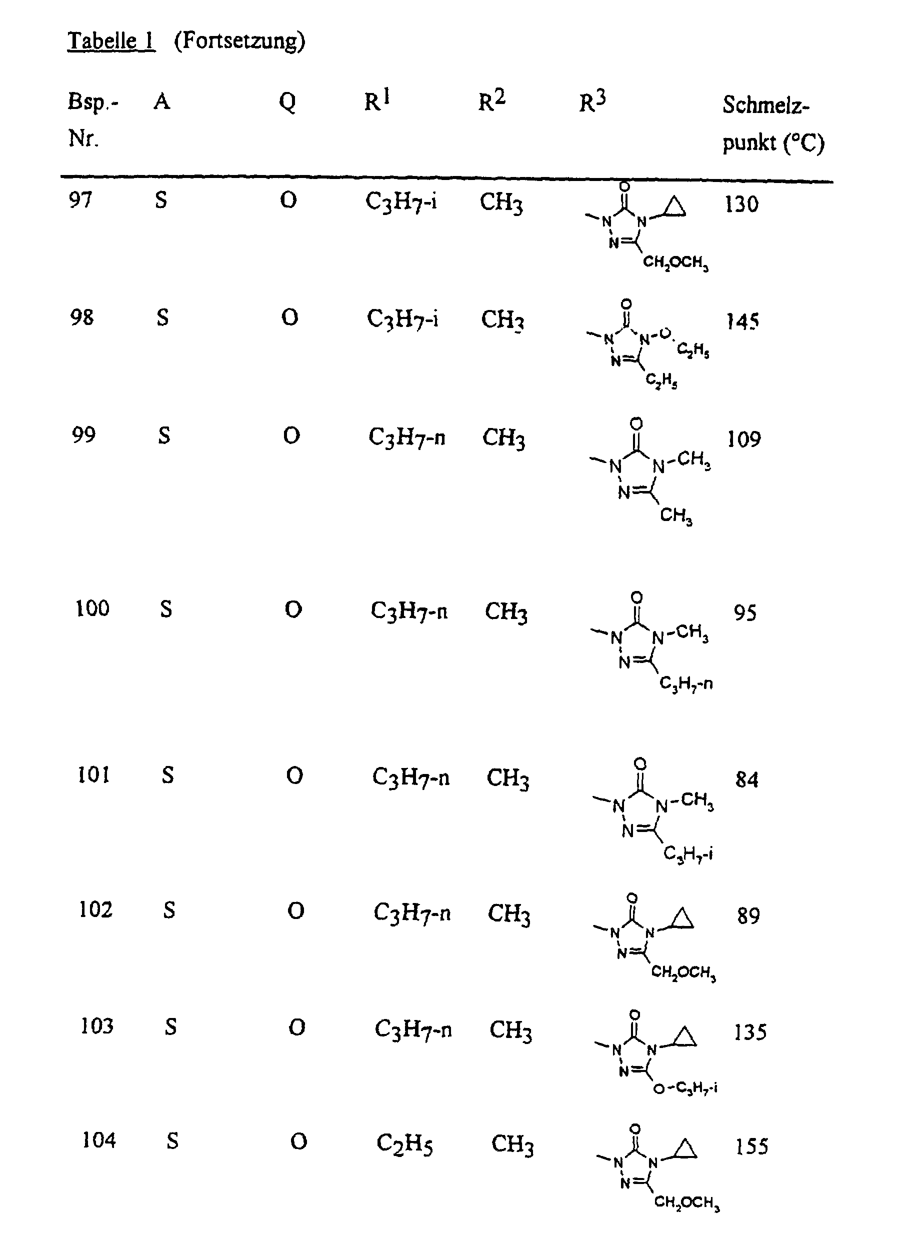

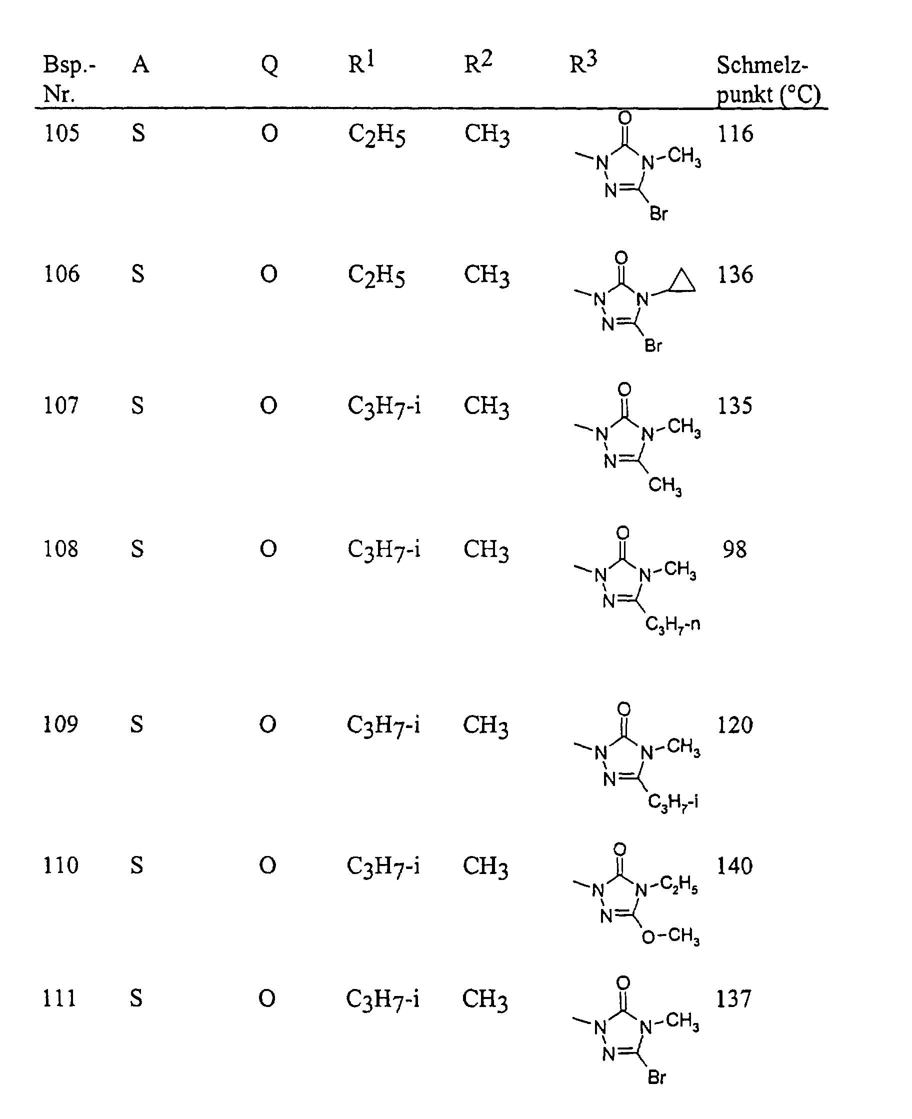

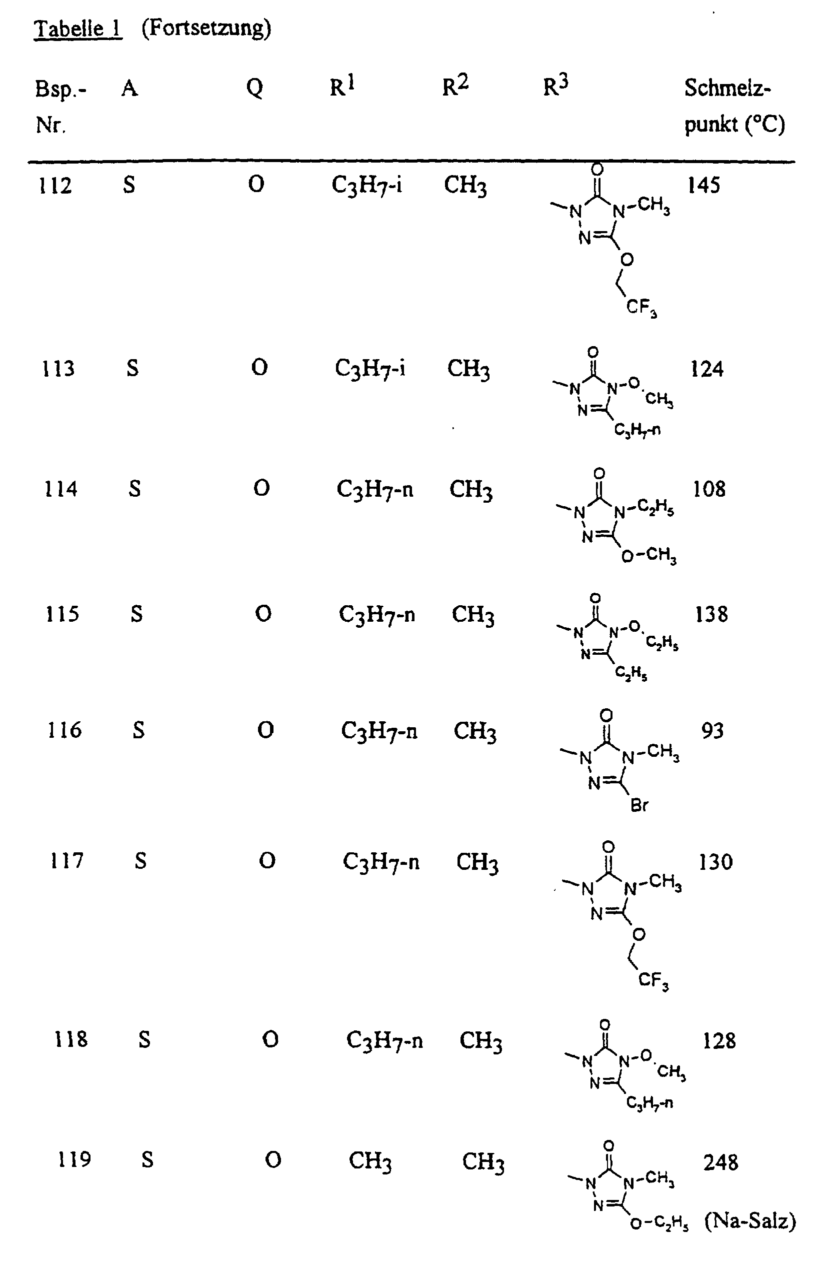

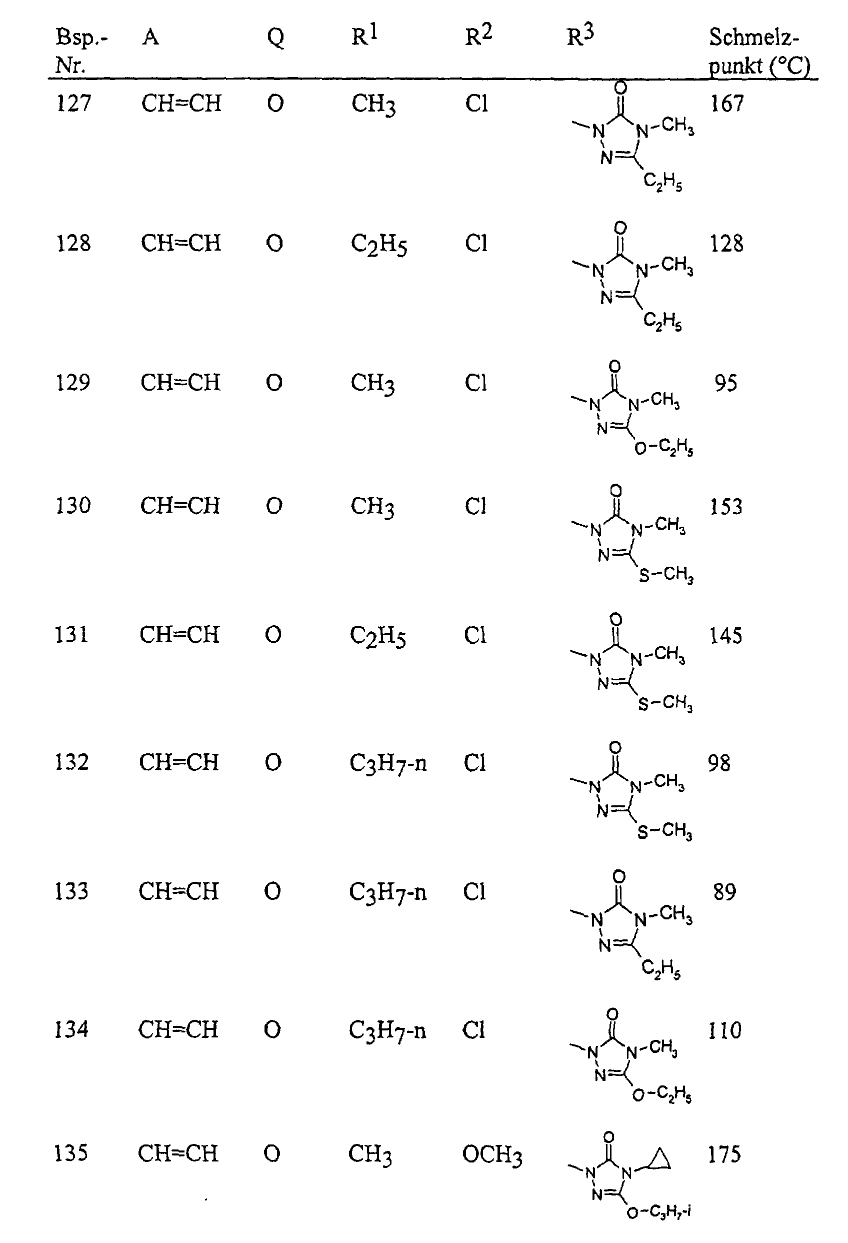

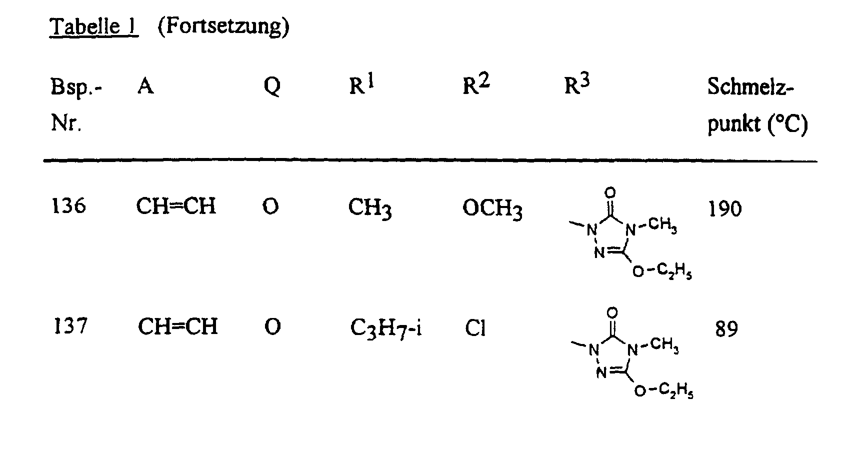

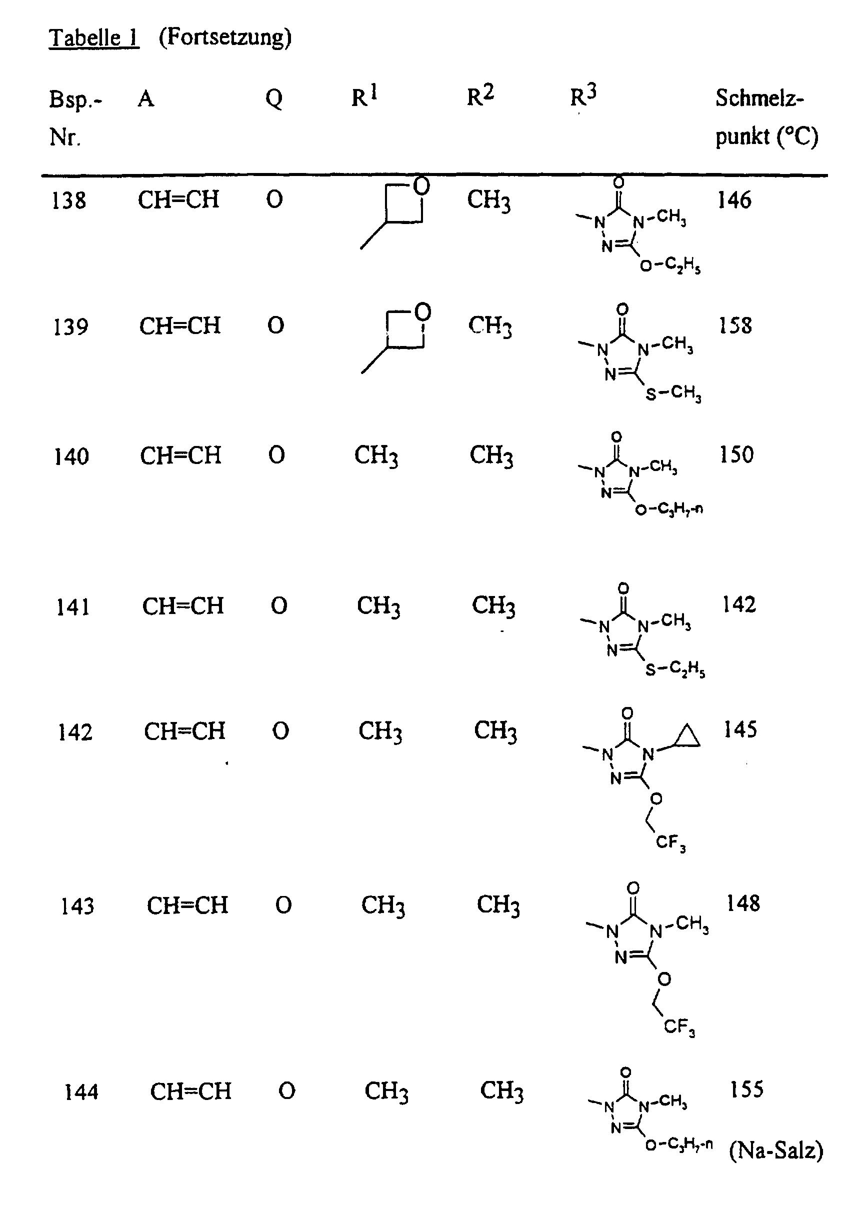

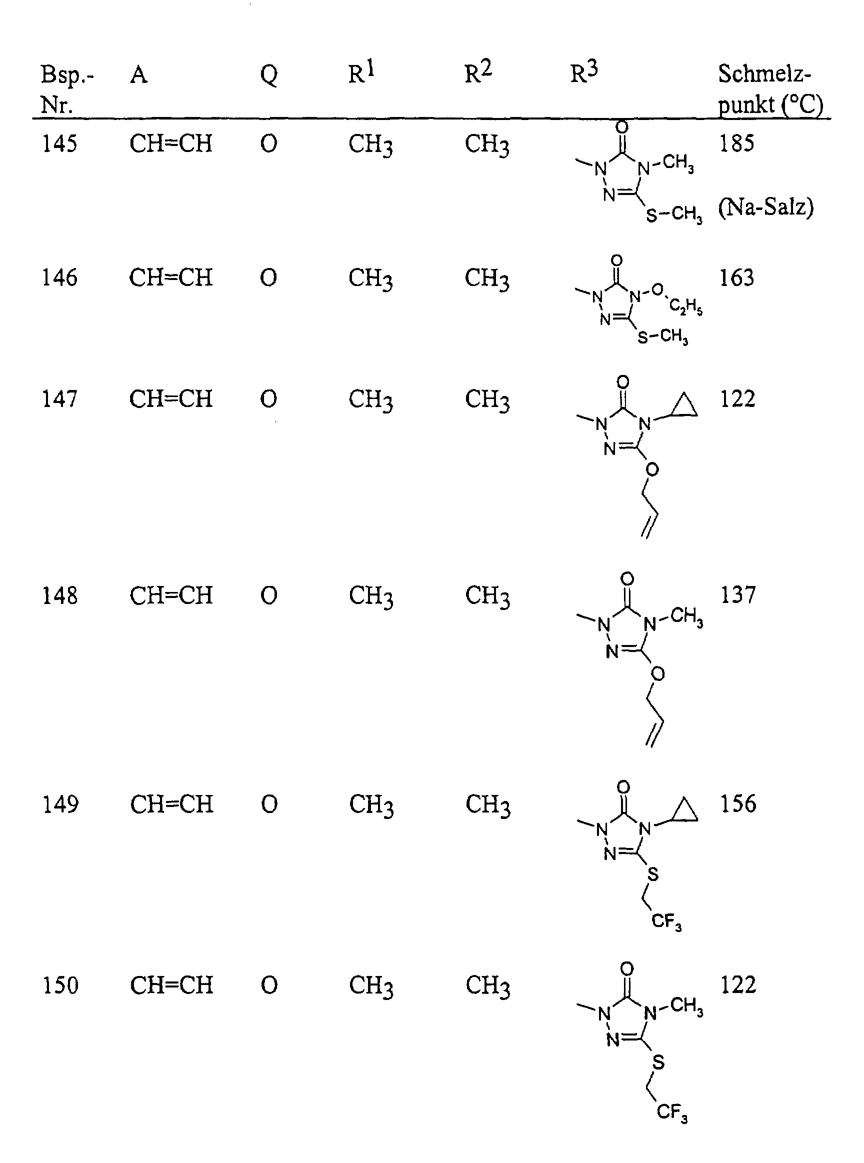

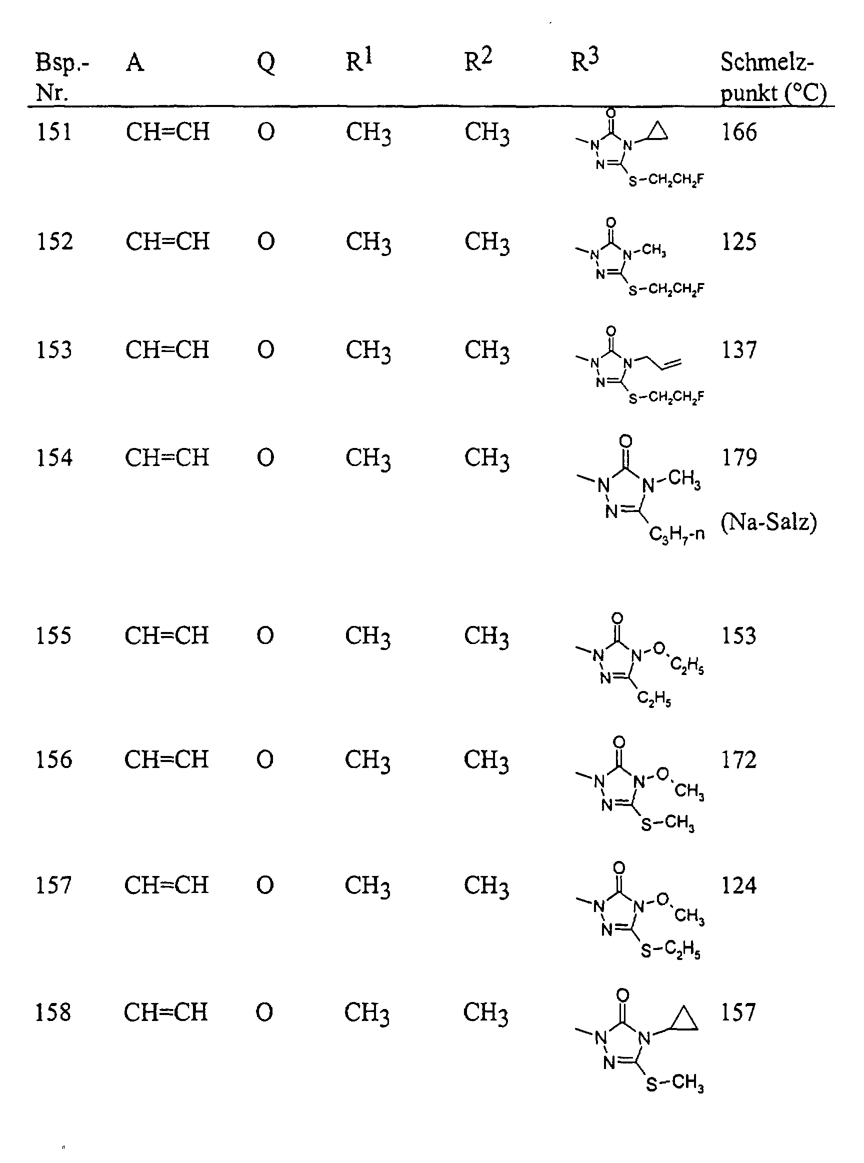

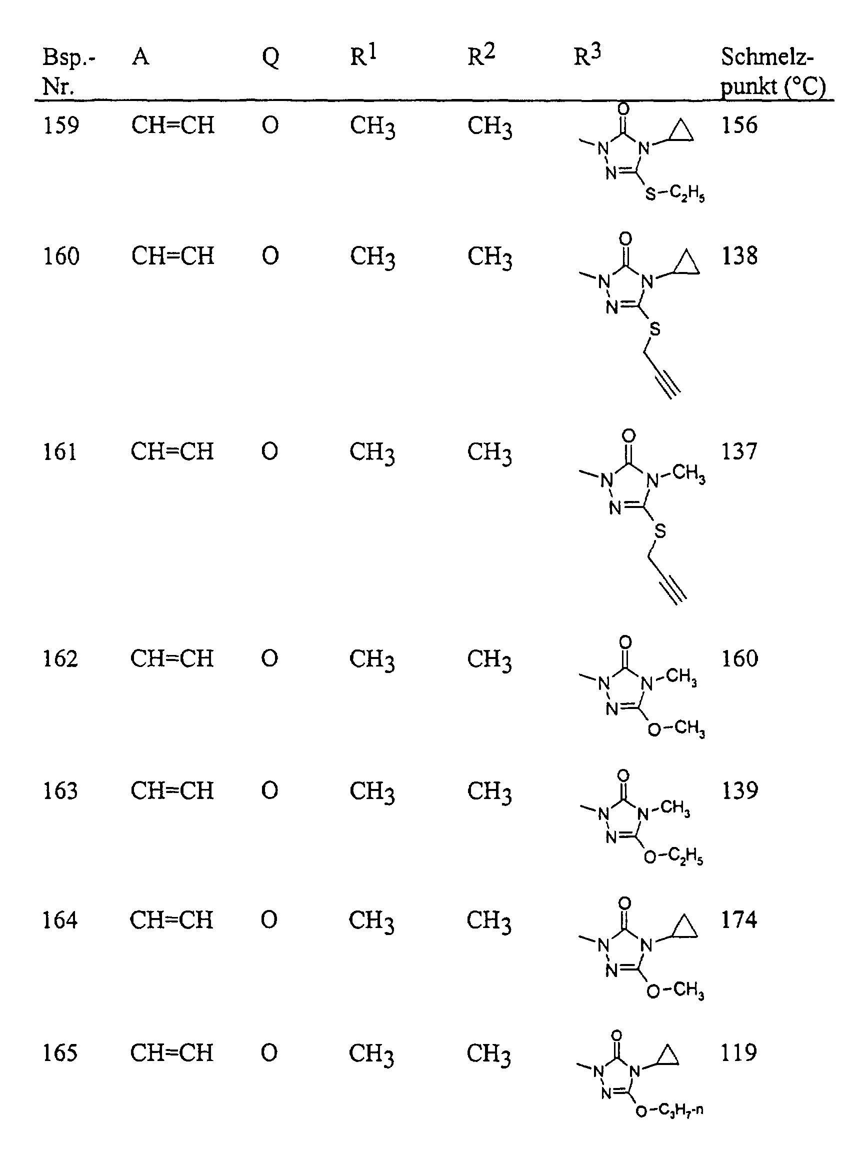

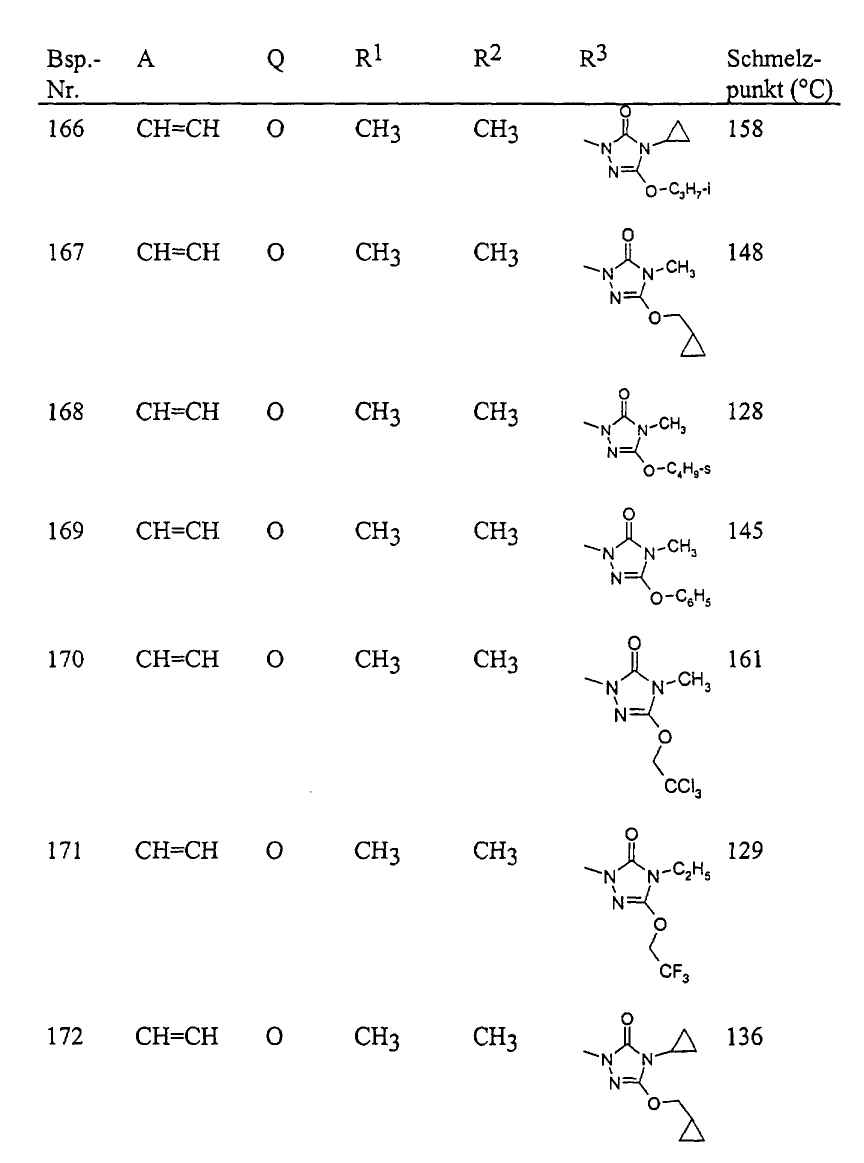

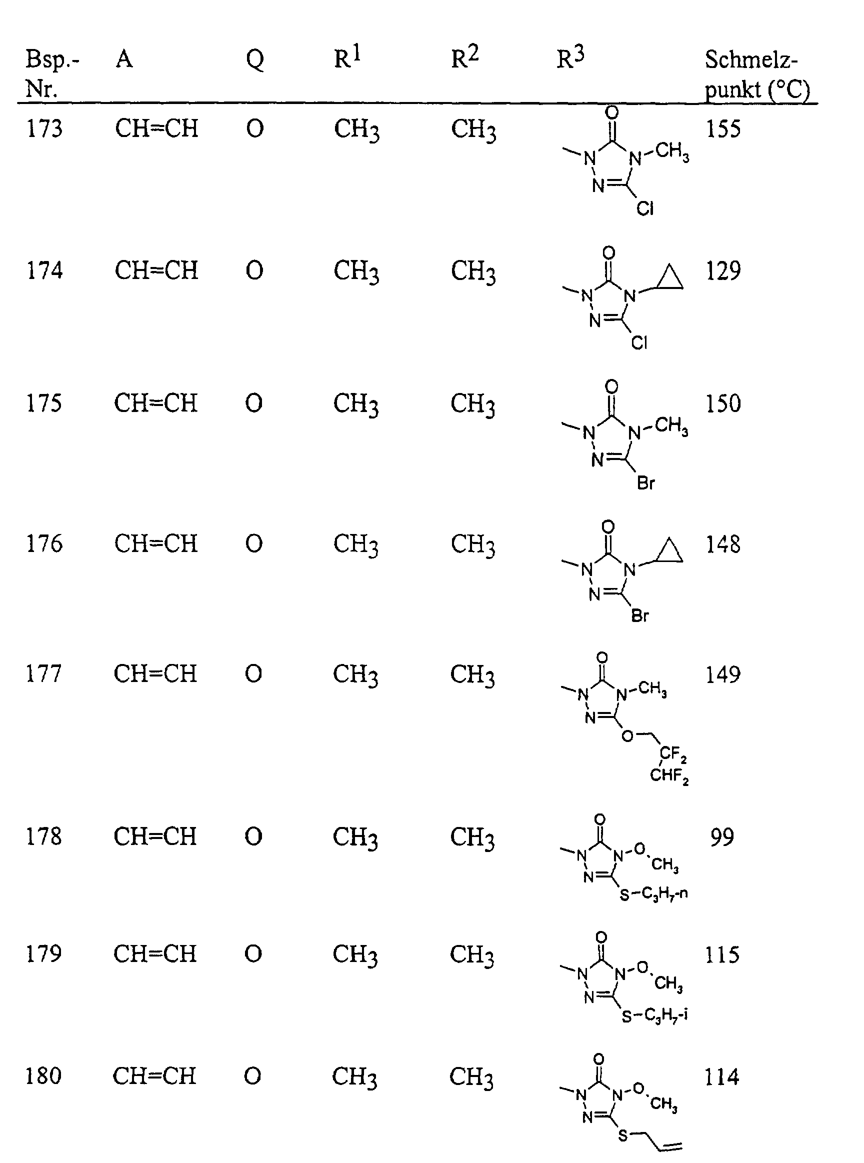

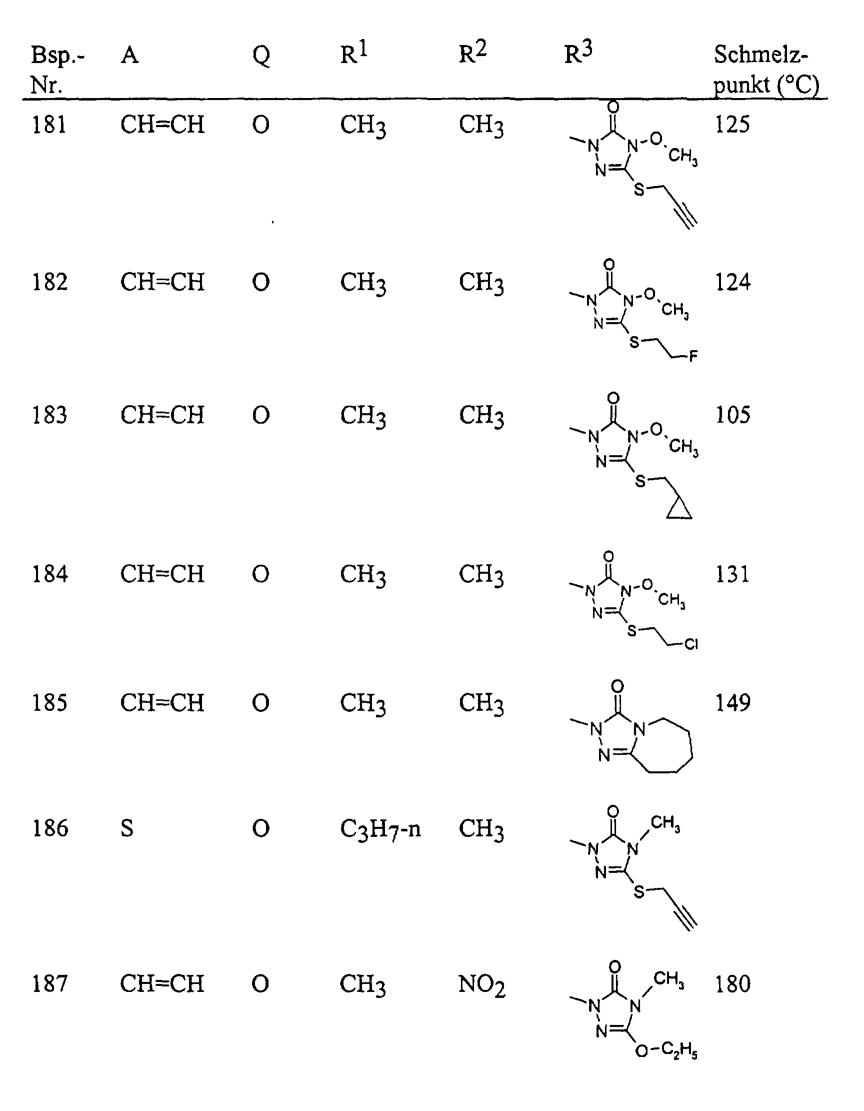

Die in Tabelle 1 als Beispiel 119 aufgeführte Verbindung kann beispielsweise wie folgt hergestellt werden:

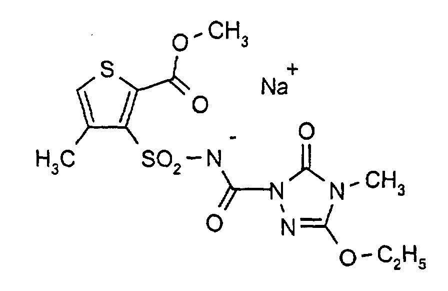

### (Salzbildung)

Eine Mischung aus 2,0 g (5 mMol) 5-Ethoxy-4-methyl-2-(4-methyl-2-methoxycarbonyl-thien-3-yl-sulfonylaminocarbonyl)-2,4-dihydro-3H-1,2,4-triazol-3-on und 75 ml Acetonitril wird mit 0,17 g (5,5 mMol) Natriumhydrid (80%ig) versetzt und die Mischung wird ca. 60 Minuten bei Raumtemperatur (ca. 20°C) gerührt. Das kristallin angefallene Produkt wird dann durch Absaugen isoliert.

Man erhält 2,0 g (94% der Theorie) des Natriumsalzes von 5-Ethoxy-4-methyl-2-(4-methyl-2-methoxycarbonyl-thien-3-yl-sulfonylaminocarbonyl)-2,4-dihydro-3H-1,2,4-triazol-3-on als farbloses kristallines Produkt vom Schmelzpunkt 248°C.

### Ausgangsstoffe der Formel (II):

### Beispiel (II-1)

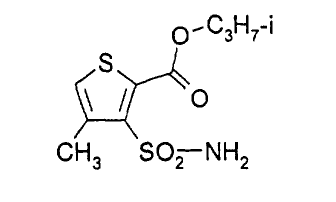

12,0 g (42,6 mMol) 4-Methyl-2-i-propoxycarbonyl-thiophen-3-sulfochlorid werden in 100 ml Methylenchlorid gelöst und mit 8,2 g (85,4 mMol) Ammoniumcarbonat versetzt. Die Mischung wird ca. 24 Stunden bei Raumtemperatur (ca. 20°C) gerührt. Dann wird vom ungelösten Salz abgesaugt, das Filtrat im Wasserstrahlvakuum eingeengt, der Rückstand mit Diethylether digeriert und das kristalline Produkt durch Absaugen isoliert.

Man erhält 9,1 g (91 % der Theorie) 4-Methyl-2-i-propoxycarbonyl-thiophen-3-sulfonamid als hellgelben Feststoff vom Schmelzpunkt 76°C.

Analog können beispielsweise die folgenden Verbindungen der Formel (II) hergestellt werden:
4-Methyl-2-methoxycarbonyl-thiophen-3-sulfonamid (Fp.: 54°C),
4-Methyl-2-ethoxycarbonyl-thiophen-3-sulfonamid (Fp.: 65°C),
4-Methyl-2-n-propoxycarbonyl-thiophen-3-sulfonamid (Fp.: 90°C),
4-Methyl-2-(oxetan-2-yl-oxycarbonyl)-thiophen-3-sulfonamid,
4-Ethyl-2-methoxycarbonyl-thiophen-3-sulfonamid,
4-Ethyl-2-ethoxycarbonyl-thiophen-3-sulfonamid,
4-Ethyl-2-n-propoxycarbonyl-thiophen-3-sulfonamid,
4-Ethyl-2-i-propoxycarbonyl-thiophen-3-sulfonamid,
4-Ethyl-2-(oxetan-2-yl-oxycarbonyl)-thiophen-3-sulfonamid,
4-Fluor-2-methoxycarbonyl-thiophen-3-sulfonamid,
4-Fluor-2-ethoxycarbonyl-thiophen-3-sulfonamid,
4-Fluor-2-n-propoxycarbonyl-thiophen-3-sulfonamid,
4-Fluor-2-i-propoxycarbonyl-thiophen-3-sulfonamid,
4-Fluor-2-(oxetan-2-yl-oxycarbonyl)-thiophen-3-sulfonamid,
4-Chlor-2-methoxycarbonyl-thiophen-3-sulfonamid,
4-Chlor-2-ethoxycarbonyl-thiophen-3-sulfonamid,
4-Chlor-2-n-propoxycarbonyl-thiophen-3-sulfonamid,
4-Chlor-2-i-propoxycarbonyl-thiophen-3-sulfonamid,
4-Chlor-2-(oxetan-2-yl-oxycarbonyl)-thiophen-3-sulfonamid,
4-Brom-2-methoxycarbonyl-thiophen-3-sulfonamid,
4-Brom-2-ethoxycarbonyl-thiophen-3-sulfonarnid,
4-Brom-2-n-propoxycarbonyl-thiophen-3-sulfonamid,
4-Brom-2-i-propoxycarbonyl-thiophen-3-sulfonamid,
4-Brom-2-(oxetan-2-yl-oxycarbonyl)-thiophen-3-sulfonamid,
4-Methoxy-2-methoxycarbonyl-thiophen-3-sulfonamid,
4-Methoxy-2-ethoxycarbonyl-thiophen-3-sulfonamid,
4-Methoxy-2-n-propoxycarbonyl-thiophen-3-sulfonamid,
4-Methoxy-2-i-propoxycarbonyl-thiophen-3-sulfonamid,
4-Methoxy-2-(oxetan-2-yl-oxycarbonyl)-thiophen-3-sulfonamid,
4-Methylthio-2-methoxycarbonyl-thiophen-3-sulfonamid,
4-Methylthio-2-ethoxycarbonyl-thiophen-3-sulfonamid,
4-Methylthio-2-n-propoxycarbonyl-thiophen-3-sulfonamid,
4-Methylthio-2-i-propoxycarbonyl-thiophen-3-sulfonamid,
4-Methylthio-2-(oxetan-2-yl-oxycarbonyl)-thiophen-3-sulfonamid,
4-Trifluormethyl-2-methoxycarbonyl-thiophen-3-sulfonamid,
4-Trifluormethyl-2-ethoxycarbonyl-thiophen-3-sulfonamid,
4-Trifluormethyl-2-n-propoxycarbonyl-thiophen-3-sulfonamid,
4-Trifluormethyl-2-i-propoxycarbonyl-thiophen-3-sulfonamid,
4-Trifluormethyl-2-(oxetan-2-yl-oxycarbonyl)-thiophen-3-sulfonamid,
4-Difluormethoxy-2-methoxycarbonyl-thiophen-3-sulfonamid,
4-Difluormethoxy-2-ethoxycarbonyl-thiophen-3-sulfonamid,
4-Difluormethoxy-2-n-propoxycarbonyl-thiophen-3-sulfonamid,
4-Difluormethoxy-2-i-propoxycarbonyl-thiophen-3-sulfonamid,
4-Difluormethoxy-2-(oxetan-2-yl-oxycarbonyl)-thiophen-3-sulfonamid,
4-Trifluormethoxy-2-methoxycarbonyl-thiophen-3-sulfonamid,
4-Trifluormethoxy-2-ethoxycarbonyl-thiophen-3-sulfonamid,
4-Trifluormethoxy-2-n-propoxycarbonyl-thiophen-3-sulfonamid,
4-Trifluormethoxy-2-i-propoxycarbonyl-thiophen-3-sulfonamid,
4-Trifluormethoxy-2-(oxetan-2-yl-oxycarbonyl)-thiophen-3-sulfonamid.
6-Methyl-2-methoxycarbonyl-benzolsulfonamid (Fp.: 118°C),
6-Methyl-2-ethoxycarbonyl-benzolsulfonamid (Fp.: 277°C),
6-Methyl-2-n-propoxycarbonyl-benzolsulfonamid,
6-Methyl-2-i-propoxycarbonyl-benzolsulfonamid (Fp.: 122°C),
6-Methyl-2-(oxetan-2-yl-oxycarbonyl)-benzolsulfonamid,
6-Ethyl-2-methoxycarbonyl-benzolsulfonamid,
6-Ethyl-2-ethoxycarbonyl-benzolsulfonamid,
6-Ethyl-2-n-propoxycarbonyl-benzolsulfonamid,
6-Ethyl-2-i-propoxycarbonyl-benzolsulfonamid,
6-Ethyl-2-(oxetan-2-yl-oxycarbonyl)-benzolsulfonamid,
6-Fluor-2-methoxycarbonyl-benzolsulfonamid,
6-Fluor-2-ethoxycarbonyl-benzolsulfonamid,
6-Fluor-2-n-propoxycarbonyl-benzolsulfonamid,
6-Fluor-2-i-propoxycarbonyl-benzolsulfonamid,
6-Fluor-2-(oxetan-2-yl-oxycarbonyl)-benzolsulfonamid,
6-Chlor-2-methoxycarbonyl-benzolsulfonamid,
6-Chlor-2-ethoxycarbonyl-benzolsulfonamid,
6-Chlor-2-n-propoxycarbonyl-benzolsulfonamid,
6-Chlor-2-i-propoxycarbonyl-benzolsulfonamid,
6-Chlor-2-(oxetan-2-yl-oxycarbonyl)-benzol-sulfonamid,
6-Brom-2-methoxycarbonyl-benzolsulfonamid,
6-Brom-2-ethoxycarbonyl-benzolsulfonamid,
6-Brom-2-n-propoxycarbonyl-benzolsulfonamid,
6-Brom-2-i-propoxycarbonyl-benzolsulfonamid,
6-Brom-2-(oxetan-2-yl-oxycarbonyl)-benzolsulfonamid,
6-Methoxy-2-methoxycarbonyl-benzolsulfonamid,
6-Methoxy-2-ethoxycarbonyl-benzolsulfonamid,
6-Methoxy-2-n-propoxycarbonyl-benzolsulfonamid,
6-Methoxy-2-i-propoxycarbonyl-benzolsulfonamid,
6-Methoxy-2-(oxetan-2-yl-oxycarbonyl)-benzolsulfonamid,
6-Methylthio-2-methoxycarbonyl-benzolsulfonamid,
6-Methylthio-2-ethoxycarbonyl-benzolsulfonamid,
6-Methylthio-2-n-propoxycarbonyl-benzolsulfonamid,
6-Methylthio-2-i-propoxycarbonyl-benzolsulfonamid,
6-Methylthio-2-(oxetan-2-yl-oxycarbonyl)-benzolsulfonamid,
6-Trifluormethyl-2-methoxycarbonyl-benzolsulfonamid,
6-Trifluormethyl-2-ethoxycarbonyl-benzolsulfonamid,
6-Trifluormethyl-2-n-propoxycarbonyl-benzolsulfonamid,
6-Trifluormethyl-2-i-propoxycarbonyl-benzolsulfonamid,
6-Trifluormethyl-2-(oxetan-2-yl-oxycarbonyl)-benzolsulfonamid,
6-Difluormethoxy-2-methoxycarbonyl-benzolsulfonamid,
6-Difluormethoxy-2-ethoxycarbonyl-benzolsulfonamid,
6-Difluormethoxy-2-n-propoxycarbonyl-benzolsulfonamid,
6-Difluormethoxy-2-i-propoxycarbonyl-benzolsulfonamid,
6-Difluormethoxy-2-(oxetan-2-yl-oxycarbonyl)-benzolsulfonamid,
6-Trifluormethoxy-2-methoxycarbonyl-benzolsulfonamid,
6-Trifluormethoxy-2-ethoxycarbonyl-benzolsulfonamid,
6-Trifluormethoxy-2-n-propoxycarbonyl-benzolsulfonamid,
6-Trifluormethoxy-2-i-propoxycarbonyl-benzolsulfonamid,
6-Trifluormethoxy-2-(oxetan-2-yI-oxycarbonyl)-benzolsulfonamid.

### Ausgangsstoffe der Formel (IV):

### Beispiel (IV-1)

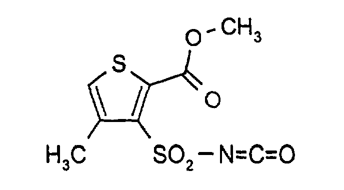

Eine Mischung aus 25 g (95 mMol) 4-Methyl-2-methoxycarbonyl-thiophen-3-sulfonamid, 9,4 g (95 mMol) n-Butylisocyanat und 100 ml Chlorbenzol wird zum Sieden erhitzt und bei der Rückflußtemperatur wird 4 Stunden lang Phosgen eingeleitet. Anschließend wird im Wasserstrahlvakuum eingeengt und der Rückstand durch Destillation unter vermindertem Druck (2 mBar) gereinigt.

Man erhält 11 g (40 % der Theorie) 4-Methyl-2-methoxycarbonyl-thiophen-3-yl-sulfonylisocyanat vom Siedebereich 140°C - 145°C (bei 2 mBar), welches zu farblosen Kristallen erstarrt.

Analog können beispielsweise die folgenden Verbindungen der Formel (II) hergestellt werden:
4-Methyl-2-ethoxycarbonyl-thiophen-3-yl-sulfonylisocyanat,
4-Methyl-2-n-propoxycarbonyl-thiophen-3-yl-sulfonylisocyanat,
4-Methyl-2-i-propoxycarbonyl-thiophen-3-yl-sulfonylisocyanat,
4-Methyl-2-(oxetan-2-yl-oxycarbonyl)-thiophen-3-yl-sulfonylisocyanat,
4-Ethyl-2-methoxycarbonyl-thiophen-3-yl-sulfonylisocyanat,
4-Ethyl-2-ethoxycarbonyl-thiophen-3-yl-sulfonylisocyanat,
4-Ethyl-2-n-propoxycarbonyl-thiophen-3-yl-sulfonylisocyanat,
4-Ethyl-2-i-propoxycarbonyl-thiophen-3-yl-sulfonylisocyanat,
4-Ethyl-2-(oxetan-2-yl-oxycarbonyl)-thiophen-3-yl-sulfonylisocyanat,
4-Fluor-2-methoxycarbonyl-thiophen-3-yl-sulfonylisocyanat,
4-Fluor-2-ethoxycarbonyl-thiophen-3-yl-sulfonylisocyanat,
4-Fluor-2-n-propoxycarbonyl-thiophen-3-yl-sulfonylisocyanat,
4-Fluor-2-i-propoxycarbonyl-thiophen-3-yl-sulfonylisocyanat,
4-Fluor-2-(oxetan-2-yl-oxycarbonyl)-thiophen-3-yl-sulfonylisocyanat,
4-Chlor-2-methoxycarbonyl-thiophen-3-yl-sulfonylisocyanat,
4-Chlor-2-ethoxycarbonyl-thiophen-3-yl-sulfonylisocyanat,
4-Chlor-2-n-propoxycarbonyl-thiophen-3-yl-sulfonylisocyanat,
4-Chlor-2-i-propoxycarbonyl-thiophen-3-yl-sulfonylisocyanat,
4-Chlor-2-(oxetan-2-yl-oxycarbonyl)-thiophen-3-yl-sulfonylisocyanat,
4-Brom-2-methoxycarbonyl-thiophen-3-yl-sulfonylisocyanat,
4-Brom-2-ethoxycarbonyl-thiophen-3-yl-sulfonylisocyanat,
4-Brom-2-n-propoxycarbonyl-thiophen-3-yl-sulfonylisocyanat,
4-Brom-2-i-propoxycarbonyl-thiophen-3-yl-sulfonylisocyanat,
4-Brom-2-(oxetan-2-yl-oxycarbonyl)-thiophen-3-yl-sulfonylisocyanat,
4-Methoxy-2-methoxycarbonyl-thiophen-3-yl-sulfonylisocyanat,
4-Methoxy-2-ethoxycarbonyl-thiophen-3-yl-sulfonylisocyanat,
4-Methoxy-2-n-propoxycarbonyl-thiophen-3-yl-sulfonylisocyanat,
4-Methoxy-2-i-propoxycarbonyl-thiophen-3-yl-sulfonylisocyanat,
4-Methoxy-2-(oxetan-2-yl-oxycarbonyl)-thiophen-3-yl-sulfonylisocyanat,
4-Methylthio-2-methoxycarbonyl-thiophen-3-yl-sulfonylisocyanat,
4-Methylthio-2-ethoxycarbonyl-thiophen-3-yl-sulfonylisocyanat,
4-Methylthio-2-n-propoxycarbonyl-thiophen-3-yl-sulfonylisocyanat,
4-Methylthio-2-i-propoxycarbonyl-thiophen-3-yl-sulfonylisocyanat,
4-Methylthio-2-(oxetan-2-yl-oxycarbonyl)-thiophen-3-yl-sulfonylisocyanat,
4-Trifluormethyl-2-methoxycarbonyl-thiophen-3-yl-sulfonylisocyanat,
4-Trifluormethyl-2-ethoxycarbonyl-thiophen-3-yl-sulfonylisocyanat,
4-Trifluormethyl-2-n-propoxycarbonyl-thiophen-3-yl-sulfonylisocyanat,
4-Trifluormethyl-2-i-propoxycarbonyl-thiophen-3-yl-sulfonylisocyanat,
4-Trifluormethyl-2-(oxetan-2-yl-oxycarbonyl)-thiophen-3-yl-sulfonylisocyanat,
4-Difluormethoxy-2-methoxycarbonyl-thiophen-3-yl-sulfonylisocyanat,
4-Difluormethoxy-2-ethoxycarbonyl-thiophen-3-yl-sulfonylisocyanat,
4-Difluormethoxy-2-n-propoxycarbonyl-thiophen-3-yl-sulfonylisocyanat,
4-Difluorrnethoxy-2-i-propoxycarbonyl-thiophen-3-yl-sulfonylisocyanat,
4-Difluormethoxy-2-(oxetan-2-yl-oxycarbonyl)-thiophen-3-yl-sulfonylisocyanat,
4-Trifluormethoxy-2-methoxycarbonyl-thiophen-3-yl-sulfonylisocyanat,
4-Trifluormethoxy-2-ethoxycarbonyl-thiophen-3-yl-sulfonylisocyanat,
4-Trifluormethoxy-2-n-propoxycarbonyl-thiophen-3-yl-sulfonylisocyanat,
4-Trifluormethoxy-2-i-propoxycarbonyl-thiophen-3-yl-sulfonylisocyanat,
4-Trifluormethoxy-2-(oxetan-2-yl-oxycarbonyl)-thiophen-3-yl-sulfonylisocyanat,
6-Methyl-2-methoxycarbonyl-phenylsulfonylisocyanat,
6-Methyl-2-ethoxycarbonyl-phenylsulfonylisocyanat,
6-Methyl-2-n-propoxycarbonyl-phenylsulfonylisocyanat,
6-Methyl-2-i-propoxycarbonyl-phenylsulfonylisocyanat,
6-Methyl-2-(oxetan-2-yl-oxycarbonyl)-phenylsulfonylisocyanat,
6-Ethyl-2-methoxycarbonyl-phenylsulfonylisocyanat,
6-Ethyl-2-ethoxycarbonyl-phenylsulfonylisocyanat,
6-Ethyl-2-n-propoxycarbonyl-phenylsulfonylisocyanat,
6-Ethyl-2-i-propoxycarbonyl-phenylsulfonylisocyanat,
6-Ethyl-2-(oxetan-2-yl-oxycarbonyl)-phenylsulfonylisocyanat,
6-Fluor-2-methoxycarbonyl-phenylsulfonylisocyanat,
6-Fluor-2-ethoxycarbonyl-phenylsulfonylisocyanat,
6-Fluor-2-n-propoxycarbonyl-phenylsulfonylisocyanat,
6-Fluor-2-i-propoxycarbonyl-phenylsulfonylisocyanat,
6-Fluor-2-(oxetan-2-yl-oxycarbonyl)-phenylsulfonylisocyanat,
6-Chlor-2-methoxycarbonyl-phenylsulfonylisocyanat,
6-Chlor-2-ethoxycarbonyl-phenylsulfonylisocyanat,
6-Chlor-2-n-propoxycarbonyl-phenylsulfonylisocyanat,
6-Chlor-2-i-prppoxycarbonyl-phenylsulfonylisocyanat,
6-Chlor-2-(oxetan-2-yl-oxycarbonyl)-phenylsulfonylisocyanat,
6-Brom-2-methoxycarbonyl-phenylsulfonylisocyanat,
6-Brom-2-ethoxycarbonyl-phenylsulfonylisocyanat,
6-Brom-2-n-propoxycarbonyl-phenylsulfonylisocyanat,
6-Brom-2-i-propoxycarbonyl-phenylsulfonylisocyanat,
6-Brom-2-(oxetan-2-yl-oxycarbonyl)-phenylsulfonylisocyanat,
6-Methoxy-2-methoxycarbonyl-phenylsulfonylisocyanat,
6-Methoxy-2-ethoxycarbonyl-phenylsulfonylisocyanat,
6-Methoxy-2-n-propoxycarbonyl-phenylsulfonylisocyanat,
6-Methoxy-2-i-propoxycarbonyl-phenylsulfonylisocyanat,
6-Methoxy-2-(oxetan-2-yl-oxycarbonyl)-phenylsulfonylisocyanat,
6-Methylthio-2-methoxycarbonyl-phenylsulfonylisocyanat,
6-Methylthio-2-ethoxycarbonyl-phenylsulfonylisocyanat,
6-Methylthio-2-n-propoxycarbonyl-phenylsulfonylisocyanat,
6-Methylthio-2-i-propoxycarbonyl-phenylsulfonylisocyanat,
6-Methylthio-2-(oxetan-2-yl-oxycarbonyl)-phenylsulfonylisocyanat,
6-Trifluormethyl-2-methoxycarbonyl-phenylsulfonylisocyanat,
6-Trifluormethyl-2-ethoxycarbonyl-phenylsulfonylisocyanat,
6-Trifluormethyl-2-n-propoxycarbonyl-phenylsulfonylisocyanat,
6-Trifluormethyl-2-i-propoxycarbonyl-phenylsulfonylisocyanat,
6-Trifluormethyl-2-(oxetan-2-yl-oxycarbonyl)-phenylsulfonylisocyanat,
6-Difluormethoxy-2-methoxycarbonyl-phenylsulfonylisocyanat,
6-Difluormethoxy-2-ethoxycarbonyl-phenylsulfonylisocyanat,
6-Difluormethoxy-2-n-propoxycarbonyl-phenylsulfonylisocyanat,
6-Difluormethoxy-2-i-propoxycarbonyl-phenylsulfonylisocyanat,
6-Difluormethoxy-2-(oxetan-2-yl-oxycarbonyl)-phenylsulfonylisocyanat,
6-Trifluormethoxy-2-methoxycarbonyl-phenylsulfonylisocyanat,
6-Trifluormethoxy-2-ethoxycarbonyl-phenylsulfonylisocyanat,
6-Trifluormethoxy-2-n-propoxycarbonyl-phenylsulfonylisocyanat,
6-Trifluormethoxy-2-i-propoxycarbonyl-phenylsulfonylisocyanat,
6-Trifluormethoxy-2-(oxetan-2-yl-oxycarbonyl)-phenylsulfonylisocyanat.

### Ausgangsstoffe der Formel (VI):

### Beispiel (VI-1)

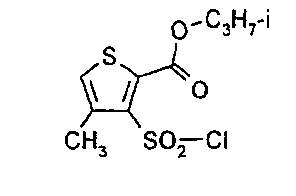

Eine Lösung von 21,2 g (90 mMol) 3-Amino-4-methyl-thiophen-2-carbonsäure-i-propylester-Hydrochlorid in 60 ml konz. Salzsäure wird auf ca. -10°C abgekühlt und bei -10°C bis -5°C wird dann unter Rühren eine Lösung von 6,9 g (100 mMol) Natriumnitrit in 13 ml Wasser tropfenweise dazu gegeben. Die Reaktionsmischung wird ca. 60 Minuten bei -5°C bis 0°C gerührt. Die so erhaltene Diazoniumsalzlösung wird bei ca. 15°C zu einer Lösung von 50 g Schwefeldioxid in 110 ml Essigsäure, welche 10 ml einer gesättigten wässrigen Lösung von Kupfer(II)-chlorid enthält, tropfenweise gegeben. Die Mischung wird 12 Stunden bei Raumtemperatur (ca. 20°C) gerührt, dann mit ca. 500 ml Methylenchlorid verdünnt, zweimal mit Eiswasser gewaschen, mit Magnesiumsulfat getrocknet und filtriert. Das Filtrat wird im Wasserstrahlvakuum eingeengt, der Rückstand mit Petrolether digeriert und das hierbei kristallin angefallene Produkt durch Absaugen isoliert.

Man erhält 17,0 g (67% der Theorie) 4-Methyl-2-i-propoxycarbonyl-thiophen-3-sulfochlorid als gelben Feststoff vom Schmelzpunkt 58°C.

Analog können beispielsweise die folgenden Verbindungen der Formel (VI) hergestellt werden:
4-Methyl-2-methoxycarbonyl-thiophen-3-sulfochlorid (Fp.: 56°C),
4-Methyl-2-ethoxycarbonyl-thiophen-3-sulfochlorid (Fp.: 47°C),
4-Methyl-2-n-propoxycarbonyl-thiophen-3-sulfochlorid (Fp.: 42°C),
4-Methyl-2-(oxetan-2-yl-oxycarbonyl)-thiophen-3-sulfochlorid,
4-Ethyl-2-methoxycarbonyl-thiophen-3-sulfochlorid,
4-Ethyl-2-ethoxycarbonyl-thiophen-3-sulfochlorid,
4-Ethyl-2-n-propoxycarbonyl-thiophen-3-sulfochlorid,
4-Ethyl-2-i-propoxycarbonyl-thiophen-3-sulfochlorid,
4-Ethyl-2-(oxetan-2-yl-oxycarbonyl)-thiophen-3-sulfochlorid,
4-Fluor-2-methoxycarbonyl-thiophen-3-sulfochlorid,
4-Fluor-2-ethoxycarbonyl-thiophen-3-sulfochlorid,
4-Fluor-2-n-propoxycarbonyl-thiophen-3-sulfochlorid,
4-Fluor-2-i-propoxycarbonyl-thiophen-3-sulfochlorid,
4-Fluor-2-(oxetan-2-yl-oxycarbonyl)-thiophen-3-sulfochlorid,
4-Chlor-2-methoxycarbonyl-thiophen-3-sulfochlorid,
4-Chlor-2-ethoxycarbonyl-thiophen-3-sulfochlorid,
4-Chlor-2-n-propoxycarbonyl-thiophen-3-sulfochlorid,
4-Chlor-2-i-propoxycarbonyl-thiophen-3-sulfochlorid,
4-Chlor-2-(oxetan-2-yl-oxycarbonyl)-thiophen-3-sulfochlorid,
4-Brom-2-methoxycarbonyl-thiophen-3-sulfochlorid,
4-Brom-2-ethoxycarbonyl-thiophen-3-sulfochlorid,
4-Brom-2-n-propoxycarbonyl-thiophen-3-sulfochlorid,
4-Brom-2-i-propoxycarbonyi-thiophen-3-sulfochlorid,
4-Brom-2-(oxetan-2-yl-oxycarbonyl)-thiophen-3-sulfochlorid,
4-Methoxy-2-methoxycarbonyl-thiophen-3-sulfochlorid,
4-Methoxy-2-ethoxycarbonyl-thiophen-3-sulfochlorid,
4-Methoxy-2-n-propoxycarbonyl-thiophen-3-sulfochlorid,
4-Methoxy-2-i-propoxycarbonyl-thiophen-3-sulfochlorid,
4-Methoxy-2-(oxetan-2-yl-oxycarbonyl)-thiophen-3-sulfochlorid,
4-Methylthio-2-methoxycarbonyl-thiophen-3-sulfochlorid,
4-Methylthio-2-ethoxycarbonyl-thiophen-3-sulfochlorid,
4-Methylthio-2-n-propoxycarbonyl-thiophen-3-sulfochlorid,
4-Methylthio-2-i-propoxycarbonyl-thiophen-3-sulfochlorid,
4-Methylthio-2-(oxetan-2-yl-oxycarbonyl)-thiophen-3-sulfochlorid,
4-Trifluormethyl-2-methoxycarbonyl-thiophen-3-sulfochlorid,
4-Trifluormethyl-2-ethoxycarbonyl-thiophen-3-sulfochlorid,
4-Trifluormethyl-2-n-propoxycarbonyl-thiophen-3-sulfochlorid,
4-Trifluormethyl-2-i-propoxycarbonyl-thiophen-3-sulfochlorid,
4-Trifluormethyl-2-(oxetan-2-yl-oxycarbonyl)-thiophen-3-sulfochlorid,
4-Difluormethoxy-2-methoxycarbonyl-thiophen-3-sulfochlorid,
4-Difluorrnethoxy-2-ethoxycarbonyl-thiophen-3-sulfochlorid,
4-Difluormethoxy-2-n-propoxycarbonyl-thiophen-3-sulfochlorid,
4-Difluormethoxy-2-i-propoxycarbonyl-thiophen-3-sulfochlorid,
4-Difluormethoxy-2-(oxetan-2-yl-oxycarbonyl)-thiophen-3-sulfochlorid,
4-Trifluormethoxy-2-methoxycarbonyl-thiophen-3-sulfochlorid,
4-Trifluormethoxy-2-ethoxycarbonyl-thiophen-3-sulfochlorid,
4-Trifluormethoxy-2-n-propoxycarbonyl-thiophen-3-sulfochlorid,
4-Trifluormethoxy-2-i-propoxycarbonyl-thiophen-3-sulfochlorid,
4-Trifluormethoxy-2-(oxetan-2-yl-oxycarbonyl)-thiophen-3-sulfochlorid.
6-Methyl-2-methoxycarbonyl-benzolsulfochlorid (Fp.: 109°C),
6-Methyl-2-ethoxycarbonyl-benzolsulfochlorid (Fp.: 82°C),
6-Methyl-2-n-propoxycarbonyl-benzolsulfochiorid (Fp.: 52°C),
6-Methyl-2-i-propoxycarbonyl-benzolsulfochlorid (Fp.: 51°C),
6-Methyl-2-(oxetan-2-yl-oxycarbonyl)-benzolsulfochlorid,
6-Ethyl-2-methoxycarbonyl-benzolsulfochlorid,
6-Ethyl-2-ethoxycarbonyl-benzolsulfochlorid,
6-Ethyl-2-n-propoxycarbonyl-benzolsulfochlorid,
6-Ethyl-2-i-propoxycarbonyl-benzolsulfochlorid,
6-Ethyl-2-(oxetan-2-yl-oxycarbonyl)-benzolsulfochlorid,
6-Fluor-2-methoxycarbonyl-benzolsulfochlorid,
6-Fluor-2-ethoxycarbonyl-benzolsulfochlorid,
6-Fluor-2-n-propoxycarbonyl-benzolsulfochlorid,
6-Fluor-2-i-propoxycarbonyl-benzolsulfochlorid,
6-Fluor-2-(oxetan-2-yl-oxycarbonyl)-benzolsulfochlorid,
6-Chlor-2-methoxycarbonyl-benzolsulfochlorid,
6-Chlor-2-ethoxycarbonyl-benzolsulfochlorid,
6-Chlor-2-n-propoxycarbonyl-benzolsulfochlorid,
6-Chlor-2-i-propoxycarbonyl-benzolsulfochlorid,
6-Chlor-2-(oxetan-2-yl-oxycarbonyl)-benzol-sulfochlorid,
6-Brom-2-methoxycarbonyl-benzolsulfochlorid,
6-Brom-2-ethoxycarbonyl-benzolsulfochlorid,
6-Brom-2-n-propoxycarbonyl-benzolsulfochlorid,
6-Brom-2-i-propoxycarbonyl-benzolsulfochlorid,
6-Brom-2-(oxetan-2-yl-oxycarbonyl)-benzolsulfochlorid,
6-Methoxy-2-methoxycarbonyl-benzolsulfochlorid,
6-Methoxy-2-ethoxycarbonyl-benzolsulfochlorid,
6-Methoxy-2-n-propoxycarbonyl-benzolsulfochlorid,
6-Methoxy-2-i-propoxycarbonyl-benzolsulfochlorid,
6-Methoxy-2-(oxetan-2-yl-oxycarbonyl)-benzolsulfochlorid,
6-Methylthio-2-methoxycarbonyl-benzolsulfochlorid,
6-Methylthio-2-ethoxycarbonyl-benzolsulfochlorid,
6-Methylthio-2-n-propoxycarbonyl-benzolsulfochlorid,
6-Methylthio-2-i-propoxycarbonyl-benzolsulfochlorid,
6-Methylthio-2-(oxetan-2-yl-oxycarbonyl)-benzolsulfochlorid,
6-Trifluormethyl-2-methoxycarbonyl-benzolsulfochlorid,
6-Trifluormethyl-2-ethoxycarbonyl-benzolsulfochlorid (amorph),
6-Trifluormethyl-2-n-propoxycarbonyl-benzolsulfochlorid,
6-Trifluormethyl-2-i-propoxycarbonyl-benzolsulfochlorid (Fp.: 64°C),
6-Trifluormethyl-2-(oxetan-2-yl-oxycarbonyl)-benzolsulfochlorid,
6-Difluormethoxy-2-methoxycarbonyl-benzolsulfochlorid,
6-Difluormethoxy-2-ethoxycarbonyl-benzolsulfochlorid,
6-Difluormethoxy-2-n-propoxycarbonyl-benzolsulfochlorid,
6-Difluormethoxy-2-i-propoxycarbonyl-benzolsulfochlorid,
6-Difluormethoxy-2-(oxetan-2-yl-oxycarbonyl)-benzolsulfochlorid,
6-Trifluormethoxy-2-methoxycarbonyl-benzolsulfochlorid,
6-Trifluormethoxy-2-ethoxycarbonyl-benzolsulfochlorid,
6-Trifluormethoxy-2-n-propoxycarbonyl-benzolsulfochlorid,
6-Trifluormethoxy-2-i-propoxycarbonyl-benzolsulfochlorid,
6-Trifluormethoxy-2-(oxetan-2-yl-oxycarbonyl)-benzolsulfochlorid.

### Ausgangsstoffe der Forrnel (X):

### Beispiel (X-1)

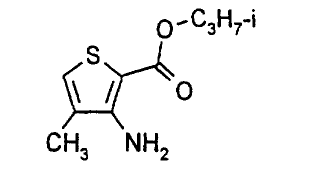

### Stufe i

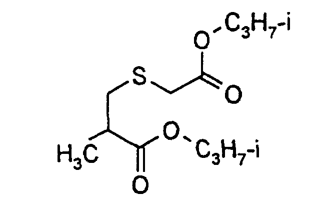

Eine Mischung aus 192 g (1,43 Mol) Mercaptoessigsäure-i-propylester und 183 g (1,43 Mol) Methacrylsäure-i-propylester wird unter Schütteln mit 5 Tropfen Piperidin versetzt. Die Mischung wird auf 50°C erwärmt, mit weiteren 20 Tropfen Piperidin versetzt und 12 Stunden bei einer Badtemperatur von 100°C gerührt. Anschließend wird unter vermindertem Druck destilliert.

Man erhält 288 g (77% der Theorie) 2-Methyl-3-(i-propoxycarbonylmethylthio)-propionsäure-i-propylester als farbloses Öl vom Siedebereich 135°C bis 140°C bei 2 mBar.

Analog können beispielsweise die folgenden Verbindungen hergestellt werden:
2-Methyl-3-(methoxycarbonylmethylthio)-propionsäure-methylester,
2-Methyl-3-(ethoxycarbonylmethylthio)-propionsäure-ethylester
   (Kp.: 105°C bis 112°C bei 1 mBar),
2-Methyl-3-(n-propoxycarbonylmethylthio)-propionsäure-n-propylester
   (Kp.: 145°C bis 147°C bei 3 mBar),
2-Methyl-3-(ethoxycarbonylmethylthio)-propionsäure-methylester
   (Kp.: 150°C bis 152°C bei 0,1 mBar),
2-Methyl-3-(n-propoxycarbonylmethylthio)-propionsäure-methylester
   (Kp.: 158°C bis 160°C bei 0,1 mBar),
2-Methyl-3-(i-propoxycarbonylmethylthio)-propionsäure-methylester
   (Kp.: 162°C bis 165°C bei 0,1 mBar),
2-Ethyl-3-(methoxycarbonylmethylthio)-propionsäure-methylester,
2-Ethyl-3-(ethoxycarbonylmethylthio)-propionsäure-ethylester,
2-Ethyl-3-(n-propoxycarbonylmethylthio)-propionsäure-n-propylester,
2-Ethyl-3-(i-propoxycarbonylmethylthio)-propionsäure-i-propylester,
2-Ethyl-3-(ethoxycarbonylmethylthio)-propionsäure-methylester,
2-Ethyl-3-(n-propoxycarbonylmethylthio)-propionsäure-methylester,
2-Ethyl-3-(i-propoxycarbonylmethylthio)-propionsäure-methylester,
2-Trifluormethyl-3-(methoxycarbonylmethylthio)-propionsäure-methylester,
2-Trifluormethyl-3-(ethoxycarbonylmethylthio)-propionsäure-ethylester,
2-Trifluormethyl-3-(n-propoxycarbonylmethylthio)-propionsäure-n-propylester,
2-Trifluormethyl-3-(i-propoxycarbonylmethylthio)-propionsäure-i-propylester,
2-Trifluormethyl-3-(ethoxycarbonylmethylthio)-propionsäure-methylester,
2-Trifluormethyl-3-(n-propoxycarbonylmethylthio)-propionsäure-methylester,
2-Trifluormethyl-3-(i-propoxycarbonylmethylthio)-propionsäüre-methylester,
2-Chlor-3-(methoxycarbonylmethylthio)-propionsäure-methylester,
2-Chlor-3-(ethoxycarbonylmethylthio)-propionsäure-ethylester,
2-Chlor-3-(n-propoxycarbonylmethylthio)-propionsäure-n-propylester,
2-Chlor-3-(i-propoxycarbonylmethylthio)-propionsäure-i-propylester,
2-Chlor-3-(ethoxycarbonylmethylthio)-propionsäure-methylester,
2-Chlor-3-(n-propoxycarbonylmethylthio)-propionsäure-methylester,
2-Chlor-3 -(i-propoxycarbonylmethylthio)-propionsäure-methylester.

### Stufe 2

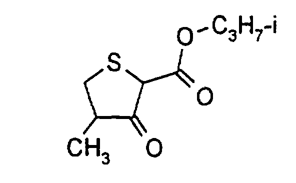

480 g (1,83 Mol) 2-Methyl-3-(i-propoxycarbonylmethylthio)-propionsäure-i-propylester werden bei Raumtemperatur (ca. 20°C) zu einer Suspension von 246 g (3,0 Mol) Natrium-i-propylat in 1 Liter Toluol tropfenweise gegeben und die Mischung wird dann ca. 12 Stunden bei ca. 90°C gerührt. Anschließend wird sie auf eiskalte 2N-Salzsäure gegossen. Dann wird dreimal mit Diethylether extrahiert und die vereinigten Extraktionslösungen werden zweimal mit Wasser gewaschen. Die organischePhase wird mit Magnesiumsulfat getrocknet und filtriert. Das Filtrat wird im Wasserstrahlvakuum eingeengt und der Rückstand unter vermindertem Druck destilliert.

Man erhält 254 g (69% der Theorie) 4-Methyl-3-oxo-tetrahydrothiophen-2-carbon säure-i-propylester als hellgelbes Öl vom Siedepunkt 119°C bis 120°C bei 1 mBar.

Analog können beispielsweise die folgenden Verbindungen hergestellt werden:
4-Methyl-3-oxo-tetrahydrothiophen-2-carbonsäure-methylester,
4-Methyl-3-oxo-tetrahydrothiophen-2-carbonsäure-ethylester
   (Kp.: 112°C bei 4 mBar),
4-Methyl-3-oxo-tetrahydrothiophen-2-carbonsäure-n-propylester
   (Kp.: 127°C bis 128°C bei 1 mBar),
4-Ethyl-3-oxo-tetrahydrothiophen-2-carbonsäure-methylester,
4-Ethyl-3-oxo-tetrahydrothiophen-2-carbonsäure-ethylester,
4-Ethyl-3-oxo-tetrahydrothiophen-2-carbonsäure-n-propylester,
4-Ethyl-3-oxo-tetrahydrothiophen-2-carbonsäure-i-propylester,
4-Trifluormethyl-3-oxo-tetrahydrothiophen-2-carbonsäure-methylester,
4-Trifluormethyl-3-oxo-tetrahydrothiophen-2-carbonsäure-ethylester,
4-Trifluormethyl-3-oxo-tetrahydrothiophen-2-carbonsäure-n-propylester,
4-Trifluormethyl-3-oxo-tetrahydrothiophen-2-carbonsäure-i-propylester,
4-Chlor-3-oxo-tetrahydrothiophen-2-carbonsäure-methylester,
4-Chlor-3-oxo-tetrahydrothiophen-2-carbonsäure-ethylester,
4-Chlor-3-oxo-tetrahydrothiophen-2-carbonsäure-n-propylester,
4-Chlor-3-oxo-tetrahydrothiophen-2-carbonsäure-i-propylester.

### Stufe 3

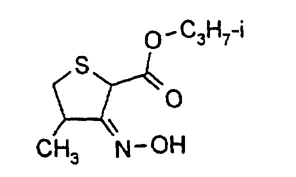

Eine Mischung aus 505 g (2,5 Mol) 4-Methyl-3-oxo-tetrahydrothiophen-2-carbonsäure-i-propylester, 497 g (7,2 Mol) Hydroxylamin-Hydrochlorid, 753 g (3,8 Mol) Bariumcarbonat und 2,5 Liter Isopropanol wird ca. 12 Stunden unter Rückfluß erhitzt. Anschließend wird abgesaugt, der Filterkuchen mit heißem Isopropanol gewaschen und das Filtrat im Wasserstrahlvakuum eingeengt. Der Rückstand wird in 2 Liter Diethylether aufgenommen, zweimal mit Wasser gewaschen, mit Magnesiumsulfat getrocknet und filtriert. Vom Filtrat wird das Lösungsmittel im Wasserstrahlvakuum sorgfältig abdestilliert.

Man erhält 445 g (82% der Theorie) 4-Methyl-3-hydroximino-tetrahydrothiophen-2-carbonsäure-i-propylester als hellgelbes öliges Rohprodukt, das ohne weitere Reinigung für die nächste Stufe eingesetzt wird.

Analog können beispielsweise die folgenden Verbindungen hergestellt werden:
4-Methyl-3-hydroximino-tetrahydrothiophen-2-carbonsäure-methylester,
4-Methyl-3-hydroximino-tetrahydrothiophen-2-carbonsäure-ethylester
   (farbloses Öl),
4-Methyl-3-hydroximino-tetrahydrothiophen-2-carbonsäure-n-propylester
   (hellgelbes Öl),
4-Ethyl-3-hydroximino-tetrahydrothiophen-2-carbonsäure-methylester,
4-Ethyl-3-hydroximino-tetrahydrothiophen-2-carbonsäure-ethylester,
4-Ethyl-3-hydroximino-tetrahydrothiophen-2-carbonsäure-n-propylester,
4-Ethyl-3-hydroximino-tetrahydrothiophen-2-carbonsäure-i-propylester,
4-Trifluormethyl-3-hydroximino-tetrahydrothiophen-2-carbonsäure-methylester,
4-Trifluormethyl-3-hydroximino-tetrahydrothiophen-2-carbonsäure-ethylester,
4-Trifluormethyl-3-hydroximino-tetrahydrothiophen-2-carbonsäure-n-propylester,
4-Trifluonnethyl-3-hydroximino-tetrahydrothiophen-2-carbonsäure-i-propylester,
4-Chlor-3-hydroximino-tetrahydrothiophen-2-carbonsäure-methylester,
4-Chlor-3-hydroximino-tetrahydrothiophen-2-carbonsäure-ethylester,
4-Chlor-3-hydroximino-tetrahydrothiophen-2-carbonsäure-n-propylester,
4-Chlor-3-hydroximino-tetrahydrothiophen-2-carbonsäure-i-propylester.

### Stufe 4

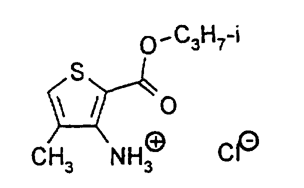

334 g (1,54 Mol) 4-Methyl-3-hydroximino-tetrahydrothiophen-2-carbonsäure-i-propylester werden in 5 Liter Methyl-t-butylether gelöst und mit einem Eisbad abgekühlt. Dann wird - nachdem das Eisbad wieder entfernt worden ist - zwei Stunden lang Chlorwasserstoff (Hydrogenchlorid) unter Rühren eingeleitet. Anschließend wird im Wasserstrahlvakuum eingeengt und der amorphe Rückstand aus Aceton kristallisiert.

Man erhält 358 g (99% der Theorie) 3-Amino-4-methyl-thiophen-2-carbonsäure-i-propylester-Hydrochlorid als beigefarbenen Feststoff vom Schmelzpunkt 153°C.

Analog können beispielsweise die folgenden Verbindungen hergestellt werden:
3-Amino-4-methyl-thiophen-2-carbonsäure-ethylester-Hydrochlorid
   (Fp.: 133°C),
3-Amino-4-methyl-thiophen-2-carbonsäure-n-propylester-Hydrochlorid
   (Fp.: 152°C),
3-Amino-4-ethyl-thiophen-2-carbonsäure-ethylester-Hydrochlorid,
3-Amino-4-ethyl-thiophen-2-carbonsäure-n-propylester-Hydrochiorid,
3-Amino-4-ethyl-thiophen-2-carbonsäure-i-propylester-Hydrochlorid,
3-Amino-4-trifluormethyl-thiophen-2-carbonsäure-ethylester-Hydrochlorid,
3-Amino-4-trifluormethyl-thiophen-2-carbonsäure-n-propylester-Hydrochlorid,
3-Amino-4-trifluormethyl-thiophen-2-carbonsäure-i-propylester-Hydrochlorid,
3-Amino-4-chlor-thiophen-2-carbonsäure-ethylester-Hydrochlorid,
3-Amino-4-chlor-thiophen-2-carbonsäure-n-propylester-Hydrochlorid,
3-Amino-4-chlor-thiophen-2-carbonsäure-i-propylester-Hydrochlorid.

### Stufe 5

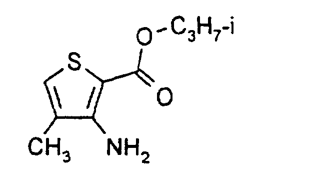

11,75 g (50 mMol) 3-Amino-4-methyl-thiophen-2-carbonsäure-i-propylester-Hydrochlorid werden in 100 ml Wasser gelöst. Zu dieser Lösung werden 150 ml Methylenchlorid gegeben und dann wird Natriumhydrogencarbonat portionsweise dazu gegeben, bis pH 7 überschritten ist. Die Mischung wird 8 Stunden gerührt, die organische Phase abgetrennt, mit Wasser gewaschen, mit Magnesiumsulfat getrocknet und filtriert. Das Filtrat wird im Wasserstrahlvakuum eingeengt und der amorphe Rückstand aus Petrolether kristallisiert.

Man erhält 7,2 g (73% der Theorie) 3-Amino-4-methyl-thiophen-2-carbonsäure-i-propylester als beigefarbenen Feststoff vom Schmelzpunkt 40°C.

Analog können beispielsweise die folgenden Verbindungen hergestellt werden:
3-Amino-4-methyl-thiophen-2-carbonsäure-ethylester (Fp.: 133°C),
3 -Amino-4-methyl-thiophen-2-carbonsäure-n-propylester (amorph),
3-Amino-4-ethyl-thiophen-2-carbonsäure-ethylester,
3-Amino-4-ethyl-thiophen-2-carbonsäure-n-propylester,
3-Amino-4-ethyl-thiophen-2-carbonsäure-i-propylester,
3-Amino-4-trifluormethyl-thiophen-2-carbonsäure-ethylester,
3-Amino-4-trifluormethyl-thiophen-2-carbonsäure-n-propylester,
3-Amino-4-trifluormethyl-thiophen-2-carbonsäure-i-propylester,
3-Amino-4-chlor-thiophen-2-carbonsäure-ethylester,
3-Amino-4-chlor-thiophen-2-carbonsäure-n-propylester,
3-Amino-4-chlor-thiophen-2-carbonsäure-i-propylester.

### Anwendungsbeispiele:

### Beispiel A

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit.

Nach drei Wochen wird der Schädigungsgrad der Pflanzen bonitiert in % Schädigung im Vergleich zur Entwicklung der unbehandelten Kontrolle.

Es bedeuten:
- 0% =: keine Wirkung (wie unbehandelte Kontrolle)
- 100% =: totale Vernichtung

In diesem Test zeigen beispielsweise die Verbindungen gemäß Herstellungsbeispiel 1, 3, 5, 6, 7, 8, 16, 17, 18, 19, 20, 21, 22, 23, 28, 30, 31, 32, 33, 34, 35, 37, 38, 39, 40, 41, 42, 43, 44, 45, 46, 47, 48, 51, 53, 54, 55, 56, 57, 58, 59, 61, 62, 63, 64, 65, 66, 67, 68, 70, 72, 76, 77, 80, 81, 82, 84, 85, 86, 89, 93, 108, 121, 129, 130, 131, 134, 135, 136, 140, 141, 142, 143, 144, 145, 146, 147, 148, 149, 150, 151, 152, 153, 154, 155, 156, 157, 158, 159, 160, 161, 162, 163, 164, 165, 166, 167 und 172 bei weitgehend guter Verträglichkeit gegenüber Kulturpflanzen, wie z.B. Mais und Soja, sehr starke Wirkung gegen Unkräuter.
"ai." = Wirkstoff("active ingredient")

**Tabelle A:**

| Pre-emergence-Test/Gewächshaus | | | | | | | | | |
|---|---|---|---|---|---|---|---|---|---|
| Wirkstoff gemäß Herstellungsbeispiel | Aufwandmenge (g ai,/ha) | Mais | Soja | Lolium | Poa | Sorghum | Ambrosia | Matricaria | Solanum |
| 5 | 30 | 0 | 20 | 95 | 95 | 90 | 95 | 95 | 95 |
| 1 | 30 | 10 | - | 95 | 95 | 90 | 95 | 95 | 95 |
| 6 | 125 | 0 | - | 95 | 95 | 95 | 95 | 95 | 95 |
| 7 | 60 | 0 | 0 | 95 | - | 95 | 95 | 95 | - |
| 8 | 60 | 30 | 0 | 95 | 90 | 80 | 90 | 95 | 95 |
| 16 | 30 | - | 0 | 95 | 95 | 90 | 95 | 95 | 95 |
| 17 | 30 | - | 0 | 80 | 95 | 60 | 95 | 95 | 95 |
| 18 | 60 | 10 | 0 | 80 | 95 | 90 | 95 | 95 | 95 |
| 19 | 60 | 0 | 0 | 95 | 95 | - | 95 | 95 | 90 |
| 20 | 125 | 10 | 0 | 95 | 95 | 90 | 95 | 95 | 95 |
| 21 | 125 | 0 | 0 | 95 | 95 | 95 | 95 | 95 | 95 |
| 22 | 125 | 20 | 10 | 95 | 95 | 80 | 95 | 95 | 95 |
| 23 | 125 | 0 | 0 | - | 80 | 60 | 90 | 95 | 90 |
| 28 | 60 | 0 | - | 95 | 95 | 95 | 95 | 95 | 95 |
| 30 | 30 | 0 | - | 95 | 95 | 80 | 95 | 95 | 95 |
| 31 | 15 | 10 | - | 95 | 95 | 80 | 95 | 95 | 95 |
| 32 | 60 | 20 | 20 | 95 | 95 | 60 | 95 | 95 | 95 |
| 33 | 125 | 10 | - | - | 90 | 80 | 90 | 95 | 95 |
| 34 | 60 | 10 | 0 | 60 | 70 | 80 | 90 | 95 | 90 |
| 35 | 60 | 0 | - | 95 | 95 | 95 | 95 | 100 | 95 |
| 37 | 60 | 10 | 10 | 90 | 70 | - | 80 | 95 | 90 |
| 38 | 60 | 0 | - | 95 | 70 | 60 | 95 | 95 | 95 |
| 39 | 60 | 0 | 20 | 95 | 90 | - | 95 | 70 | 90 |
| 40 | 60 | 10 | 30 | 90 | 95 | - | 95 | 95 | 95 |
| 41 | 60 | 0 | - | 60 | 80 | 60 | 95 | 95 | 90 |
| 42 | 60 | 10 | 10 | 95 | 95 | - | 95 | 95 | 95 |
| 43 | 60 | - | 10 | 95 | 95 | 80 | 95 | 95 | 95 |
| 44 | 8 | 30 | 0 | 95 | 95 | 95 | 95 | 95 | 95 |
| 45 | 125 | 10 | 20 | 90 | 95 | - | 95 | 90 | 95 |
| 46 | 8 | 0 | - | 80 | 95 | 80 | 95 | 95 | 95 |
| 47 | 125 | 10 | 10 | 90 | 90 | 60 | 95 | 95 | 95 |
| 48 | 125 | 30 | 20 | 95 | 95 | - | 95 | 100 | 95 |
| 51 | 125 | 0 | 30 | 80 | 80 | 80 | 95 | 95 | 95 |
| 53 | 125 | 0 | - | 95 | 95 | - | 95 | 95 | 95 |
| 54 | 30 | 0 | - | 95 | 95 | 80 | 95 | 95 | 95 |
| 55 | 125 | 10 | - | 100 | 90 | 60 | 100 | 100 | 100 |
| 56 | 125 | - | 0 | 60 | 80 | - | - | 100 | 95 |
| 57 | 60 | 0 | - | 95 | 95 | 95 | - | 95 | 95 |
| 58 | 125 | 20 | - | 80 | 95 | - | - | 100 | 95 |
| 59 | 125 | 0 | - | 100 | 70 | - | 95 | 100 | 95 |
| 61 | 125 | 0 | - | 95 | 95 | 80 | 100 | 95 | 95 |
| 62 | 125 | 0 | - | 80 | 90 | 60 | - | 95 | 95 |
| 63 | 60 | 10 | - | 100 | 95 | 80 | 100 | 100 | 100 |
| 64 | 60 | 10 | 30 | 90 | 60 | - | 100 | 100 | 100 |
| 65 | 125 | 20 | - | 80 | 80 | 80 | - | 95 | 95 |
| 66 | 125 | 0 | - | 100 | - | 100 | - | 100 | 100 |
| 67 | 60 | 0 | - | 100 | - | 90 | - | 100 | 100 |
| 68 | 125 | 5 | 10 | 80 | 90 | 60 | 95 | 100 | 95 |
| 70 | 60 | 10 | - | 90 | 90 | - | 90 | 100 | 100 |
| 76 | 60 | 0 | 30 | 95 | - | 95 | - | 100 | 95 |
| 77 | 60 | 0 | - | 95 | - | 90 | - | 100 | 90 |
| 80 | 125 | 0 | - | 95 | - | 90 | - | 100 | 95 |
| 81 | 60 | - | - | 100 | - | 95 | - | 100 | 100 |
| 82 | 60 | 0 | - | 60 | - | 90 | - | 100 | 100 |
| 84 | 125 | 5 | 30 | 95 | - | 90 | - | 95 | 100 |
| 85 | 125 | 60 | - | 95 | - | 95 | - | 100 | 100 |
| 86 | 125 | 10 | 40 | 80 | - | 70 | - | 100 | 95 |
| 89 | 125 | 0 | - | 100 | - | 90 | - | 100 | 100 |
| 93 | 60 | 0 | - | 90 | - | 80 | - | 95 | 90 |
| 108 | 125 | 5 | - | 90 | - | 95 | - | 95 | 90 |
| 129 | 250 | 10 | 10 | 95 | 95 | 90 | 60 | 90 | 95 |
| 130 | 250 | 10 | 0 | 95 | 95 | 80 | 95 | 95 | 95 |
| 131 | 250 | 0 | 0 | 95 | 90 | 90 | 95 | 80 | 95 |
| 134 | 500 | 0 | 20 | 95 | 90 | 70 | 80 | 90 | 95 |
| 135 | 125 | - | 40 | 95 | 95 | 90 | 90 | 90 | 95 |
| 136 | 125 | 30 | 20 | 95 | 95 | 90 | 80 | 90 | 95 |
| 140 | 30 | - | 10 | - | 95 | 95 | 80 | 80 | 95 |
| 141 | 15 | 30 | 0 | 90 | 80 | 80 | 90 | 80 | 90 |
| 142 | 15 | 20 | 10 | 95 | 80 | - | 80 | 80 | 80 |
| 143 | 60 | 20 | 0 | 90 | 90 | 90 | 80 | - | 90 |
| 144 | 125 | - | - | 90 | 90 | 95 | 80 | 95 | 95 |
| 145 | 60 | 0 | 0 | 95 | 95 | 95 | 95 | 60 | 95 |
| 3 | 60 | 10 | 20 | 90 | 70 | - | 80 | 95 | 80 |
| 146 | 60 | 0 | 10 | 80 | 90 | - | 80 | 80 | 90 |
| 147 | 15 | 30 | 0 | 95 | 90 | - | 70 | 70 | 80 |
| 148 | 60 | 40 | 0 | 80 | 95 | 90 | 80 | 80 | 95 |
| 149 | 125 | 0 | 30 | 95 | 95 | 80 | 95 | 95 | 95 |
| 150 | 125 | - | 10 | 80 | 90 | 80 | 70 | 80 | 80 |
| 151 | 15 | 20 | 10 | 90 | 90 | 50 | 80 | 90 | 90 |
| 152 | 125 | - | 20 | 95 | 95 | 95 | 95 | 80 | 95 |
| 153 | 125 | 0 | 30 | - | 95 | - | 95 | 70 | 95 |
| 154 | 60 | 0 | 0 | 95 | 95 | 95 | 70 | 70 | 90 |
| 155 | 60 | - | 40 | 95 | 95 | 95 | 90 | 80 | 95 |
| 156 | 60 | 0 | 10 | 70 | 90 | 70 | 70 | 95 | 70 |
| 157 | 60 | 0 | 20 | 90 | 80 | 80 | 95 | 100 | 90 |
| 158 | 30 | 10 | - | 95 | 95 | 60 | 95 | 95 | 95 |
| 159 | 30 | 10 | - | 90 | 95 | 60 | 95 | 95 | 95 |
| 160 | 60 | 30 | 10 | 90 | 90 | 60 | 90 | 95 | 90 |
| 161 | 60 | 0 | 20 | 95 | 95 | - | 95 | 95 | 95 |
| 162 | 60 | - | 0 | 95 | 95 | 90 | 80 | 95 | 95 |
| 163 | 4 | 20 | 0 | 80 | 95 | 60 | 80 | - | 90 |
| 164 | 8 | 20 | 0 | 95 | 95 | 90 | 100 | 70 | 95 |
| 165 | 8 | 0 | 10 | - | 90 | 70 | 80 | 90 | 80 |
| 166 | 15 | 40 | - | 90 | 90 | 70 | 80 | 95 | 90 |
| 167 | 60 | - | 30 | 80 | 95 | 95 | 90 | 95 | 90 |
| 172 | 125 | - | 60 | 90 | 95 | 90 | 90 | 95 | 95 |

### Beispiel B

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 I Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen beispielsweise die Verbindungen gemäß Herstellungsbeispiel 1, 5, 6, 7, 28, 30, 31, 32, 38, 44, 54, 63, 140, 141, 144, 151, 159, 162, 163, 164, 165 und 166 bei weitgehend guter Verträglichkeit gegenüber Kulturpflanzen, wie z.B. Mais, sehr starke Wirkung gegen Unkräuter.

**Tabelle B:**

| Post-emergence-Test/Gewächshaus | | | | | | | | | | |
|---|---|---|---|---|---|---|---|---|---|---|
| Wirkstoff gemäß Herstellungsbeispiel | Aufwandmenge (g ai./ha) | Mais | Digitaria | Echinochloa | Lolium | Sorghum | Amaranthus | Gallium | Matricaria | Stellaria |
| 5 | 30 | 10 | 95 | 80 | 70 | 90 | 100 | - | 95 | 90 |
| 1 | 125 | 60 | 95 | 95 | 90 | 95 | 100 | - | 100 | 100 |
| 6 | 400 | 20 | 90 | 95 | 95 | 80 | 100 | 90 | 100 | 100 |
| 7 | 60 | 15 | 95 | 95 | 95 | 100 | - | 95 | 95 | 100 |
| 28 | 60 | 10 | 90 | 80 | 60 | 95 | 100 | 80 | 90 | 100 |
| 30 | 60 | 15 | 90 | 70 | 60 | 95 | 100 | 85 | 100 | 100 |
| 31 | 30 | 5 | 95 | 95 | 90 | 80 | 100 | 80 | 100 | 95 |
| 32 | 60 | 70 | 70 | 100 | 70 | 95 | 95 | 70 | 80 | 90 |
| 38 | 60 | 10 | 70 | 90 | - | 95 | 100 | - | 95 | 95 |
| 44 | 60 | 50 | 60 | 90 | 70 | 100 | 100 | 95 | 95 | - |
| 54 | 60 | 30 | - | 95 | 95 | 95 | 100 | 85 | 100 | 100 |
| 63 | 60 | - | 50 | 90 | 70 | 80 | 100 | 90 | 95 | 100 |
| 140 | 250 | - | 95 | 90 | - | 95 | 100 | 95 | 90 | 70 |
| 141 | 30 | 10 | 70 | 90 | - | 90 | 100 | 90 | 70 | 95 |
| 144 | 250 | - | 95 | 95 | 70 | 95 | 100 | 95 | 70 | 95 |
| 151 | 30 | 60 | 90 | 70 | 60 | 90 | 95 | 90 | 90 | 90 |
| 159 | 60 | - | 60 | 80 | 60 | 70 | 95 | 95 | 100 | 100 |
| 162 | 60 | - | 70 | 80 | 90 | 80 | - | - | 60 | 90 |
| 163 | 60 | 60 | 80 | 70 | 80 | 95 | 100 | 95 | 100 | 95 |
| 164 | 60 | - | 60 | 80 | 70 | 95 | 100 | 90 | 100 | 95 |
| 165 | 60 | - | 50 | 80 | 50 | 90 | 95 | 95 | 95 | 95 |
| 166 | 60 | - | 70 | 70 | 50 | 90 | 95 | 95 | 100 | 95 |

## Patentansprüche

1. Substituierte Sulfonylamino(thio)carbonylverbindungen der allgemeinen Formel (I), 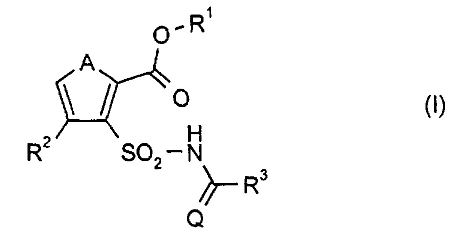 in welcher
A für Schwefel oder -CH=CH- steht,
Q für Sauerstoff oder Schwefel steht,
R¹ für gegebenenfalls durch Cyano, Nitro, Halogen oder C₁-C₄-Alkoxy substituiertes C₁-C₆-Alkyl, für jeweils gegebenenfalls durch Cyano oder Halogen substituiertes C₂-C₆-Alkenyl oder C2-C6-Alkinyl, für jeweils gegebenenfalls durch Cyano, Halogen oder C₁-C₄-Alkyl substituiertes C₃-C₆-Cycloalkyl, C₃-C₆-Cycloalkyl-C₁-C₄-alkyl, für jeweils gegebenenfalls durch Cyano, Nitro, Halogen, C₁-C₄-Alkyl, C₁-C₄-Halogenalkyl, C₁-C₄-Alkoxy oder C₁-C₄-Halogenalkoxy substituiertes Phenyl oder Phenyl-C₁-C₄-alkyl, oder für jeweils gegebenenfalls durch Cyano, Nitro, Halogen, C₁-C₄-Alkyl, C₁-C₄-Halogenalkyl, C₁-C₄-Alkoxy oder C₁-C₄-Halogenalkoxy substituiertes Heterocyclyl oder Heterocyclyl-C₁-C₄-alkyl steht, wobei jeweils die Heterocyclylgruppe aus der Reihe Oxetanyl, Thietanyl, Furyl, Tetrahydrofuryl, Thienyl, Tetrahydrothienyl, Oxazolyl, Isoxazolyl, Thiazolyl, Oxadiazolyl, Thiadiazolyl ausgewählt ist,
R² für Cyano, Nitro, Halogen, für jeweils gegebenenfalls durch Cyano, Halogen oder C₁-C₄-Alkoxy substituiertes C₁-C₄-Alkyl, C₁-C₄-Alkoxy, C₁-C₄-Alkoxy-carbonyl, C₁-C₄-Alkylthio, C₁-C₄-Alkylsulfinyl oder C₁-C₄-Alkylsulfonyl, oder für jeweils gegebenenfalls durch Cyano oder Halogen substituiertes C₂-C₄-Alkenyl, C₂-C₄-Alkinyl, C₂-C₄-Alkenyloxy oder C₂-C₄-Alkinyloxy steht, und
R³ für gegebenenfalls substituiertes Heterocyclyl der nachstehenden Formel steht, 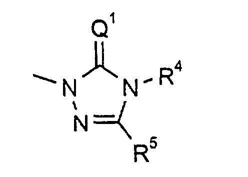 worin
Q¹ für Sauerstoff oder Schwefel steht sowie
R⁴ für Wasserstoff, Hydroxy, Amino, Cyano, für C₂-C₁₀-Alkylidenamino, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor, Chlor, Brom, Cyano, C₁-C₄-Alkoxy oder C₁-C₄-Alkoxy-carbonyl substituiertes C₁-C₆-Alkoxy, C₁-C₆-Alkylamino oder C₁-C₆-Alkyl-carbonylamino, für C₃-C₆-Alkenyloxy, für Di-(C₁-C₄-alkyl)-amino, für jeweils gegebenenfalls durch Fluor, Chlor, Brom, Cyano und/oder C₁-C₄-Alkyl substituiertes C₃-C₆-Cycloalkyl, C₃-C₆-Cycloalkylamino oder C₃-C₆-Cycloalkyl-C₁-C₄-alkyl, oder für jeweils gegebenenfalls durch Fluor, Chlor, Brom, Cyano, Nitro, C₁-C₄-Alkyl, Trifluormethyl und/oder C₁-C₄-Alkoxy substituiertes Phenyl oder Phenyl-C₁-C₄-alkyl steht, und
R⁵ für Wasserstoff, Hydroxy, Mercapto, Amino, Cyano, Fluor, Chlor, Brom, Iod, für gegebenenfalls durch Fluor, Chlor, Brom, Cyano, C₁-C₄-Alkoxy, C₁-C₄-Alkyl-carbonyl oder C₁-C₄-Alkoxycarbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor, Chlor, Cyano, C₁-C₄-Alkoxy oder C₁-C₄-Alkoxy-carbonyl substituiertes C₁-C₆-Alkoxy, C₁-C₆-Alkylthio, C₁-C₆-Alkylamino oder C₁-C₆-Alkyl-carbonylamino, für C₃-C₆-Alkenyloxy, C₃-C₆-Alkinyloxy, C₃-C₆-Alkenylthio, C₃-C₆-Alkinylthio, C₃-C₆-Alkenylamino oder C₃-C₆-Alkinylamino, für Di-(C₁-C₄-alkyl)-amino, für jeweils gegebenenfalls durch Methyl und/oder Ethyl substituiertes Aziridino, Pyrrolidino, Piperidino oder Morpholino, für jeweils gegebenenfalls durch Fluor, Chlor, Brom, Cyano und/oder C₁-C₄-Alkyl substituiertes C₃-C₆-Cycloalkyl, C₅-C₆-Cycloalkenyl, C₃-C₆-Cycloalkyloxy, C₃-C₆-Cycloalkylthio, C₃-C₆-Cycloalkylamino, C₃-C₆-Cycloalkyl-C₁-C₄-alkyl, C₃-C₆-Cycloalkyl-C₁-C₄-alkoxy, C₃-C₆-Cycloalkyl-C₁-C₄-alkylthio oder C₃-C₆-Cycloalkyl-C₁-C₄-alkylamino, oder für jeweils gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Phenyl, Phenyl-C₁-C₄-alkyl, Phenoxy, Phenyl-C₁-C₄-alkoxy, Phenylthio, Phenyl-C₁-C₄-alkylthio, Phenylamino oder Phenyl-C₁-C₄-alkylamino steht, oder
R⁴ und R⁵ zusammen für gegebenenfalls verzweigtes Alkandiyl mit 3 bis 11 Kohlenstoffatomen stehen, sowie die Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium, Tri-(C₁-C₄-alkyl)-sulfonium-, C₅- oder C₆-Cycloalkyl-ammonium- und Di-(C₁-C₂-alkyl)-benzyl-ammonium-Salze von Verbindungen der Formel (I).

2. Verbindungen der Formel (I) gemäß Anspruch 1, **dadurch gekennzeichnet, daß** darin
R¹ für jeweils gegebenenfalls durch Cyano, Fluor, Chlor, Methoxy oder Ethoxy substituiertes Methyl, Ethyl, n- oder i-Propyl, n-, i- oder s-Butyl, für jeweils gegebenenfalls durch Cyano, Fluor oder Chlor substituiertes Propenyl, Butenyl, Propinyl oder Butinyl, für jeweils gegebenenfalls durch Cyano, Fluor, Chlor, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclo-hexylmethyl, für jeweils gegebenenfalls durch Cyano, Fluor, Chlor, Brom, Methyl, Ethyl, Trifluormethyl, Methoxy, Ethoxy, Difluormethoxy oder Trifluormethoxy substituiertes Phenyl, Phenylmethyl oder Phenylethyl, oder für jeweils gegebenenfalls durch Cyano, Fluor, Chlor, Brom, Methyl, Ethyl, Methoxy oder Ethoxy substituiertes Heterocyclyl oder Heterocyclyl-methyl steht, wobei jeweils die Heterocyclylgruppe aus der Reihe Oxetanyl, Thietanyl, Furyl, Tetrahydrofuryl, Thienyl, Tetrahydrothienyl ausgewählt ist,
R² für Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i- oder s-Butyl, Methoxy, Ethoxy, n- oder i-Propoxy, Methoxycarbonyl, Ethoxycarbonyl, n- oder i-Propoxycarbonyl, Methylthio, Ethylthio, Methylsulfinyl, Ethylsulfinyl, Methylsulfonyl oder Ethylsulfonyl, oder für jeweils gegebenenfalls durch Cyano, Fluor oder Chlor substituiertes Propenyl, Butenyl, Propinyl, Butinyl, Propenyloxy oder Propinyloxy steht, und
R³ für gegebenenfalls substituiertes Heterocyclyl der nachtehenden Formel steht, 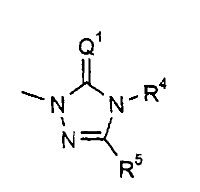 worin
Q¹ für Sauerstoff oder Schwefel steht sowie
R⁴ für Wasserstoff, Hydroxy, Amino, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylamino, Ethylamino, n- oder i-Propylamino, n-, i-, s- oder t-Butylamino, für Propenyloxy oder Butenyloxy, für Dimethylamino oder Diethylamino, für jeweils gegebenenfalls durch Fluor, Chlor, Methyl und/oder Ethyl substituiertes Cyclopropyl, Cyclobutyl, Cyclopentyl, Cyclohexyl, Cyclopropylamino, Cyclobutylamino, Cyclopentylamino, Cyclohexylamino, Cyclopropylmethyl, Cyclobutylmethyl, Cyclopentylmethyl oder Cyclohexylmethyl, oder für jeweils gegebenenfalls durch Fluor, Chlor, Methyl, Trifluormethyl und/oder Methoxy substituiertes Phenyl oder Benzyl steht, und
R⁵ für Wasserstoff, Hydroxy, Mercapto, Amino, Fluor, Chlor, Brom, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Ethenyl, Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, für Propenyloxy, Butenyloxy, Propinyloxy, Butinyloxy, Propenylthio, Propadienylthio Butenylthio, Propinylthio, Butinylthio, Propenylamino, Butenylamino, Propinylamino oder Butinylamino, für Dimethylamino, Diethylamino oder Dipropylamino, für jeweils gegebenenfalls durch Fluor, Chlor, Methyl und/oder Ethyl substituiertes Cyclopropyl, Cyclobutyl, Cyclopentyl, Cyclohexyl, Cyclopentenyl, Cyclohexenyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopropylmethylthio, Cyclobutylmethylthio, Cyclopentylmethylthio, Cyclohexylmethylthio, Cyclopropylmethylamino, Cyclobutylmethylamino, Cyclopentylmethylamino oder Cyclohexylmethylamino, oder für jeweils gegebenenfalls durch Fluor, Chlor, Methyl, Trifluormethyl, Methoxy und/oder Methoxycarbonyl substituiertes Phenyl, Benzyl, Phenoxy, Benzyloxy, Phenylthio, Benzylthio, Phenylamino oder Benzylamino steht, oder
R⁴ und R⁵ zusammen für gegebenenfalls verzweigtes Alkandiyl mit 3 bis 11 Kohlenstoffatomen stehen.

3. Verbindungen der Formel (I) gemäß Anspruch 1, **dadurch gekennzeichnet, daß** darin
R¹ für Methyl, Ethyl, n- oder i-Propyl, 2-Cyano-ethyl, 2-Fluor-ethyl, 2,2-Difluor-ethyl, 2,2,2-Trifluor-ethyl, 2-Chlor-ethyl, 2,2-Dichlorethyl, 2,2,2-Trichlor-ethyl, 2-Methoxy-ethyl, 2-Ethoxy-ethyl oder Oxetanyl steht,
R² für Fluor, Chlor, Brom, Methyl, Ethyl, n- oder i-Propyl, n-, i- oder s-Butyl, Trifluormethyl, Methoxy, Ethoxy, n- oder i-Propoxy, Difluormethoxy, Trifluormethoxy oder Trifluorethoxy steht, und
R³ für gegebenenfalls substituiertes Triazolinyl der nachstehenden Formel steht, 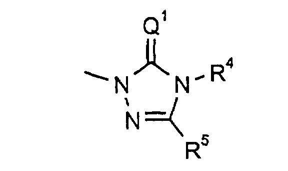 worin
Q¹ für Sauerstoff oder Schwefel steht sowie
R⁴ für jeweils gegebenenfalls durch Fluor, Chlor, Methoxy oder Ethoxy substituiertes Methyl, Ethyl, n- oder i-Propyl, für jeweils gegebenenfalls durch Fluor oder Chlor substituiertes Propenyl oder Propinyl, für Methoxy, Ethoxy, n- oder i-Propoxy, Methylamino, Ethylamino, n- oder i-Propylamino, für Propenyloxy, für Dimethylamino oder für Cyclopropyl steht, und
R⁵ für Chlor oder Brom, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Ethenyl, Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, für Propenyloxy, Butenyloxy, Propinyloxy, Butinyloxy, Propenylthio, Propadienylthio, Butenylthio, Propinylthio, Butinylthio, Propenylamino, Butenylamino, Propinylamino oder Butinylamino, für Dimethylamino, Diethylamino oder Dipropylamino, für jeweils gegebenenfalls durch Fluor, Chlor, Methyl und/oder Ethyl substituiertes Cyclopropyl, Cyclobutyl, Cyclopentyl, Cyclohexyl, Cyclopentenyl, Cyclohexenyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopropylmethylthio, Cyclobutylmethylthio, Cyclopentylmethylthio, Cyclohexylmethylthio, Cyclopropylmethylamino, Cyclobutylmethylamino, Cyclopentylmethylamino oder Cyclohexylmethylamino, oder für jeweils gegebenenfalls durch Fluor, Chlor, Methyl, Trifluormethyl, Methoxy und/oder Methoxycarbonyl substituiertes Phenyl, Benzyl, Phenoxy, Benzyloxy, Phenylthio, Benzylthio, Phenylamino oder Benzylamino steht, oder
R⁴ und R⁵ zusammen für gegebenenfalls verzweigtes Alkandiyl mit 3 bis 11 Kohlenstoffatomen stehen.

4. Verfahren zur Herstellung von Verbindungen der Formel (I) gemäß Anspruch 1, **dadurch gekennzeichnet, daß** man
(a) Sulfonamide der allgemeinen Formel (II) 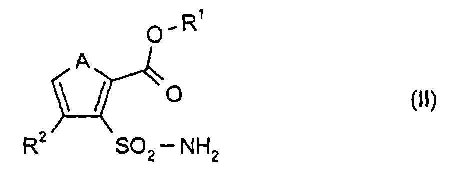 in welcher
A, R¹ und R² die in Anspruch 1 angegebene Bedeutung haben,
mit (Thio)Carbonsäurederivaten der allgemeinen Formel (III) 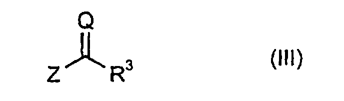 in welcher
Q und R³ die in Anspruch 1 angegebene Bedeutung haben und
Z für Halogen, Alkoxy, Aryloxy oder Arylalkoxy steht,
gegebenenfalls in Gegenwart eines Säureakzeptors und gegebenenfalls in Gegenwart eines Verdünnungsmittels umsetzt,
oder daß man
(b) Sulfonyliso(thio)cyanate der allgemeinen Formel (IV) 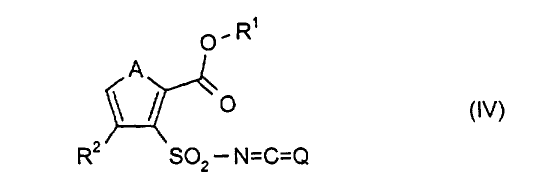 in welcher
A, Q, R¹ und R² die oben angegebene Bedeutung haben,
mit Heterocyclen der allgemeinen Formel (V)
H-R³ (V)
in welcher
R³ die oben angegebene Bedeutung hat,
gegebenenfalls in Gegenwart eines Reaktionshilfsmittels und gegebenenfalls in Gegenwart eines Verdünnungsmittels umsetzt,
oder daß man
(c) Sulfonsäurechloride der allgemeinen Formel (VI) 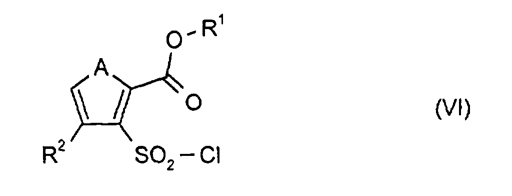 in welcher
A, R¹ und R² die oben angegebene Bedeutung haben,
mit Heterocyclen der allgemeinen Formel (V)
H-R³ (V)
in welcher
R³ die oben angegebene Bedeutung hat,
und Metall(thio)cyanaten der allgemeinen Formel (VII)
MQCN (VII)
in welcher
Q die oben angegebene Bedeutung hat,
gegebenenfalls in Gegenwart eines Reaktionshilfsmittels und gegebenenfalls in Gegenwart eines Verdünnungsmittels umsetzt,
oder daß man
(d) Sulfonsäurechloride der allgemeinen Formel (VI) 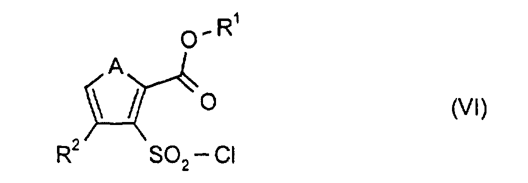 in welcher
A, R¹ und R² die oben angegebene Bedeutung haben,
mit (Thio)Carbonsäureamiden der allgemeinen Formel (VIII) 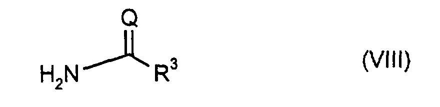 in welcher
Q und R³ die oben angegebene Bedeutung haben,
gegebenenfalls in Gegenwart eines Säureakzeptors und gegebenenfalls in Gegenwart eines Verdünnungsmittels umsetzt,
oder daß man
(e) Sulfonylamino(thio)carbonylverbindungen der allgemeinen Formel (IX) 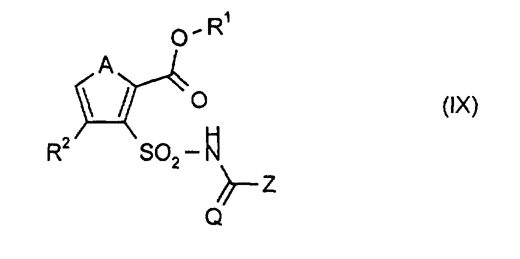 in welcher
A, Q, R¹ und R² die oben angegebene Bedeutung haben und
Z für Halogen, Alkoxy, Aryloxy oder Arylalkoxy steht,
mit Heterocyclen der allgemeinen Formel (V)
H-R³ (V)
in welcher
R³ die oben angegebene Bedeutung hat,
gegebenenfalls in Gegenwart eines Säureakzeptors und gegebenenfalls in Gegenwart eines Verdünnungsmittels umsetzt,
und gegebenenfalls die nach den Verfahren (a), (b), (c), (d) oder (e) erhaltenen Verbindungen der Formel (I) nach üblichen Methoden in Salze überführt.

5. Herbizide Mittel, **gekennzeichnet durch** einen Gehalt an mindestens einer Verbindung der Formel (I) oder einem ihrer Salze gemäß Anspruch 1.

6. Verwendung von Verbindungen der allgemeinen Formel (I) oder deren Salzen gemäß Anspruch 1 zur Bekämpfung von unerwünschtem Pflanzenwachstum.

7. Verfahren zur Bekämpfung von Unkräutern, **dadurch gekennzeichnet, daß** man Verbindungen der allgemeinen Formel (I) oder deren Salze gemäß Anspruch 1 auf die Unkräuter oder ihren Lebensraum einwirken läßt.

8. Verfahren zur Herstellung von herbiziden Mitteln, **dadurch gekennzeichnet, daß** man Verbindungen der allgemeinen Formel (I) oder deren Salze gemäß Anspruch 1 mit Streckmitteln und/oder oberflächenaktiven Mitteln vermischt.

9. Sulfonsäurechloride der Formel (IVa) 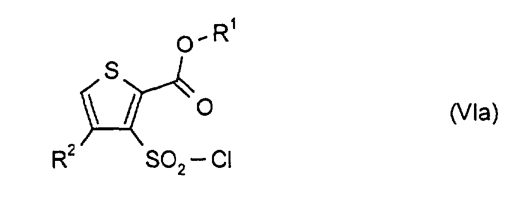 in welcher
R¹ und R² wie in Anspruch 1 definiert sind.

10. Sulfonamide der Formel (IIa) 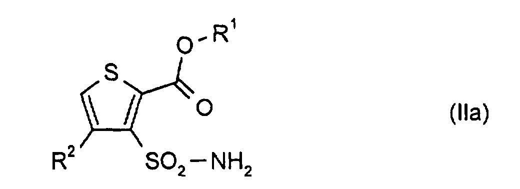 in welcher
R¹ für jeweils gegebenenfalls durch Cyano, Fluor, Chlor, Methoxy oder Ethoxy substituiertes Methyl, Ethyl, n- oder i-Propyl, n-, i- oder s-Butyl, für jeweils gegebenenfalls durch Cyano, Fluor oder Chlor substituiertes Propenyl, Butenyl, Propinyl oder Butinyl, für jeweils gegebenenfalls durch Cyano, Fluor, Chlor, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclo-hexylmethyl, für jeweils gegebenenfalls durch Cyano, Fluor, Chlor, Brom, Methyl, Ethyl, Trifluormethyl, Methoxy, Ethoxy, Difluormethoxy oder Trifluormethoxy substituiertes Phenyl, Phenylmethyl oder Phenylethyl, oder für jeweils gegebenenfalls durch Cyano, Fluor, Chlor, Brom, Methyl, Ethyl, Methoxy oder Ethoxy substituiertes Heterocyclyl oder Heterocyclyl-methyl steht, wobei jeweils die Heterocyclylgruppe aus der Reihe Oxetanyl, Thietanyl, Furyl, Tetrahydrofuryl, Thienyl, Tetrahydrothienyl ausgewählt ist, und
R² für Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i- oder s-Butyl, Methoxy, Ethoxy, n- oder i-Propoxy, Methoxycarbonyl, Ethoxycarbonyl, n- oder i-Propoxycarbonyl, Methylsulfinyl, Ethylsulfinyl, Methylsulfonyl oder Ethylsulfonyl, oder für jeweils gegebenenfalls durch Cyano, Fluor oder Chlor substituiertes Propenyl, Butenyl, Propinyl, Butinyl, Propenyloxy oder Propinyloxy steht, und

11. Verbindungen gemäß Anspruch 1, bei denen die folgenden Kombinationen der Restedefinitionen A, Q, R¹, R² und R³ vorliegen:

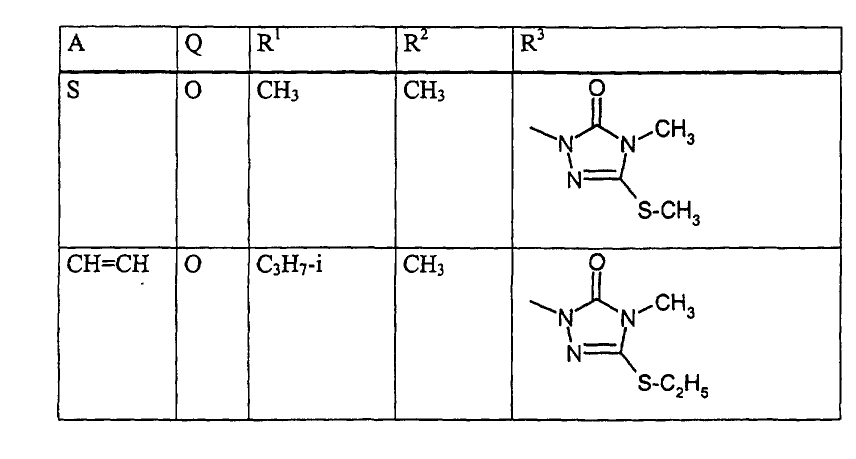

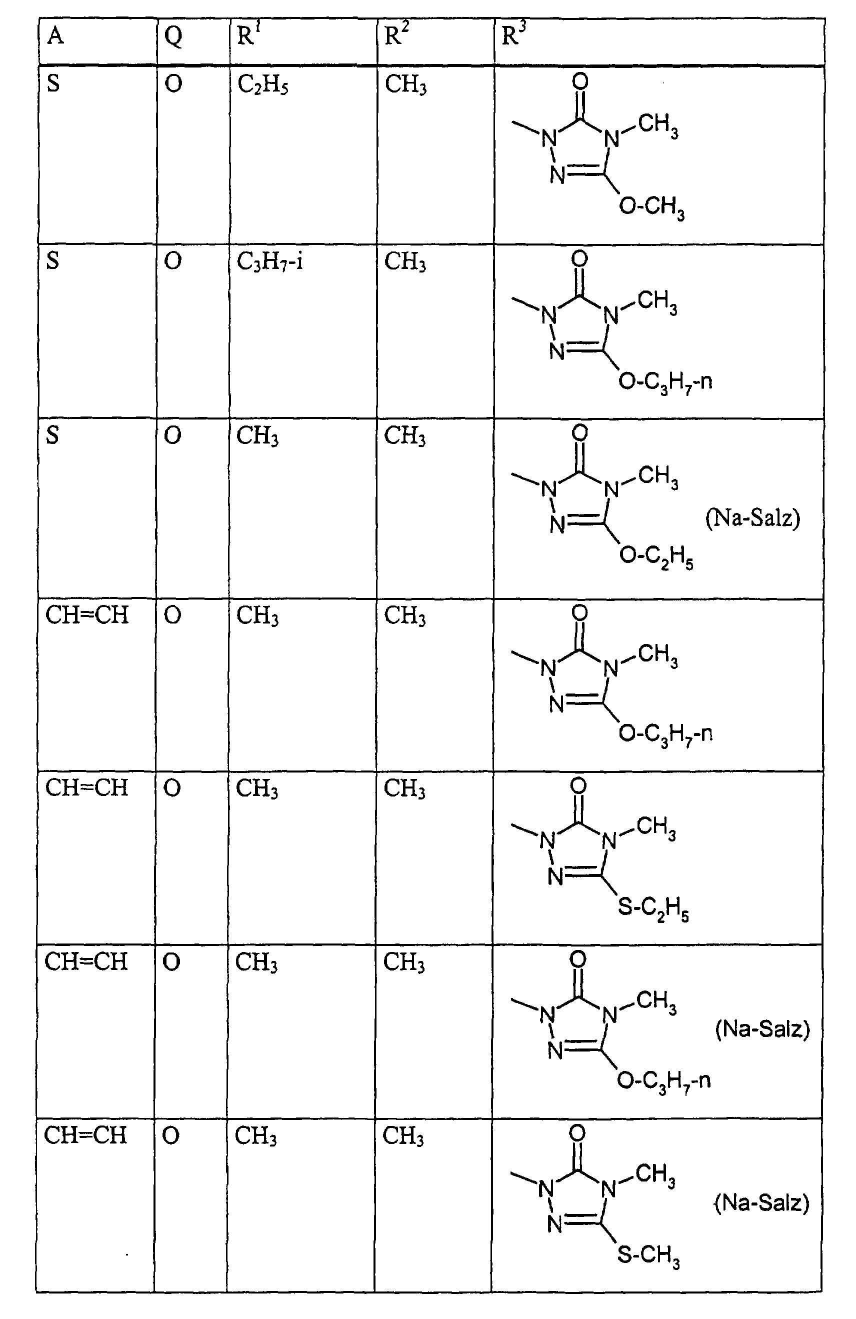

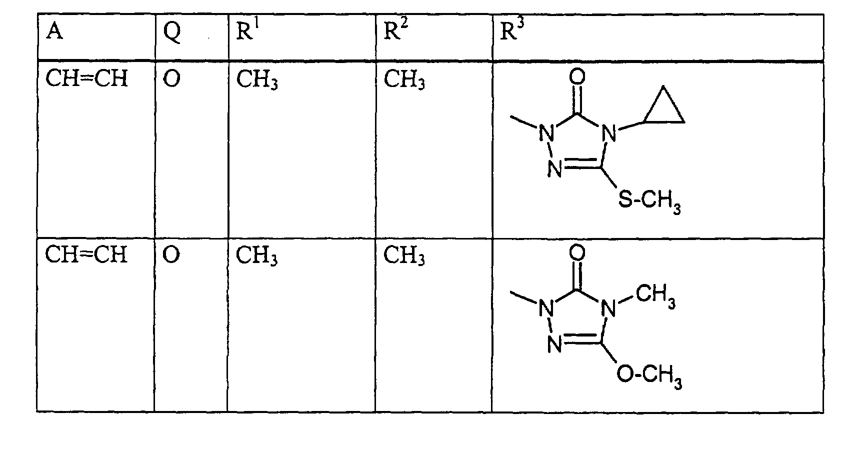

## Claims

1. Substituted sulphonylamino(thio)carbonyl compounds of the general formula (I), 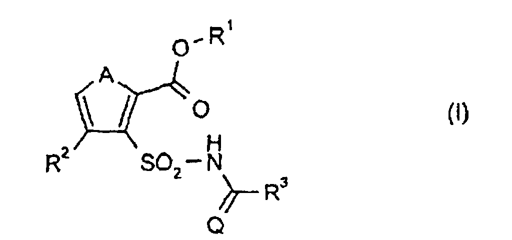 in which
A represents sulphur or -CH=CH-,
Q represents oxygen or sulphur,
R¹ represents optionally cyano-, nitro-, halogen- or C₁-C₄-alkoxy-substituted C₁-C₆-alkyl, represents respectively optionally cyano- or halogen-substituted C₂-C₆-alkenyl or C₂-C₆-alkinyl, represents respectively optionally cyano-, halogen- or C₁-C₄-alkyl-substituted C₃-C₆-cycloalkyl, C₃-C₆-cycloalkyl-C₁-C₄-alkyl, represents respectively optionally cyano-, nitro-, halogen-, C₁-C₄-alkyl-, C₁-C₄-halogenoalkyl-, C₁-C₄-alkoxy- or C₁-C₄-halogenoalkoxy-substituted phenyl or phenyl-C₁-C₄-alkyl, or represents respectively optionally cyano-, nitro-, halogen-, C₁-C₄-alkyl-, C₁-C₄-halogenoalkyl-, C₁-C₄-alkoxy- or C₁-C₄-halogenoalkoxy-substituted heterocyclyl or heterocyclyl-C₁-C₄-alkyl, where in each case the heterocyclyl group is selected from the group consisting of oxetanyl, thietanyl, furyl, tetrahydrofuryl, thienyl, tetrahydrothienyl, oxazolyl, isoxazolyl, thiazolyl, oxadiazolyl, thiadiazolyl,
R² represents cyano, nitro, halogen, represents respectively optionally cyano-, halogen- or C₁-C₄-alkoxy-substituted C₁-C₄-alkyl, C₁-C₄-alkoxy, C₁-C₄-alkoxy-carbonyl, C₁-C₄-alkylthio, C₁-C₄-alkylsulphinyl or C₁-C₄-alkylsulphonyl, or represents respectively optionally cyano- or halogen-substituted C₂-C₄-alkenyl, C₂-C₄-alkinyl, C₂-C₄-alkenyloxy or C₂-C₄-alkinyloxy, and
R³ represents optionally substituted heterocyclyl of the formula below, 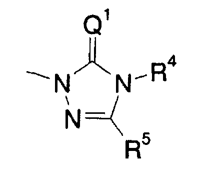 in which
Q¹ represents oxygen or sulphur and
R⁴ represents hydrogen, hydroxyl, amino, cyano, represents C₂-C₁₀-alkylidene-amino, represents optionally fluorine-, chlorine-, bromine-, cyano-, C₁-C₄-alkoxy-, C₁-C₄-alkyl-carbonyl- or C₁-C₄-alkoxy-carbonyl-substituted C₁-C₆-alkyl, represents respectively optionally fluorine-, chlorine- and/or bromine-substituted C₂-C₆-alkenyl or C₂-C₆-alkinyl, represents respectively optionally fluorine-, chlorine-, bromine-, cyano-, C₁-C₄-alkoxy- or C₁-C₄-alkoxy-carbonyl-substituted C₁-C₆-alkoxy, C₁-C₆-alkylamino or C₁-C₆-alkyl-carbonyl-amino, represents C₃-C₆-alkenyloxy, represents di-(C₁-C₄-alkyl)-amino, represents respectively optionally fluorine-, chlorine-, bromine-, cyano- and/or C₁-C₄-alkyl-substituted C₃-C₆-cycloalkyl, C₃-C₆-cycloalkyl-amino or C₃-C₆-cycloalkyl-C₁-C₄-alkyl, or represents respectively optionally fluorine-, chlorine-, bromine-, cyano-, nitro-, C₁-C₄-alkyl-, trifluoromethyl- and/or C₁-C₄-alkoxy-substituted phenyl or phenyl-C₁-C₄-alkyl, and
R⁵ represents hydrogen, hydroxyl, mercapto, amino, cyano, fluorine, chlorine, bromine, iodine, represents optionally fluorine-, chlorine-, bromine-, cyano-, C₁-C₄-alkoxy-, C₁-C₄-alkyl-carbonyl- or C₁-C₄-alkoxy-carbonyl-substituted C₁-C₆-alkyl, represents respectively optionally fluorine-, chlorine- and/or bromine-substituted C₂-C₆-alkenyl or C₂-C₆-alkinyl, represents respectively optionally fluorine-, chlorine-, cyano-, C₁-C₄-alkoxy- or C₁-C₄-alkoxy-carbonyl-substituted C₁-C₆-alkoxy, C₁-C₆-alkylthio, C₁-C₆-alkylamino or C₁-C₆-alkyl-carbonylamino, represents C₃-C₆-alkenyloxy, C₃-C₆-alkinyloxy, C₃-C₆-alkenylthio, C₃-C₆-alkinylthio, C₃-C₆-alkenylamino or C₃-C₆-alkinylamino, represents di-(C₁-C₄-alkyl)-amino, represents respectively optionally methyl- and/or ethyl-substituted aziridino, pyrrolidino, piperidino or morpholino, represents respectively optionally fluorine-, chlorine-, bromine-, cyano- and/or C₁-C₄-alkyl-substituted C₃-C₆-cycloalkyl, C₅-C₆-cycloalkenyl, C₃-C₆-cycloalkyloxy, C₃-C₆-cycloalkylthio, C₃-C₆-cycloalkylamino, C₃-C₆-cycloalkyl-C₁-C₄-alkyl, C₃-C₆-cycloalkyl-C₁-C₄-alkoxy, C₃-C₆-cycloalkyl-C₁-C₄-alkylthio or C₃-C₆-cycloalkyl-C₁-C₄-alkylamino, or represents respectively optionally fluorine-, chlorine-, bromine-, cyano-, nitro-, C₁-C₄-alkyl-, trifluoromethyl-, C₁-C₄-alkoxy- and/or C₁-C₄-alkoxy-carbonyl-substituted phenyl, phenyl-C₁-C₄-alkyl, phenoxy, phenyl-C₁-C₄-alkoxy, phenylthio, phenyl-C₁-C₄-alkylthio, phenylamino or phenyl-C₁-C₄-alkylamino, or
R⁴ and R⁵ together represent optionally branched alkanediyl having 3 to 11 carbon atoms,
and the sodium, potassium, magnesium, calcium, ammonium, C₁-C₄-alkyl-ammonium, di-(C₁-C₄-alkyl)-ammonium, tri-(C₁-C₄-alkyl)-ammonium, tetra-(C₁-C₄-alkyl)-ammonium, tri-(C₁-C₄-alkyl)-sulphonium, C₅- or C₆-cycloalkyl-ammonium and di-(C₁-C₂-alkyl)-benzyl-ammonium salts of compounds of the formula (I).

2. Compounds of the formula (I) according to Claim 1, **characterized in that**
R¹ represents respectively optionally cyano-, fluorine-, chlorine-, methoxy- or ethoxy-substituted methyl, ethyl, n- or i-propyl, n-, i- or s-butyl, represents respectively optionally cyano-, fluorine- or chlorine-substituted propenyl, butenyl, propinyl or butinyl, represents respectively optionally cyano-, fluorine-, chlorine-, methyl- or ethyl-substituted cyclopropyl, cyclobutyl, cyclopentyl, cyclohexyl, cyclopropylmethyl, cyclobutylmethyl, cyclopentylmethyl or cyclohexylmethyl, represents respectively optionally cyano-, fluorine-, chlorine-, bromine-, methyl-, ethyl-, trifluoromethyl-, methoxy-, ethoxy-, difluoromethoxy- or trifluoromethoxy-substituted phenyl, phenylmethyl or phenylethyl, or represents respectively optionally cyano-, fluorine-, chlorine-, bromine-, methyl-, ethyl-, methoxy- or ethoxy-substituted heterocyclyl or heterocyclyl-methyl, where in each case the heterocyclyl group is selected from the group consisting of oxetanyl, thietanyl, furyl, tetrahydrofuryl, thienyl, tetrahydrothienyl,
R² represents cyano, fluorine, chlorine, bromine, represents respectively optionally cyano-, fluorine-, chlorine-, methoxy- or ethoxy-substituted methyl, ethyl, n- or i-propyl, n-, i- or s-butyl, methoxy, ethoxy, n- or i-propoxy, methoxycarbonyl, ethoxycarbonyl, n- or i-propoxycarbonyl, methylthio, ethylthio, methylsulphinyl, ethylsulphinyl, methylsulphonyl or ethylsulphonyl, or represents respectively optionally cyano-, fluorine- or chlorine-substituted propenyl, butenyl, propinyl, butinyl, propenyloxy or propinyloxy, and
R³ represents optionally substituted heterocyclyl of the formula below, 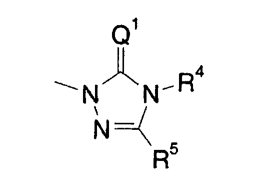 in which
Q¹ represents oxygen or sulphur and
R⁴ represents hydrogen, hydroxyl, amino, represents respectively optionally fluorine-, chlorine-, cyano-, methoxy- or ethoxy-substituted methyl, ethyl, n- or i-propyl, n-, i-, s- or t-butyl, represents respectively optionally fluorine-, chlorine- or bromine-substituted propenyl, butenyl, propinyl or butinyl, represents respectively optionally fluorine-, chlorine-, cyano-, methoxy- or ethoxy-substituted methoxy, ethoxy, n- or i-propoxy, n-, i-, s- or t-butoxy, methylamino, ethylamino, n- or i-propylamino, n-, i-, s- or t-butylamino, represents propenyloxy or butenyloxy, represents dimethylamino or diethylamino, represents respectively optionally fluorine-, chlorine-, methyl- and/or ethyl-substituted cyclopropyl, cyclobutyl, cyclopentyl, cyclohexyl, cyclopropylamino, cyclobutylamino, cyclopentylamino, cyclohexylamino, cyclopropylmethyl, cyclobutylmethyl, cyclopentylmethyl or cyclohexylmethyl, or represents respectively optionally fluorine-, chlorine-, methyl-, trifluoromethyl- and/or methoxy-substituted phenyl or benzyl, and
R⁵ represents hydrogen, hydroxyl, mercapto, amino, fluorine, chlorine, bromine, represents respectively optionally fluorine-, chlorine-, cyano-, methoxy- or ethoxy-substituted methyl, ethyl, n- or i-propyl, n-, i-, s- or t-butyl, represents respectively optionally fluorine-, chlorine- or bromine-substituted ethenyl, propenyl, butenyl, propinyl or butinyl, represents respectively optionally fluorine-, chlorine-, cyano-, methoxy- or ethoxy-substituted methoxy, ethoxy, n- or i-propoxy, n-, i-, s- or t-butoxy, methylthio, ethylthio, n- or i-propylthio, n-, i-, s- or t-butylthio, methylamino, ethylamino, n- or i-propylamino, n-, i-, s- or t-butylamino, represents propenyloxy, butenyloxy, propinyloxy, butinyloxy, propenylthio, propadienylthio, butenylthio, propinylthio, butinylthio, propenylamino, butenylamino, propinylamino or butinylamino, represents dimethylamino, diethylamino or dipropylamino, represents respectively optionally fluorine-, chlorine-, methyl- and/or ethyl-substituted cyclopropyl, cyclobutyl, cyclopentyl, cyclohexyl, cyclopentenyl, cyclohexenyl, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino, cyclohexylamino, cyclopropylmethyl, cyclobutylmethyl, cyclopentylmethyl, cyclohexylmethyl, cyclopropylmethoxy, cyclobutylmethoxy, cyclopentylmethoxy, cyclohexylmethoxy, cyclopropylmethylthio, cyclobutylmethylthio, cyclopentyl-methylthio, cyclohexylmethylthio, cyclopropylmethylamino, cyclobutylmethylamino, cyclopentylmethylamino or cyclohexyl-methylamino, or represents respectively optionally fluorine-, chlorine-, methyl-, trifluoromethyl-, methoxy- and/or methoxy-carbonyl-substituted phenyl, benzyl, phenoxy, benzyloxy, phenylthio, benzylthio, phenylamino or benzylamino, or
R⁴ and R⁵ together represent optionally branched alkanediyl having 3 to 11 carbon atoms.

3. Compounds of the formula (I) according to Claim 1, **characterized in that**
R¹ represents methyl, ethyl, n- or i-propyl, 2-cyano-ethyl, 2-fluoro-ethyl, 2,2-difluoroethyl, 2,2,2-trifluoro-ethyl, 2-chloro-ethyl, 2,2-dichloro-ethyl, 2,2,2-trichloro-ethyl, 2-methoxy-ethyl, 2-ethoxy-ethyl or oxetanyl,
R² represents fluorine, chlorine, bromine, methyl, ethyl, n- or i-propyl, n-, i- or s-butyl, trifluoromethyl, methoxy, ethoxy, nor i-propoxy, difluoromethoxy, trifluoromethoxy or trifluoroethoxy, and
R³ represents optionally substituted triazolinyl of the formula below 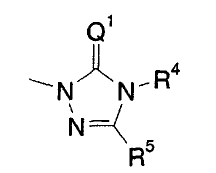 in which
Q¹ represents oxygen or sulphur and
R⁴ represents respectively optionally fluorine-, chlorine-, methoxy- or ethoxy-substituted methyl, ethyl, n- or i-propyl, represents respectively optionally fluorine- or chlorine-subsituted propenyl or propinyl, represents methoxy, ethoxy, n- or i-propoxy, methylamino, ethylamino, n- or i-propylamino, represents propenyloxy, represents dimethylamino or represents cyclopropyl, and
R⁵ represents chlorine or bromine, represents respectively optionally fluorine-, chlorine-, cyano-, methoxy- or ethoxy-substituted methyl, ethyl, n- or i-propyl, n-, i-, s- or t-butyl, represents respectively optionally fluorine-, chlorine- or bromine-substituted ethenyl, propenyl, butenyl, propinyl or butinyl, represents respectively optionally fluorine-, chlorine-, cyano-, methoxy- or ethoxy-subsituted methoxy, ethoxy, n- or i-propoxy, n-, i-, s- or t-butoxy, methylthio, ethylthio, n- or i-propylthio, n-, i-, s- or t-butylthio, methylamino, ethylamino, n- or i-propylamino, n-, i-, s- or t-butylamino, represents propenyloxy, butenyloxy, propinyloxy, butinyloxy, propenylthio, propadienylthio, butenylthio, propinylthio, butinylthio, propenylamino, butenylamino, propinylamino or butinylamino, represents dimethylamino, diethylamino or dipropylamino, represents respectively optionally fluorine-, chlorine-, methyl- and/or ethyl-substituted cyclopropyl, cyclobutyl, cyclopentyl, cyclohexyl, cyclopentenyl, cyclohexenyl, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino, cyclohexylamino, cyclopropylmethyl, cyclobutylmethyl, cyclopentylmethyl, cyclohexylmethyl, cyclopropylmethoxy, cyclobutylmethoxy, cyclopentylmethoxy, cyclohexylmethoxy, cyclopropylmethylthio, cyclobutylmethylthio, cyclopentyl-methylthio, cyclohexylmethylthio, cyclopropylmethylamino, cyclobutylmethylamino, cyclopentylmethylamino or cyclohexyl-methylamino, or represents respectively optionally fluorine-, chlorine-, methyl-, trifluoromethyl-, methoxy- and/or methoxycarbonyl-substituted phenyl, benzyl, phenoxy, benzyloxy, phenylthio, benzylthio, phenylamino or benzylamino, or
R⁴ and R⁵ together represent optionally branched alkanediyl having 3 to 11 carbon atoms.

4. Process for preparing compounds of the formula (I) according to Claim 1, **characterized in that**
(a) sulphonamides of the general formula (II) 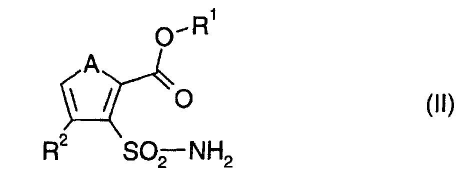 in which
A, R¹ and R² are each as defined in Claim 1,
are reacted with (thio)carboxylic acid derivatives of the general formula (III) 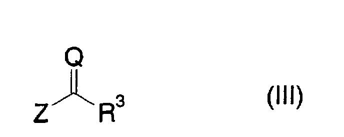 in which
Q and R³ are each as defined in Claim 1 and
Z represents halogen, alkoxy, aryloxy or arylalkoxy,
if appropriate in the presence of an acid acceptor and if appropriate in the presence of a diluent,
or **in that**
(b) sulphonyl iso(thio)cyanates of the general formula (IV) 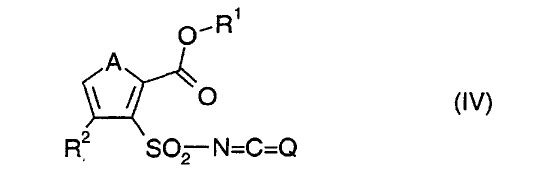 in which
A, Q, R¹ and R² are each as defined above,
are reacted with heterocycles of the general formula (V)
H-R³ (V)
in which
R³ is as defined above,
if appropriate in the presence of a reaction auxiliary and if appropriate in the presence of a diluent,
or **in that**
(c) sulphonyl chlorides of the general formula (VI) 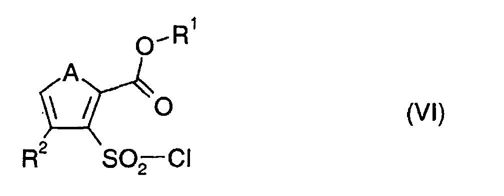 in which
A, R¹ and R² are each as defined above,
are reacted with heterocycles of the general formula (V)
H-R³ (V)
in which
R³ is as defined above,
and metal (thio)cyanates of the general formula (VII)
MQCN (VII)
in which
Q is as defined above,
if appropriate in the presence of a reaction auxiliary and if appropriate in the presence of a diluent,
or **in that**
(d) sulphonyl chlorides of the general formula (VI) 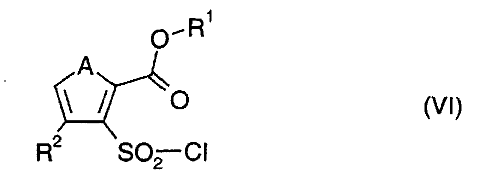 in which
A, R¹ and R² are each as defined above,
are reacted with (thio)carboxamides of the general formula (VIII) 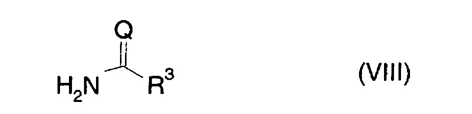 in which
Q and R³ are each as defined above,
if appropriate in the presence of an acid acceptor and if appropriate in the presence of a diluent,
or **in that**
(e) sulphonylamino(thio)carbonyl compounds of the general formula (IX) 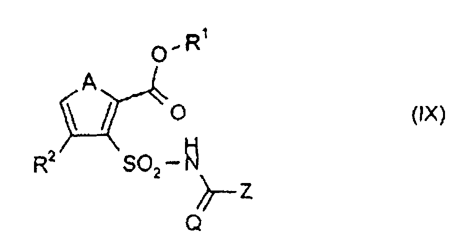 in which
A, Q, R¹ and R² are each as defined above and
Z represents halogen, alkoxy, aryloxy or arylalkoxy,
are reacted with heterocycles of the general formula (V)
H-R³ (V)
in which
R³ is as defined above,
if appropriate in the presence of an acid acceptor and if appropriate in the presence of a diluent,
and the compounds of the formula (I) obtained by processes (a), (b), (c), (d) or (e) are, if desired, converted into salts by customary methods.

5. Herbicidal composition, **characterized in that** it contains at least one compound of the formula (I) or a salt thereof according to Claim 1.

6. Use of compounds of the general formula (I) or of salts thereof according to Claim 1 for controlling unwanted vegetation.

7. Method for controlling weeds, **characterized in that** compounds of the general formula (I) or salts thereof according to Claim 1 are allowed to act on the weeds or their habitat.

8. Process for preparing herbical compositions, **characterized in that** compounds of the general formula (I) or salts thereof according to Claim 1 are mixed with extenders and/or surfactants.

9. Sulphonyl chlorides of the formula (VIa) 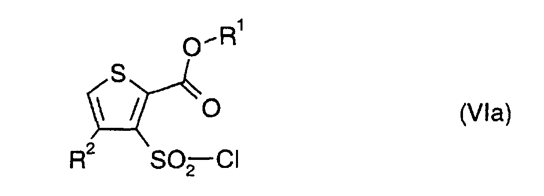 in which
R¹ and R² are as defined in claim 1.

10. Sulphonamides of the formula (IIa) 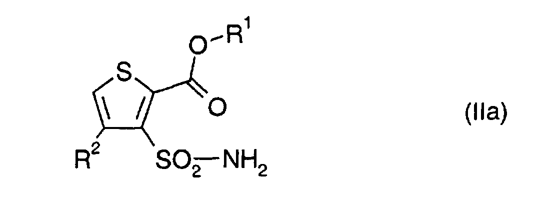 in which,
R¹ represents respectively optionally cyano-, fluorine-, chlorine-, methoxy- or ethoxy-substituted methyl, ethyl, n- or i-propyl, n-, i- or s-butyl, represents respectively optionally cyano-, fluorine- or chlorine-substituted propenyl, butenyl, propinyl or butinyl, represents respectively optionally cyano-, fluorine-, chlorine-, methyl- or ethyl-substituted cyclopropyl, cyclobutyl, cyclopentyl, cyclohexyl, cyclopropylmethyl, cyclobutylmethyl, cyclopentylmethyl or cyclohexylmethyl, represents respectively optionally cyano-, fluorine-, chlorine-, bromine-, methyl-, ethyl-, trifluoromethyl-, methoxy-, ethoxy-, difluoromethoxy- or trifluoromethoxy-substituted phenyl, phenylmethyl or phenylethyl, or represents respectively optionally cyano-, fluorine-, chlorine-, bromine-, methyl-, ethyl-, methoxy- or ethoxy-substituted heterocyclyl or heterocyclyl-methyl, where in each case the heterocyclyl group is selected from the group consisting of oxetanyl, thietanyl, furyl, tetrahydrofuryl, thienyl, tetrahydrothienyl, and
R² represents cyano, fluorine, chlorine, bromine, represents respectively optionally cyano-, fluorine-, chlorine-, methoxy- or ethoxy-substituted methyl, ethyl, n- or i-propyl, n-, i- or s-butyl, methoxy, ethoxy, n- or i-propoxy, methoxycarbonyl, ethoxycarbonyl, n- or i-propoxycarbonyl, methylsulphinyl, ethylsulphinyl, methylsulphonyl or ethylsulphonyl, or represents respectively optionally cyano-, fluorine- or chlorine-substituted propenyl, butenyl, propinyl, butinyl, propenyloxy or propinyloxy, and

11. Compounds according to Claim 1 which contain the following combinations of the radical definitions A, Q, R¹, R² and R³:

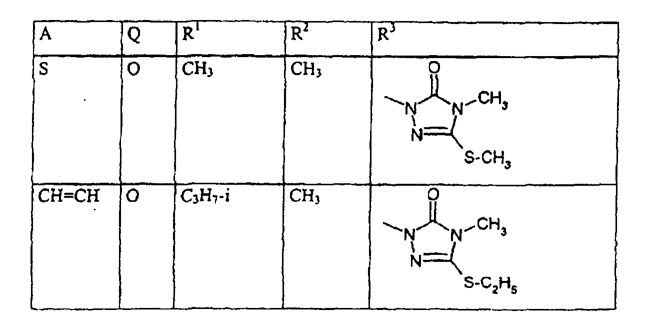

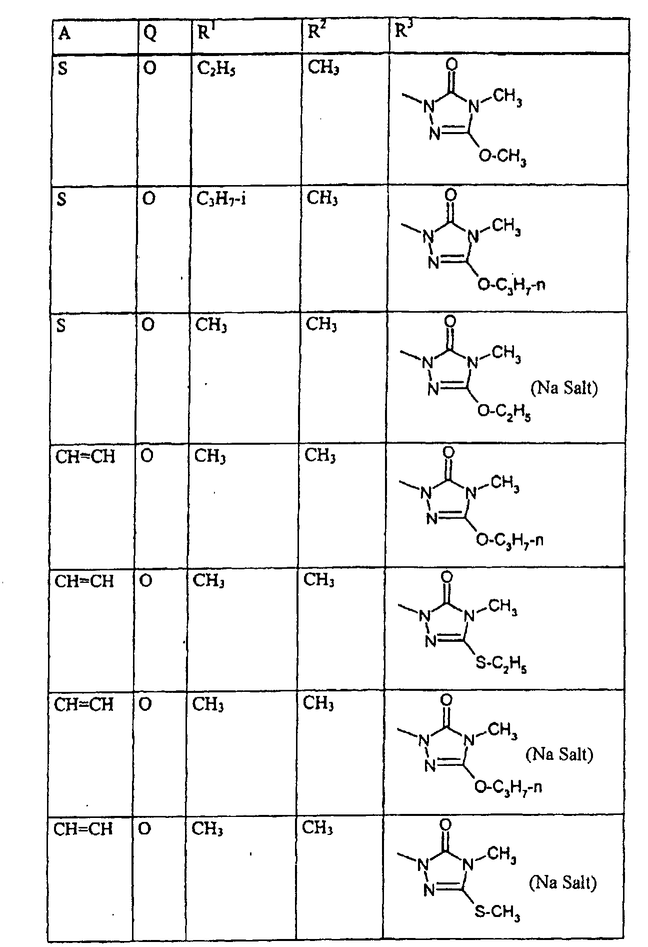

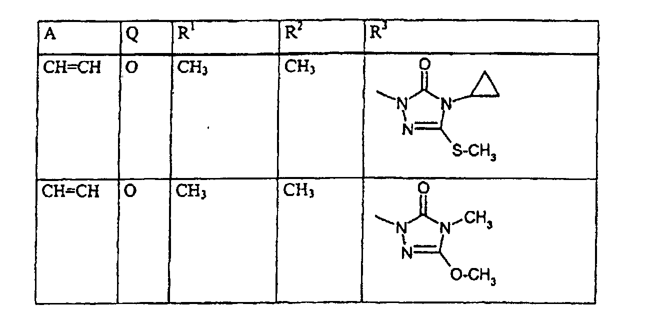

## Revendications

1. Composés portant un groupe sulfonylamino(thio)-carbonylsubstitué, de formule générale (I) 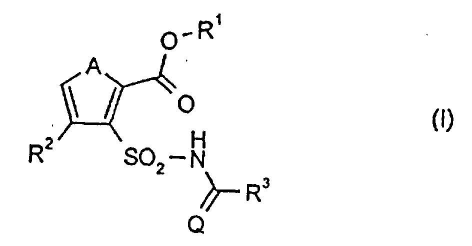 dans laquelle
A représente le soufre ou le groupe -CH=CH-,
Q est l'oxygène ou le soufre,
R¹ est un groupe alkyle en C₁ à C₆ portant éventuellement un substituant cyano, nitro, halogéno ou alkoxy en C₁ à C₄, un groupe alcényle en C₂ à C₆ ou un groupe alcynyle en C₂ à C₆ portant chacun, le cas échéant, un substituant cyano ou halogéno, un groupe cycloalkyle en C₃ à C₆ ou un groupe (cycloalkyle en C₃ à C₆)-(alkyle en C₁ à C₄) portant chacun, le cas échéant, un substituant cyano, halogéno ou alkyle en C₁ à C₄, un groupe phényle ou un groupe phényl-(alkyle en C₁ à C₄) portant chacun, le cas échéant, un substituant cyano, nitro, halogéno, alkyle en C₁ à C₄, halogénalkyle en C₁ à C₄, alkoxy en C₁ à C₄ ou halogénalkoxy en C₁ à C₄, ou bien un groupe hétérocyclyle ou un groupe hétérocyclyl-(alkyle en C₁ à C₄) portant chacun, le cas échéant, un substituant cyano, nitro, halogéno, alkyle en C₁ à C₄, halogénalkyle en C₁ à C₄, alkoxy en C₁ à C₄ ou halogénalkoxy en C₁ à C₄, le groupe hétérocyclyle étant choisi dans chaque cas dans la série oxétannyle, thiétannyle, furyle, tétrahydrofuryle, thiényle, tétrahydrothiényle, oxazolyle, isoxazolyle, thiazolyle, oxadiazolyle, thiadiazolyle,
R² est un groupe cyano, nitro, halogéno, un groupe alkyle en C₁ à C₄, alkoxy en C₁ à C₄, (alkoxy en C₁ à C₄)-carbonyle, alkylthio en C₁ à C₄, alkylsulfinyle en C₁ à C₄ ou alkylsulfonyle en C₁ à C₄ portant chacun, le cas échéant, un substituant cyano, halogéno ou alkoxy en C₁ à C₄, ou bien un groupe alcényle en C₂ à C₄, alcynyle en C₂ à C₄, alcényloxy en C₂ à C₄ ou alcynyloxy en C₂ à C₄ portant chacun, le cas échéant, un substituant cyano ou halogéno, et
R³ est un groupe hétérocyclyle éventuellement substitué de formule suivante 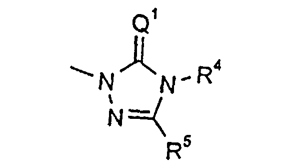 dans laquelle
Q¹ est l'oxygène ou le soufre,
R⁴ représente l'hydrogène, un groupe hydroxy, amino, cyano, un groupe alkylidène-amino en C₂ à C₁₀, un groupe alkyle en C₁ à C₆ portant éventuellement un substituant fluoro, chloro, bromo, cyano, alkoxy en C₁ à C₄, (alkyle en C₁ à C₄)carbonyle ou (alkoxy en C₁ à C₄)carbonyle, un groupe alcényle en C₂ à C₆ ou un groupe alcynyle en C₂ à C₆ portant chacun, le cas échéant, un substituant fluoro, chloro et/ou bromo, un groupe alkoxy en C₁ à C₆, alkylamino en C₁ à C₆ ou (alkyle en C₁ à C₆)carbonylamino portant chacun, le cas échéant, un substituant fluoro, chloro, bromo, cyano, alkoxy en C₁ à C₄ ou (alkoxy en C₁ à C₄)carbonyle, un groupe alcényloxy en C₃ à C₆, un groupe di-(alkyle en C₁ à C₄)-amino, un groupe cycloalkyle en C₃ à C₆), (cycloalkyle en C₃ à C₆)amino ou (cycloalkyle en C₃ à C₆)-(alkyle en C₁ à C₄) portant chacun, le cas échéant, un substituant fluoro, chloro, bromo, cyano et/ou alkyle en C₁ à C₄, ou bien un groupe phényle ou un groupe phényl-(alkyle en C₁ à C₄) portant chacun, le cas échéant, un substituant fluoro, chloro, bromo, cyano, nitro, alkyle en C₁ à C₄, trifluorométhyle et/ou alkoxy en C₁ à C₄, et
R⁵ représente l'hydrogène, un groupe hydroxy, mercapto, amino, cyano, fluoro, chloro, bromo, iodo, un groupe alkyle en C₁ à C₄ portant éventuellement un substituant fluoro, chloro, bromo, cyano, alkoxy en C₁ à C₄, (alkyle en C₁ à C₄)carbonyle ou (alkoxy en C₁ à C₄)carbonyle, un groupe alcényle en C₂ à C₆ ou un groupe alcynyle en C₂ à C₆ portant chacun, le cas échéant, un substituant fluoro, chloro et/ou bromo, un groupe alkoxy en C₁ à C₆, alkylthio en C₁ à C₆, alkylamino en C₁ à C₆ ou (alkyle en C₁ à C₆)carbonylamino portant chacun, le cas échéant, un substituant fluoro, chloro, cyano, alkoxy en C₁ à C₄ ou (alkoxy en C₁ à C₄)carbonyle, un groupe alcényloxy en C₃ à C₆, alcynyloxy en C₃ à C₆, alcénylthio en C₃ à C₆, alcynylthio en C₃ à C₆, alcénylamino en C₃ à C₆ ou alcynylamino en C₃ à C₆, un groupe di-(alkyle en C₁ à C₄)amino, un groupe aziridino, pyrrolidino, pipéridino ou morpholino portant chacun, le cas échéant, un substituant méthyle et/ou éthyle, un groupe cycloalkyle en C₃ à C₆, cycloalcényle en C₅ ou C₆, cycloalkyloxy en C₃ à C₆, cycloalkylthio en C₃ à C₆, cycloalkylamino en C₃ à C₆, (cycloalkyle en C₃ à C₆)-(alkyle en C₁ à C₄), (cycloalkyle en C₃ à C₆)-(alkoxy en C₁ à C₄), (cycloalkyle en C₃ à C₆)-(alkylthio en C₁ à C₄) ou (cycloalkyle en C₃ à C₆)-(alkylamino en C₁ à C₄) portant chacun, le cas échéant, un substituant fluoro, chloro, bromo, cyano et/ou alkyle en C₁ à C₄, ou bien un groupe phényle, phényl-(alkyle en C₁ à C₄), phénoxy, phényl-(alkoxy en C₁ à C₄), phénylthio, phényl-(alkylthio en C₁ à C₄), phénylamino ou phényl-(alkylamino en C₁ à C₄) portant chacun, le cas échéant, un substituant fluoro, chloro, bromo, cyano, nitro, alkyle en C₁ à C₄, trifluorométhyle, alkoxy en C₁ à C₄ et/ou (alkoxy en C₁ à C₄) carbonyle, ou bien
R⁴ et R⁵ forment ensemble un groupe alcanediyle de 3 à 11 atomes de carbone éventuellement ramifié,
ainsi que les sels de sodium, de potassium, de magnésium, de calcium, d'ammonium, de (alkyle en C₁ à C₄)ammonium, de di-(alkyle en C₁ à C₄)-ammonium, de tri-(alkyle en C₁ à C₄)-ammonium, de tétra-(alkyle en C₁ à C₄)-ammonium, de tri-(alkyle en C₁ à C₄)-sulfonium, de (cycloalkyle en C₅ ou C6)-ammonium et de di-(alkyle en C₁ ou C₂)-benzylammonium de composés de formule (I).

2. Composés de formule (I) suivant la revendication 1, **caractérisés en ce que** dans leur formule
R¹ représente un groupe méthyle, éthyle, n-propyle, isopropyle, n-butyle, isobutyle ou sec.-butyle portant chacun, le cas échéant, un substituant cyano, fluoro, chloro, méthoxy ou éthoxy, un groupe propényle, butényle, propynyle ou butynyle portant chacun, le cas échéant, un substituant cyano, fluoro ou chloro, un groupe cyclopropyle, cyclobutyle, cyclopentyle, cyclohexyle, cyclopropylméthyle, cyclobutylméthyle, cyclopentylméthyle ou cyclohexylméthyle portant chacun, le cas échéant, un substituant cyano, fluoro, chloro, méthyle ou éthyle, un groupe phényle, phénylméthyle ou phényléthyle portant chacun, le cas échéant, un substituant cyano, fluoro, chloro, bromo, méthyle, éthyle, trifluorométhyle, méthoxy, éthoxy, difluorométhoxy ou trifluorométhoxy, ou un groupe hétérocyclyle ou hétérocyclylméthyle portant chacun, le cas échéant, un substituant cyano, fluoro, chloro, bromo, méthyle, éthyle, méthoxy ou éthoxy, chaque groupe hétérocyclyle étant choisi dans la série oxétannyle, thiétannyle, furyle, tétrahydrofuryle, thiényle, tétrahydrothiényle,
R² est un groupe cyano, fluoro, chloro, bromo, un groupe méthyle, éthyle, n-propyle, isopropyle, n-butyle, isobutyle, sec.-butyle, méthoxy, éthoxy, n-propoxy, isopropoxy, méthoxycarbonyle, éthoxycarbonyle, n-propoxycarbonyle, isopropoxycarbonyle, méthylthio, éthylthio, méthylsulfinyle, éthylsulfinyle, méthylsulfonyle ou éthylsulfonyle portant chacun, le cas échéant, un substituant cyano, fluoro, chloro, méthoxy ou éthoxy, ou un groupe propényle, butényle, propynyle, butynyle, propényloxy ou propynyloxy portant chacun, le cas échéant, un substituant cyano, fluoro ou chloro,
et
R³ représente un groupe hétérocyclyle éventuellement substitué de formule suivante 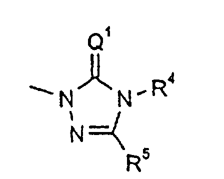 dans laquelle
Q¹ représente l'oxygène ou le soufre,
R⁴ est l'hydrogène, un groupe hydroxy, amino, un groupe méthyle, éthyle, n-propyle, isopropyle, n-butyle, isobutyle, sec.-butyle ou tertio-butyle portant chacun, le cas échéant, un substituant fluoro, chloro, cyano, méthoxy ou éthoxy, un groupe propényle, butényle, propynyle ou butynyle portant chacun, le cas échéant, un substituant fluoro, chloro ou bromo, un groupe méthoxy, éthoxy, n-propoxy, isopropoxy, n-butoxy, isobutoxy, sec.-butoxy, tertio-butoxy, méthylamino, éthylamino, n-propylamino, isopropylamino, n-butylamino, isobutylamino, sec.-butylamino ou tertio-butylamino portant chacun, le cas échéant, un substituant fluoro, chloro, cyano, méthoxy ou éthoxy, un groupe propényloxy ou butényloxy, un groupe diméthylamino ou diéthylamino, un groupe cyclopropyle, cyclobutyle, cyclopentyle, cyclohexyle, cyclopropylamino, cyclobutylamino, cyclopentylamino, cyclohexylamino, cyclopropylméthyle, cyclobutylméthyle, cyclopentylméthyle ou cyclohexylméthyle portant chacun, le cas échéant, un substituant fluoro, chloro, méthyle et/ou éthyle, ou un groupe phényle ou benzyle portant chacun, le cas échéant, un substituant fluoro, chloro, méthyle, trifluorométhyle et/ou méthoxy, et
R⁵ représente l'hydrogène, un groupe hydroxy, mercapto, amino, fluoro, chloro, bromo, un groupe méthyle, éthyle, n-propyle, isopropyle, n-butyle, isobutyle, sec.-butyle ou tertio-butyle portant chacun, le cas échéant, un substituant fluoro, chloro, cyano, méthoxy ou éthoxy, un groupe éthényle, propényle, butényle, propynyle ou butynyle portant chacun, le cas échéant, un substituant fluoro, chloro ou bromo, un groupe méthoxy, éthoxy, n-propoxy, isopropoxy, n-butoxy, isobutoxy, sec.-butoxy, tertio-butoxy, méthylthio, éthylthio, n-propylthio, isopropylthio, n-butylthio, isobutylthio, sec.-butylthio, tertio-butylthio, méthylamino, éthylamino, n-propylamino, isopropylamino, n-butylamino, isobutylamino, sec.-butylamino ou tertio-butylamino portant chacun, le cas échéant, un substituant fluoro, chloro, cyano, méthoxy ou éthoxy, un groupe propényloxy, butényloxy, propynyloxy, butynyloxy, propénylthio, propadiénylthio, buténylthio, propynylthio, butynylthio, propénylamino, buténylamino, propynylamino ou butynylamino, un groupe diméthylamino, diéthylamino ou dipropylamino, un groupe cyclopropyle, cyclobutyle, cyclopentyle, cyclohexyle, cyclopentényle, cyclohexényle, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino, cyclohexylamino, cyclopropylméthyle, cyclobutylméthyle, cyclopentylméthyle, cyclohexylméthyle, cyclopropylméthoxy, cyclobutylméthoxy, cyclopentylméthoxy, cyclohexylméthoxy, cyclopropylméthylthio, cyclobutylméthylthio, cyclopentylméthylthio, cyclohexylméthylthio, cyclopropylméthylamino, cyclobutylméthylamino, cyclopentylméthylamino ou cyclohexylméthylamino portant chacun, le cas échéant, un substituant fluoro, chloro, méthyle et/ou éthyle, ou un groupe phényle, benzyle, phénoxy, benzyloxy, phénylthio, behzylthio, phénylamino ou benzylamino portant chacun, le cas échéant, un substituant fluoro, chloro, méthyle, trifluorométhyle, méthoxy et/ou méthoxycarbonyle, ou bien
R⁴ et R⁵ forment ensemble un groupe alcanediyle de 3 à 11 atomes de carbone éventuellement ramifié.

3. Composés de formule (I) suivant la revendication 1, **caractérisés en ce que** dans leur formule
R¹ est un groupe méthyle, éthyle, n-propyle, isopropyle, 2-cyanéthyle, 2-fluoréthyle, 2,2-difluoréthyle, 2,2,2-trifluoréthyle, 2-chloréthyle, 2,2-dichloréthyle, 2,2,2-trichloréthyle, 2-méthoxyéthyle, 2-éthoxyéthyle ou oxétannyle,
R² représente le fluor, le chlore, le brome, un groupe méthyle, éthyle, n-propyle, isopropyle, n-butyle, isobutyle, sec.-butyle, trifluorométhyle, méthoxy, éthoxy, n-propoxy, isopropoxy, difluorométhoxy, trifluorométhoxy ou trifluoréthoxy, et
R³ est un groupe triazolinyle éventuellement substitué de formule suivante 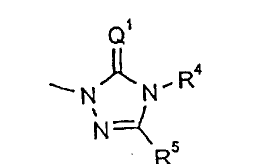 dans laquelle
Q¹ représente l'oxygène ou le soufre,
R⁴ est un groupe méthyle, éthyle, n-propyle ou isopropyle portant chacun, le cas échéant, un substituant fluoro, chloro, méthoxy ou éthoxy, un groupe propényle ou un groupe propynyle portant chacun, le cas échéant, un substituant fluoro ou chloro, un groupe méthoxy, éthoxy, n-propoxy, isopropoxy, méthylamino, éthylamino, n-propylamino ou isopropylamino, un groupe propényloxy, un groupe diméthylamino ou un groupe cyclopropyle, et
R⁵ représente le chlore ou le brome, un groupe méthyle, éthyle, n-propyle, isopropyle, n-butyle, isobutyle, sec.-butyle ou tertio-butyle portant chacun, le cas échéant, un substituant fluoro, chloro, cyano, méthoxy ou éthoxy, un groupe éthényle, propényle, butényle, propynyle ou butynyle portant chacun, le cas échéant, un substituant fluoro, chloro ou bromo, un groupe méthoxy, éthoxy, n-propoxy, isopropoxy, n-butoxy, isobutoxy, sec.-butoxy, tertio-butoxy, méthylthio, éthylthio, n-propylthio, isopropylthio, n-butylthio, isobutylthio, sec.-butylthio, tertio-butylthio, méthylamino, éthylamino, n-propylamino, isopropylamino, n-butylamino, isobutylamino, sec.-butylamino, tertio-butylamino portant chacun, le cas échéant, un substituant fluoro, chloro, cyano, méthoxy ou éthoxy, un groupe propényloxy, butényloxy, propynyloxy, butynyloxy, propénylthio, propadiénylthio, buténylthio, propynylthio, butynylthio, propénylamino, buténylamino, propynylamino ou butynylamino, un groupe diméthylamino, diéthylamino ou dipropylamino, un groupe cyclopropyle, cyclobutyle, cyclopentyle, cyclohexyle, cyclopentényle, cyclohexényle, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino, cyclohexylamino, cyclopropylméthyle, cyclobutylméthyle, cyclopentylméthyle, cyclohexylméthyle, cyclopropylméthoxy, cyclobutylméthoxy, cyclopentylméthoxy, cyclohexylméthoxy, cyclopropylméthylthio, cyclobutylméthylthio, cyclopentylméthylthio, cyclohexylméthylthio, cycloproylméthylamino, cyclobutylméthylamino, cyclopentylméthylamino ou cyclohexylméthylamino portant chacun, le cas échéant, un substituant fluoro, chloro, méthyle et/ou éthyle, ou un groupe phényle, benzyle, phénoxy, benzyloxy, phénylthio, benzylthio, phénylamino ou benzylamino portant chacun, le cas échéant, un substituant fluoro, chloro, méthyle, trifluorométhyle, méthoxy et/ou méthoxycarbonyle, ou bien
R⁴ et R⁵ forment ensemble un groupe alcanediyle de 3 à 11 atomes de carbone éventuellement ramifié.

4. Procédé de production de composés de formule (I) suivant la revendication 1, **caractérisé en ce que** :
(a) on fait réagir des sulfonamides de formule générale (II) 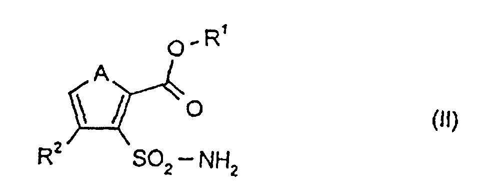 dans laquelle
A, R¹ et R² ont la définition indiquée dans la revendication 1,
avec des dérivés d'acides (thio)carboxyliques de formule générale (III) 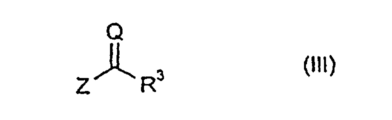 dans laquelle
Q et R³ ont la définition indiquée dans la revendication 1 et
Z représente un halogène, un groupe alkoxy, aryloxy ou arylalkoxy,
le cas échéant en présence d'un accepteur d'acide et en présence éventuelle d'un diluant,
ou **en ce que** :
(b) on fait réagir des sulfonyliso(thio)cyanates de formule générale (IV) 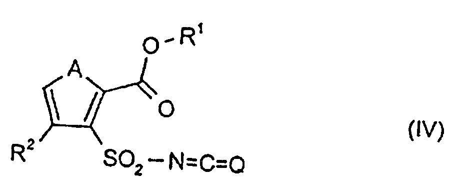 dans laquelle
A, Q, R¹ et R² ont la définition indiquée ci-dessus,
avec des hétérocycles de formule générale (V)
H-R³ (V)
dans laquelle
R³ a la définition indiquée ci-dessus,
le cas échéant en présence d'un auxiliaire de réaction et en présence éventuelle d'un diluant,
ou **en ce que** :
(c) on fait réagir des chlorures d'acides sulfoniques de formule générale (VI) 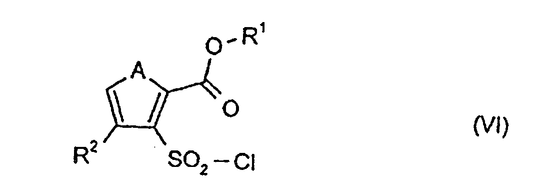 dans laquelle
A, R¹ et R² ont la définition indiquée ci-dessus,
avec des hétérocycles de formule générale (V)
H-R³ (V)
dans laquelle
R³ a la définition indiquée ci-dessus,
et des (thio)cyanates métalliques de formule générale (VII)
MQCN (VII)
dans laquelle
Q a la définition indiquée ci-dessus,
le cas échéant en présence d'auxiliaires de réaction et éventuellement en présence d'un diluant,
ou **en ce que** :
(d) on fait réagir des chlorures d'acides sulfoniques de formule générale (VI) 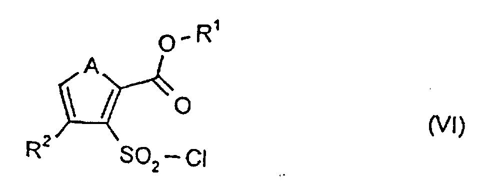 dans laquelle
A, R¹ et R² ont la définition indiquée ci-dessus,
avec des (thio)carboxamides de formule générale (VIII) 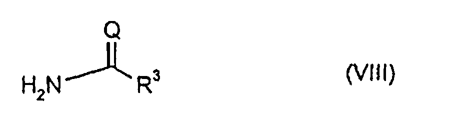 dans laquelle
Q et R³ ont la définition indiquée ci-dessus,
le cas échéant en présence d'un accepteur d'acide et en présence éventuelle d'un diluant,
ou **en ce que** :
(e) on fait réagir des composés sulfonylamino(thio)-carbonyliques de formule générale (IX) 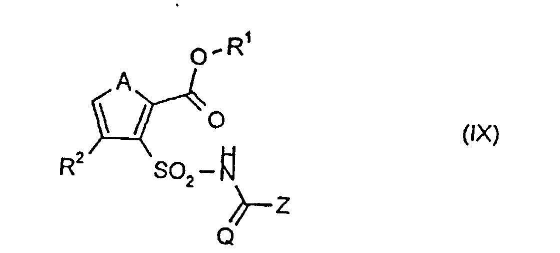 dans laquelle
A, Q, R¹ et R² ont la définition indiquée ci-dessus et
Z représente un halogène, un groupe alkoxy, aryloxy ou arylalkoxy,
avec des hétérocycles de formule générale (V)
H-R³ (V)
dans laquelle
R³ a la définition indiquée ci-dessus,
le cas échéant en présence d'un accepteur d'acide et en présence éventuelle d'un diluant,
et on transforme éventuellement en sels d'après des modes opératoires classiques les composés obtenus selon les procédés (a), (b), (c), (d) ou (e).

5. Compositions herbicides, **caractérisées par** une teneur en au moins un composé de formule (I) ou l'un de ses sels suivant la revendication 1.

6. Utilisation de composés de formule générale (I) ou de leurs sels suivant la revendication 1 pour combattre une végétation indésirable.

7. Procédé pour combattre des mauvaises herbes, **caractérisé en ce qu'**on fait agir sur les mauvaises herbes ou sur leur milieu des composés de formule générale (I) ou leurs sels suivant la revendication 1.

8. Procédé de préparation de compositions herbicides, **caractérisé en ce qu'**on mélange des composés de formule générale (I) ou leurs sels suivant la revendication 1 avec des diluants et/ou des agents tensio-actifs.

9. Chlorures d'acides sulfoniques de formule (IVa) 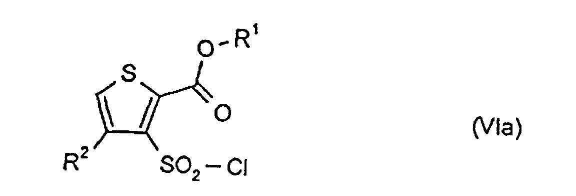 dans laquelle
R¹ et R² sont tels que définis dans la revendication 1.

10. Sulfonamides de formule (IIa) 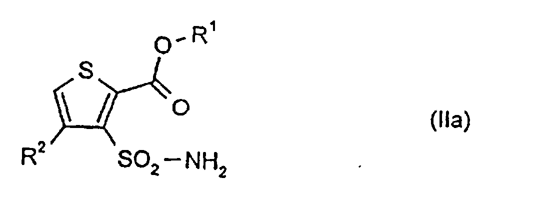 dans laquelle
R¹ est un groupe méthyle, éthyle, n-propyle, isopropyle, n-butyle, isobutyle ou sec.-butyle portant chacun, le cas échéant, un substituant cyano, fluoro, chloro, méthoxy ou éthoxy, un groupe propényle, butényle, propynyle ou butynyle portant chacun, le cas échéant, un substituant cyano, fluoro ou chloro, un groupe cyclopropyle, cyclobutyle, cyclopentyle, cyclohexyle, cyclopropylméthyle, cyclobutylméthyle, cyclopentylméthyle ou cyclohexylméthyle portant chacun, le cas échéant, un substituant cyano, fluoro, chloro, méthyle ou éthyle, un groupe phényle, phénylméthyle ou phényléthyle portant chacun, le cas échéant, un substituant cyano, fluoro, chloro, bromo, méthyle, éthyle, trifluorométhyle, méthoxy, éthoxy, difluorométhoxy ou trifluorométhoxy, un groupe hétérocyclyle ou hétérocyclylméthyle portant chacun, le cas échéant, un substituant cyano, fluoro, chloro, bromo, méthyle, éthyle, méthoxy ou éthoxy, le groupe hétérocyclyle étant choisi dans chaque cas dans la série oxétannyle, thiétannyle, furyle, tétrahydrofuryle, thiényle, tétrahydrothiényle, et
R² représente un groupe cyano, fluoro, chloro, bromo, un groupe méthyle, éthyle, n-propyle, isopropyle, n-butyle, isobutyle, sec.-butyle, méthoxy, éthoxy, n-propoxy, isopropoxy, méthoxycarbonyle, éthoxycarbonyle, n-propoxycarbonyle, isopropoxycarbonyle, méthylsulfinyle, éthylsulfinyle, méthylsulfonyle ou éthylsulfonyle portant chacun, le cas échéant, un substituant cyano, fluoro, chloro, méthoxy ou éthoxy, ou un groupe propényle, butényle, propynyle, butynyle, propényloxy ou propynyloxy portant chacun, le cas échéant, un substituant cyano, fluoro ou chloro.

11. Composés suivant la revendication 1, dans lesquels existent les combinaisons suivantes des définitions des restes A, Q, R¹, R² et R³ :
